(19) Europäisches Patentamt / European Patent Office / Office européen des brevets

(11) EP 3 656 854 A1

(12) EUROPEAN PATENT APPLICATION
published in accordance with Art. 153(4) EPC

(43) Date of publication:
27.05.2020 Bulletin 2020/22

(21) Application number: 18835927.7

(22) Date of filing: 18.07.2018

(51) Int Cl.:
*C12N 7/00* (2006.01) *C12N 15/11* (2006.01)
*A61K 35/76* (2015.01) *A61P 35/00* (2006.01)

(86) International application number:
PCT/CN2018/096100

(87) International publication number:
WO 2019/015601 (24.01.2019 Gazette 2019/04)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 21.07.2017 CN 201710600732

(71) Applicants:
- **Xiamen University**
  **Xiamen, Fujian 361005 (CN)**
- **Yang Sheng Tang Company, Ltd.**
  **Zhejiang (CN)**

(72) Inventors:
- **CHENG, Tong**
  **Xiamen**
  **Fujian 361005 (CN)**
- **WANG, Wei**
  **Xiamen**
  **Fujian 361005 (CN)**
- **WAN, Junkai**
  **Xiamen**
  **Fujian 361005 (CN)**
- **FU, Wenkun**
  **Xiamen**
  **Fujian 361005 (CN)**
- **YE, Xiangzhong**
  **Beijing 102206 (CN)**
- **ZHANG, Jun**
  **Xiamen**
  **Fujian 361005 (CN)**
- **XIA, Ningshao**
  **Xiamen**
  **Fujian 361005 (CN)**

(74) Representative: **Held, Stephan**
**Meissner Bolte Patentanwälte**
**Rechtsanwälte Partnerschaft mbB**
**Postfach 86 06 24**
**81633 München (DE)**

# (54) VIRUS FOR TREATING TUMORS

(57) Provided are an enterovirus D68 (EV-D68) or a modified form thereof, or a nucleic acid molecule comprising a genomic sequence or cDNA sequence of the EV-D68 or a modified form thereof, or a complementary sequence of the genomic sequence or cDNA sequence, or a pharmaceutical composition comprising the EV-D68 or a modified form thereof, or the nucleic acid molecule, and use of the EV-D68 or a modified form thereof, or the nucleic acid molecule in the manufacture of a pharmaceutical composition for treating a tumor.

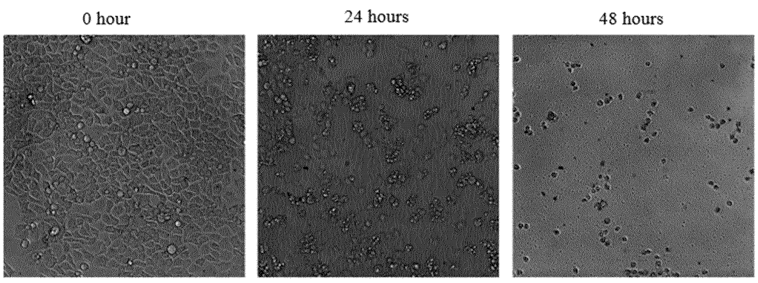


FIG. 4

EP 3 656 854 A1

## Description

### Technical Field

The present invention relates to the field of viruses and the field of tumor treatment. Specifically, the present invention relates to use of an Enterovirus D68 (EV-D68) or a modified form thereof, or a nucleic acid molecule comprising a genomic sequence or cDNA sequence of EV-D68 or a modified form thereof, or a complementary sequence of the genomic sequence or cDNA sequence, for treating a tumor in a subject (e.g., a human), and for manufacture of a medicament for treating a tumor in a subject (e.g., a human). The present invention also relates to a method for treating a tumor, which comprises a step of administering to a subject in need thereof EV-D68 or a modified form thereof, or a nucleic acid molecule comprising a genomic sequence or cDNA sequence of EV-D68 or a modified form thereof, or a complementary sequence of the genomic sequence or cDNA sequence.

### Background Art

The current methods for treatment of malignant tumors include surgery chemotherapy and radiotherapy. These traditional therapies are not satisfactory for the treatment of metastatic tumors, and may also cause great harm to patients' health. In contrast, as a new type of treatment method, the tumor treatment method using oncolytic virus has high specificity, good effectiveness, and less side effects, and is currently considered as a promising tumor treatment method.

Oncolytic virus is a virus that can self-replicate in tumor cells, thereby killing and lysing the tumor cells, or arresting the growth of the tumor cells. When used in in vivo treatment, oncolytic virus shows specificity for tumor cells, and can directly induce death of tumor cells, but has little or no effect on normal cells. Meanwhile, oncolytic virus can also induce cytotoxic T lymphocyte response in the immune system, thereby indirectly killing tumor cells.

Enterovirus belongs to *Picornaviridae* family, and its genome is single-stranded positive-sense RNA. There are following advantages in using enterovirus as oncolytic virus: firstly, as single-stranded RNA virus, its genome won't undergo any stages of DNA in the host, so that there won't be genotoxicity caused by the insertion of the viral genome into the host's DNA, which has better safety; secondly, the enterovirus genome is relatively small, and a large number of viruses can be replicated in a short period of time to further infect other tumor cells, causing a strong cytopathic effect; next, the enterovirus does not contain oncogenes and therefore does not induce tumor; and finally, the genome of the enterovirus can be modified by reverse genetics technology to achieve attenuation of virus and reduce its side effects.

At present, the reported enteroviruses with oncolytic activity include chimeric polioviruses for the treatment of human solid tumors such as malignant gliomas (Dobrikova et al., Mol Ther 2008, 16 (11): 1865-1872); Coxsackie viruses A13, A15, A18, and A21 that kill human melanoma cells (Au et al., Virol J 2011, 8: 22); Echo virus ECHO1 that kills human gastric cancer cells and ovarian cancer cells (Shafren et al., Int J Cancer 2005, 115 (2): 320-328; Haley et al., J Mol Med (Berl) 2009, 87 (4): 385-399) and the like. However, it is still necessary to obtain viruses with both tumor-specificity and tumor-killing activity.

Enterovirus D68 (EV-D68) is a kind of Enterovirus D of the genus *Enteroviruses* of *Picornaviridae* family, which was first isolated from children with respiratory infections in California in 1962 (Schieble et al., Am J Epidemiol 1967, 85 (2): 297-310). Unlike most enteroviruses, which are resistant to acids and reproduce in the human gastrointestinal tract, EV-D68 is sensitive to acids and replicates mainly in the respiratory tract. Since there have been few reports of EV-D68 infection for a long time, EV-D68 is considered to be a rare pathogen that mainly causes mild respiratory diseases, including runny nose, sneezing, and cough. At present, there is not report in the art that enterovirus 68 has oncolytic activity.

### Contents of the Invention

After a lot of experiments and repeated explorations, it is unexpectedly found that Enterovirus D68 has a broad spectrum and significant tumor cell killing ability. Based on this finding, the inventors of the present invention have developed a new oncolytic virus for treating tumors and a tumor treatment method based on the virus.

<u>Medical use</u>

In a first aspect, the present invention provides use of an Enterovirus D68 (EV-D68) or a modified form thereof, or an isolated nucleic acid molecule, in treatment of a tumor in a subject, or in the manufacture of a medicament for treating a tumor in a subject; wherein the isolated nucleic acid molecule comprises a sequence selected from the following:

(1) a genomic sequence or cDNA sequence of EV-D68 or a modified form thereof; and

(2) a complementary sequence of the genomic sequence or cDNA sequence.

In certain preferred embodiments, the EV-D68 is a wild-type EV-D68. In certain preferred embodiments, the EV-D68 may be a clinical isolate isolated from an individual infected with the Enterovirus D68.

In certain preferred embodiments, the EV-D68 or a modified form thereof has a genomic sequence that has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 12. In certain preferred embodiments, the genomic sequence of the EV-D68 or a modified form thereof is a nucleotide sequence as shown in SEQ ID NO: 12.

In certain preferred embodiments, the EV-D68 or a modified form thereof has a cDNA sequence that has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence shown in SEQ ID NO: 1. In certain preferred embodiments, the cDNA sequence of the EV-D68 or a modified form thereof is a nucleotide sequence as shown in SEQ ID NO: 1.

In certain preferred embodiments, the modified form is a modified EV-D68, which has a substitution, insertion, or deletion of one or more nucleotides in the genome as compared to a wild-type EV-D68.

In certain preferred embodiments, as compared to the wild-type EV-D68, the modified EV-D68 has one or more modifications selected from the following:

(1) one or more mutations in an untranslated region (e.g., 5'UTR or 3'UTR);

(2) an insertion of one or more exogenous nucleic acids;

(3) a deletion or mutation of one or more endogenous genes; and

(4) any combination of the above three items.

In certain preferred embodiments, the modified EV-D68 includes one or more mutations in the 5' untranslated region (5'UTR).

In certain preferred embodiments, the modified EV-D68 has a substitution of all or part of the 5'UTR sequence. In certain preferred embodiments, the internal ribosome entry site (IRES) sequence in the 5'UTR of the modified EV-D68 is replaced with an exogenous IRES sequence, such as the internal ribosome entry site sequence of human rhinovirus 2 (HRV2). In certain preferred embodiments, the internal ribosome entry site sequence of the human rhinovirus 2 (HRV2) is shown in SEQ ID NO: 2.

The use of the internal ribosome entry site sequence of human rhinovirus 2 (HRV2) is advantageous in some cases, for example, it is conducive to improvement of the tumor specificity of oncolytic viruses. It has been previously reported that in normal human nerve cells, the internal ribosome entry site sequence of human rhinovirus 2 is specifically bound by host RNA-binding proteins (DRBP76 and NF45), thereby preventing the recruitment of factors such as eIF4G (Merrill et al. J Virol 2006,80 (7): 3147-3156; Merrill and Gromeier, J Virol 2006,80 (14): 6936-6942; Neplioueva et al., PLoS One 2010,5 (7): e11710); meanwhile, due to the lack of support of signaling pathways such as Raf/Erk1/2/MAPK, it is difficult for ribosomes to bind to the internal ribosome entry site sequence of human rhinovirus 2, so that it is impossible to initiate translation of viral protein (Dobrikov et al., Mol Cell Biol 2011, 31(14): 2947-2959; Dobrikov et al., Mol Cell Biol 2013, 33(5): 937-946). In human glioma tumor cells, the internal ribosome entry site of human rhinovirus 2 is not affected by the above two factors, and thus can normally initiate transcription and translation of viral protein. Therefore, in some cases, replacing the internal ribosome entry site sequence of EV-D68 with the internal ribosome entry site sequence of human rhinovirus 2 is beneficial to avoid or reduce the toxic and side effect of the virus of the present invention to normal human nerve cells, without affecting the use of the virus in the treatment of human gliomas.

In certain preferred embodiments, the modified EV-D68 comprises an exogenous nucleic acid.

In certain preferred embodiments, the exogenous nucleic acid encodes a cytokine (e.g., GM-CSF, preferably human GM-CSF), or an antitumor protein or polypeptide (e.g., a scFv against PD-1 or PD-L1, preferably a scFv against human PD-1 or PD-L1). In certain preferred embodiments, the exogenous nucleic acid is inserted between the 5'UTR gene and the VP4 gene, or between the VP1 gene and the 2A gene of the genome of the modified EV-D68.

In certain preferred embodiments, the exogenous nucleic acid comprises a target sequence of microRNA (miRNA) (e.g., miR-133 or miR-206). In certain preferred embodiments, the target sequence of microRNA inserted in the 3' untranslated region (3'UTR) of the genome of the modified EV-D68.

It has been previously reported that the expression level of certain microRNA in tumor cells is significantly lower than normal cells and/or has obvious tissue specificity. Therefore, in some cases, the modified EV-D68 of the present invention containing a target sequence of such microRNA is advantageous, because such microRNA that are highly expressed in normal cells or tissues can reduce or even block the replication of the modified EV-D68 in the normal cells or tissues by the corresponding target sequence, thereby reducing even avoiding the toxic side effects of the modified EV-D68 on non-tumor cells. Such microRNAs include but are not limited to miR-133, miR-206, miR-1, miR-143, miR-145, miR-217, let-7, miR-15, miR-16, etc. (see, for example, PCT International Application WO2008103755A1, US patent application US20160143969A1, or Baohong Zhang et al., Developmental Biology, Volume 302, Issue 1, 1 February 2007, Pages 1-12; all of these documents are incorporated herein by reference).

In certain preferred embodiments, the exogenous nucleic acid comprises a target sequence of one or more (e.g., 2, 3, or 4) microRNA as described above. In certain preferred embodiments, the exogenous nucleic acid comprises a target sequence of miR-133 and/or miR-206. In certain preferred embodiments, the target sequence of miR-133 is shown in SEQ ID NO: 3. In certain preferred embodiments, the target sequence of miR-206 is shown in SEQ ID NO: 4. In some cases, the insertion of the target sequence of miR-133 and/or miR-206 is advantageous. This is because miR-133 and miR-206 are specifically expressed in muscle tissue, so that the tissue tropism of the oncolytic virus can be changed by inserting the target sequence of miR-133 and/or miR-206 into the modified EV-D68, thereby reducing or avoiding damage to normal muscle tissue.

In certain preferred embodiments, the modified EV-D68 comprises at least one insertion of the exogenous nucleic acid as described above and/or at least one mutation in the untranslated region as described above.

In certain preferred embodiments, the genomic sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 13-16. In certain preferred embodiments, the genomic sequence of the modified EV-D68 is selected from the nucleotide sequences as shown in any one of SEQ ID NOs: 13-16.

In certain preferred embodiments, the cDNA sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 8-11. In certain preferred embodiments, the cDNA sequence of the modified EV-D68 is selected from the nucleotide sequences as shown in any one of SEQ ID NOs: 8-11.

In the present invention, the modified EV-D68 can be obtained by reverse genetics technology, and the reverse genetics technology is known in the art, for example, see Yang LS, Li SX, Liu YJ, et al Virus Res, 2015, 210: 165-168; Hou WH, Yang LS, Li SX, et al. Virus Res, 2015, 205: 41-44; which is incorporated herein by reference in its entirety. In such embodiments, the modified EV-D68 is typically obtained by modifying the cDNA of wild-type EV-D68 (e.g., insertion of an exogenous nucleic acid, deletion or mutation of an endogenous gene, or mutation in a non-translated region).

In the present invention, the EV-D68 or a modified form thereof may be pretreated to reduce or eliminate the immune response against the virus in a subject, wherein the pretreatment may comprise: packaging the EV-D68 in a lipidosome or micelle, and/or using a protease (e.g., chymotrypsin or trypsin) to remove the capsid protein of the virus to reduce the humoral and/or cellular immunity against the virus in host.

In the present invention, the EV-D68 or a modified form thereof can be serially passaged for adaptation in tumor cells. In certain preferred embodiments, the tumor cells may be tumor cell lines or tumor cell strains known in the art, or may be tumor cells obtained by surgical resection or clinical isolation from an individual (e.g., a subject) having a tumor. In certain preferred embodiments, the EV-D68 or a modified form thereof is serially passaged for adaptation in tumor cells obtained from an individual (e.g., a subject) having a tumor. In certain preferred embodiments, the tumor cells are obtained by surgical resection or clinical isolation from an individual (e.g., a subject) having a tumor. In certain preferred embodiments, the method for serial passaging for adaptation comprises a plurality of (e.g., at least 5, at least 10, at least 15, at least 20) cycles consisting of the following processes: 1) infecting a target tumor cell with a virus; 2) harvesting the virus in a supernatant; and 3) reinfecting a fresh target tumor cell with the obtained virus.

In certain preferred embodiments, the EV-D68 and modified forms thereof as described above can be used in combination. Therefore, the medicament may comprise one or several of EV-D68 and modified forms thereof.

In certain preferred embodiments, the isolated nucleic acid molecule consists of a genomic sequence or cDNA sequence of EV-D68 or a modified form thereof as described above, or a complementary sequence of the genomic sequence or cDNA sequence. In certain preferred embodiments, the isolated nucleic acid molecule has a genomic sequence of EV-D68 or a modified form thereof as described above. In certain preferred embodiments, the isolated nucleic acid molecule is RNA. In certain preferred embodiments, the isolated nucleic acid molecule has a nucleotide sequence as shown in any one of SEQ ID NOs: 12-16.

In certain preferred embodiments, the isolated nucleic acid molecule is a vector (e.g. cloning vector or expression vector) comprising a genomic sequence or cDNA sequence of EV-D68 or a modified form thereof as described above, or a complementary sequence of the genomic sequence or cDNA sequence. In certain preferred embodiments, the isolated nucleic acid molecule is a vector (e.g., cloning vector or expression vector) comprising a cDNA sequence of EV-D68 or a modified form thereof as described above, or a complementary sequence of the cDNA sequence. In certain preferred embodiments, the isolated nucleic acid molecule is a vector comprising a nucleotide sequence as shown in any one of SEQ ID NOs: 1, 8-11 or a complementary sequence thereof.

In certain preferred embodiments, the isolated nucleic acid molecule comprises the complementary sequence of the genomic sequence of EV-D68 or a modified form thereof as described above. In certain preferred embodiments, the complementary sequence is complementary to a nucleotide sequence selected from:

(1) a nucleotide sequence as shown in SEQ ID NO: 12;

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO: 12;

(3) a nucleotide sequence as shown in any one of SEQ ID NOs: 13-16; and

(4) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in any of SEQ ID NOs: 13-16.

In certain preferred embodiments, the isolated nucleic acid molecule comprises a complementary sequence to the cDNA sequence of EV-D68 or a modified form thereof as described above. In certain preferred embodiments, the complementary sequence is complementary to a nucleotide sequence selected from:

(1) a nucleotide sequence as shown in SEQ ID NO: 1;

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO: 1;

(3) a nucleotide sequence as shown in any one of SEQ ID NOs: 8-11; and

(4) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in any one of SEQ ID NOs: 8-11.

In the present invention, the isolated nucleic acid molecule can be delivered by any means known in the art, for example, a naked nucleic acid molecule (e.g., a naked RNA) can be directly injected, or a non-viral delivery system can be used. The non-viral delivery system can be obtained from a variety of materials well known in the art, including, but not limited to, the materials described in detail in "Yin H, et al. Nat Rev Genet. 2014 Aug; 15(8): 541-55." and "Riley MK, Vermerris W. Nanomaterials (Basel). 2017 Apr 28; 7(5). Pii: E94.", which are incorporated herein by reference in their entirety, such as liposomes, inorganic nanoparticles (such as gold nanoparticles), polymers (such as PEG), and so on.

In certain preferred embodiments, the medicament comprises a therapeutically effective amount of the EV-D68 and/or a modified form thereof as described above, or a therapeutically effective amount of the isolated nucleic acid molecule as described above. In certain preferred embodiments, the medicament may be in any form known in the medical arts. For example, the medicament may be in the form of a tablet, a pill, a suspension, an emulsion, a solution, a gel, a capsule, a powder, a granule, an elixir, a lozenge, a suppository, or an injection (including injection solution, lyophilized powder) and so on. In some embodiments, the medicament is an injection solution or a lyophilized powder.

In certain preferred embodiments, the medicament further comprises a pharmaceutically acceptable carrier or excipient. In certain preferred embodiments, the medicament comprises a stabilizer.

In certain preferred embodiments, the medicament optionally further comprises an additional pharmaceutically active agent. In a preferred embodiment, the additional pharmaceutically active agent is a medicament having antitumor activity, such as an additional oncolytic virus, chemotherapeutic agent or immunotherapeutic agent.

In the present invention, the additional oncolytic virus includes, but is not limited to, herpesvirus, adenovirus, parvovirus, reovirus, Newcastle disease virus, vesicular stomatitis virus, measles virus, or any combination thereof. The chemotherapeutic agent includes but is not limited to 5-fluorouracil, mitomycin, methotrexate, hydroxyurea, cyclophosphamide, dacarbazine, mitoxantrone, anthracyclines (e.g., epirubicin or doxorubicin), etoposide, platinum compounds (e.g., carboplatin or cisplatin), taxanes (e.g., paclitaxel or taxotere), or any combination thereof. The immunotherapeutic agent includes, but is not limited to, immune checkpoint inhibitors (e.g., PD-L1/PD-1 inhibitors or CTLA-4 inhibitors), tumor-specific targeting antibodies (e.g., rituximab or Herceptin) or any combination thereof.

In certain preferred embodiments, the medicament comprises a unit dose of the EV-D68 and/or a modified form thereof as described above, for example comprising at least $1\times10^2$ pfu, at least $1\times10^3$ pfu, at least $1\times10^4$ pfu, $1\times10^5$ pfu, $1\times10^6$ pfu, at least $1\times10^7$ pfu, at least $1\times10^8$ pfu, at least $1\times10^9$ pfu, at least $1\times10^{10}$ pfu, at least $1\times10^{11}$ pfu, at least $1\times10^{12}$ pfu, at least $1\times10^{13}$ pfu, at least $1\times10^{14}$ pfu, or at least $1\times10^{16}$ pfu of the EV-D68 and/or a modified form thereof. In certain preferred embodiments, the medicament comprises $1\times10^2$ pfu to $1\times10^{17}$ pfu of the EV-D68 and/or a modified form thereof as described above.

In certain preferred embodiments, the medicament contains a unit dose of an isolated nucleic acid molecule as described above, such as the nucleic acid molecule containing $3\text{x}10^{10}$ to $3\text{x}10^{14}$ virus genome copies.

In certain preferred embodiments, the medicament may be administered in combination with an additional therapy. This additional therapy may be any therapy known for tumors, such as surgery, chemotherapy, radiation therapy, immunotherapy, hormone therapy or gene therapy. This additional therapy may be administered before, concurrently with, or after the administration of the medicament.

In certain preferred embodiments, the tumor includes, but is not limited to, cervical cancer, ovarian cancer, endometrial cancer, lung cancer, liver cancer, kidney cancer, neuroblastoma, glioma, breast cancer, melanoma, prostate cancer, bladder cancer, pancreatic cancer, gastric cancer, colorectal cancer, esophageal cancer, thyroid cancer, laryngeal cancer, osteosarcoma, hematopoietic malignancy (e.g., lymphoma or leukemia).

In certain preferred embodiments, the subject is a mammal, such as a human.

In another aspect, the invention also relates to use of the EV-D68 and/or a modified form thereof as defined in the first aspect, or the isolated nucleic acid molecule as defined in the first aspect, as a medicament.

<u>Treatment method</u>

In a second aspect, the present invention provides a method for treating a tumor, comprising the step of administering to a subject in need thereof an effective amount of an EV-D68 or a modified form thereof, or an effective amount of an isolated nucleic acid molecule; wherein the isolated nucleic acid molecule comprises a sequence selected from the group consisting of:

(1) a genomic sequence or cDNA sequence of EV-D68 or a modified form thereof; and

(2) a complementary sequence of the genomic sequence or cDNA sequence.

In certain preferred embodiments, EV-D68 is administered to the subject. In certain preferred embodiments, the EV-D68 is wild-type EV-D68. In certain preferred embodiments, the EV-D68 may be a clinical isolate that is isolated from an individual infected with Enterovirus D68.

In certain preferred embodiments, the genomic sequence of the EV-D68 or a modified form thereof has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO: 12. In certain preferred embodiments, the genomic sequence of the EV-D68 or a modified form thereof is a nucleotide sequence as shown in SEQ ID NO: 12.

In certain preferred embodiments, the cDNA sequence of the EV-D68 or a modified form thereof has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO: 1. In certain preferred embodiments, the cDNA sequence of the EV-D68 or a modified form thereof is a nucleotide sequence as shown in SEQ ID NO: 1.

In certain preferred embodiments, a modified form of EV-D68 is administered to the subject. In certain preferred embodiments, as compared to the wild-type EV-D68, the modified form is a modified EV-D68, which has a substitution, insertion, or deletion of one or more nucleotides in the genome.

In certain preferred embodiments, as compared to the wild-type EV-D68, the modified EV-D68 has one or more modifications selected from the following:

(1) one or more mutations in an untranslated region (e.g., 5'UTR or 3'UTR);

(2) an insertion of one or more exogenous nucleic acids;

(3) a deletion or mutation of one or more endogenous genes; and

(4) any combination of the above three items.

In certain preferred embodiments, the modified EV-D68 includes one or more mutations in the 5' untranslated region (5'UTR).

In certain preferred embodiments, the modified EV-D68 has a substitution of all or part of the 5'UTR sequence. In certain preferred embodiments, the internal ribosome entry site (IRES) sequence in the 5'UTR of the modified EV-D68 is replaced with an exogenous IRES sequence, such as the interior ribosome entry site sequence of human rhinovirus 2 (HRV2). In certain preferred embodiments, the internal ribosome entry site sequence of the human rhinovirus 2 (HRV2) is shown in SEQ ID NO: 2.

In certain preferred embodiments, the modified EV-D68 comprises an exogenous nucleic acid.

In certain preferred embodiments, the exogenous nucleic acid encodes a cytokine (e.g., GM-CSF, preferably human GM-CSF), or an antitumor protein or polypeptide (e.g., scFv against PD-1 or PD-L1, preferably scFv against human PD-1 or PD-L1). In certain preferred embodiments, the exogenous nucleic acid is inserted between the 5'UTR gene and the VP4 gene, or between the VP1 gene and the 2A gene of the genome of the modified EV-D68.

In certain preferred embodiments, the exogenous nucleic acid comprises a target sequence of microRNA (miRNA) (e.g., miR-133 or miR-206). In certain preferred embodiments, the target sequence of microRNA is inserted in the 3' untranslated region (3'UTR) of the genome of the modified EV-D68.

In certain preferred embodiments, the exogenous nucleic acid comprises a target sequence of one or more (e.g., 2, 3, or 4) microRNA as described above. In certain preferred embodiments, the exogenous nucleic acid comprises a target sequence of miR-133 and/or miR-206. In certain preferred embodiments, the target sequence of miR-133 is shown in SEQ ID NO: 3. In certain preferred embodiments, the target sequence of miR-206 is shown in SEQ ID NO: 4.

In certain preferred embodiments, the modified EV-D68 comprises at least one insertion of the exogenous nucleic acid as described above and/or at least one mutation in the untranslated region as described above.

In certain preferred embodiments, the genomic sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 13-16. In certain preferred embodiments, the genomic sequence of the modified EV-D68 is selected from the nucleotide sequence as shown in any one of SEQ ID NOs: 13-16.

In certain preferred embodiments, the cDNA sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 8-11. In certain preferred embodiments, the cDNA sequence of the modified EV-D68 is selected from the nucleotide sequence as shown in any one of SEQ ID NOs: 8-11.

In certain preferred embodiments, the EV-D68 and modified forms thereof as described above can be used in combination. Thus, one or more of the EV-D68 and modified forms can be administered to a subject.

In certain preferred embodiments, the isolated nucleic acid molecule as described above is administered to the subject.

In certain preferred embodiments, the isolated nucleic acid molecule consists of the genomic sequence or cDNA sequence of the EV-D68 or a modified form thereof as described above, or the complementary sequence of the genomic sequence or cDNA sequence. In certain preferred embodiments, the isolated nucleic acid molecule has the genomic sequence of the EV-D68 or a modified form thereof as described above. In certain preferred embodiments, the isolated nucleic acid molecule is RNA. In certain preferred embodiments, the isolated nucleic acid molecule has a nucleotide sequence as shown in any one of SEQ ID NOs: 12-16.

In certain preferred embodiments, the isolated nucleic acid molecule is a vector (e.g. cloning vector or expression vector) comprising the genomic sequence or cDNA sequence of EV-D68 or a modified form thereof as described above, or the complementary sequence of the genomic sequence or cDNA sequence. In certain preferred embodiments, the isolated nucleic acid molecule is a vector (e.g., cloning vector or expression vector) comprising the cDNA sequence of EV-D68 or a modified form thereof as described above, or the complementary sequence of the cDNA sequence. In certain preferred embodiments, the isolated nucleic acid molecule is a vector comprising the nucleotide sequence as shown in any one of SEQ ID NOs: 1, 8-11 or the complementary sequence thereof.

In certain preferred embodiments, the isolated nucleic acid molecule comprises the complementary sequence of the genomic sequence of EV-D68 or a modified form thereof as described above. In certain preferred embodiments, the complementary sequence is complementary to a nucleotide sequence selected from:

(1) a nucleotide sequence as shown in SEQ ID NO: 12;

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO: 12;

(3) a nucleotide sequence as shown in any one of SEQ ID NOs: 13-16; and

(4) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence shown in any of SEQ ID NOs: 13-16.

In certain preferred embodiments, the isolated nucleic acid molecule comprises the complementary sequence of the cDNA sequence of EV-D68 or a modified form thereof as described above. In certain preferred embodiments, the complementary sequence is complementary to a nucleotide sequence selected from:

(1) a nucleotide sequence as shown in SEQ ID NO: 1;

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in SEQ ID NO: 1;

(3) a nucleotide sequence as shown in any one of SEQ ID NOs: 8-11; and

(4) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to the nucleotide sequence as shown in any one of SEQ ID NOs: 8-11.

In the present invention, the isolated nucleic acid molecule can be delivered by any means known in the art, for example, a naked nucleic acid molecule (e.g., naked RNA) can be directly injected, or a non-viral delivery system can be used. The non-viral delivery system can be obtained from a variety of materials well known in the art, including, but not limited to, the materials described in detail in "Yin H, et al. Nat Rev Genet. 2014 Aug; 15(8): 541-55." and "Riley MK, Vermerris W. Nanomaterials (Basel). 2017 Apr 28; 7(5). Pii: E94.", which are incorporated herein by reference in their entirety, such as liposomes, inorganic nanoparticles (such as gold nanoparticles), polymers (such as PEG), and so on.

In certain preferred embodiments, the EV-D68 and/or a modified form thereof as described above, or the isolated nucleic acid molecule as described above, can be formulated and administered as a pharmaceutical composition. Such a pharmaceutical composition may comprise a therapeutically effective amount of the EV-D68 and/or a modified form thereof as described above, or a therapeutically effective amount of the isolated nucleic acid molecule as described above. In certain preferred embodiments, the pharmaceutical composition may be in any form known in the medical arts. For example, the pharmaceutical composition may be in the form of a tablet, a pill, a suspension, an emulsion, a solution, a gel, a capsule, a powder, a granule, an elixir, a lozenge, a suppository, or an injection (including injection solution, lyophilized powder) and so on. In some embodiments, the medicament is an injection solution or a lyophilized powder.

In certain preferred embodiments, the pharmaceutical composition further comprises a pharmaceutically acceptable carrier or excipient. In certain preferred embodiments, the pharmaceutical composition comprises a stabilizer.

In the present invention, the EV-D68 and/or a modified form thereof, or the isolated nucleic acid molecule as described above can be administered to a subject by any suitable administration route. In some cases, the route of administration of the EV-D68 and/or a modified form thereof, or the isolated nucleic acid molecules as described above, depends on the location and type of tumor. For example, for a solid tumor that is easily accessible, the virus or nucleic acid molecule is optionally administered by injection directly into the tumor (e.g., intratumoral injection); for a tumor of hematopoietic system, the virus or nucleic acid molecule can be administered by intravenous or other intravascular routes; for a tumor that is not easily accessible in the body (e.g., metastases), the virus or nucleic acid molecule can be administered systematically so that it can run over the whole body and thereby reaching the tumor (e.g., intravenous or intramuscular injection). Optionally, the virus or nucleic acid molecule of the present invention can be administrated via subcutaneous, intraperitoneal, intrathecal (e.g., for brain tumors), topical (e.g., for melanoma), oral (e.g., for oral or esophageal cancer), intranasal or inhalation spray (e.g., for lung cancer) routes and so on. In certain preferred embodiments, the EV-D68 and/or a modified form thereof as described above, or the isolated nucleic acid as described above, can be administered via intradermal, subcutaneous, intramuscular, intravenous, oral routes etc.

In certain preferred embodiments, the method further comprises administering an additional pharmaceutically active agent having antitumor activity. This additional pharmaceutically active agent may be administered before, concurrently with or after the administration of the EV-D68 and/or a modified form thereof, or an isolated nucleic acid molecule as described above.

In certain preferred embodiments, the additional pharmaceutically active agent includes an additional oncolytic virus, chemotherapeutic agent, or immunotherapeutic agent. In the present invention, the additional oncolytic virus includes, but is not limited to, herpesvirus, adenovirus, parvovirus, reovirus, Newcastle disease virus, vesicular stomatitis virus, measles virus, or any combination thereof. The chemotherapeutic agent includes but is not limited to 5-fluorouracil, mitomycin, methotrexate, hydroxyurea, cyclophosphamide, dacarbazine, mitoxantrone, anthracyclines (such as epirubicin or doxorubicin), etoposide, platinum compounds (such as carboplatin or cisplatin), taxanes (such as paclitaxel or taxotere), or any combination thereof. The immunotherapeutic agents include, but are not limited to, immune check point inhibitors (such as PD-L1/PD-1 inhibitors or CTLA-4 inhibitors), tumor-specific targeting antibodies (such as rituximab or Herceptin) or any combination thereof.

In certain preferred embodiments, the EV-D68 and/or a modified form thereof can be administered in any amount from 1 to $1\times10^{15}$ pfu/kg of the subject's body weight, for example, the EV-D68 and/or a modified form thereof is administered in an amount of at least $1\times10^{3}$ pfu/kg, at least $1\times10^{4}$ pfu/kg, $1\times10^{5}$ pfu/kg, $1\times10^{6}$ pfu/kg, at least $1\times10^{7}$ pfu/kg, at least $1\times10^{8}$ pfu/kg, at least $1\times10^{9}$ pfu/kg, at least $1\times10^{10}$ pfu/kg, at least $1\times10^{11}$ pfu/kg, or at least $1\times10^{12}$ pfu/kg of the subject's body weight. In certain preferred embodiments, the isolated nucleic acid molecule as described above can be administered in any amount of $3\times10^{10}$ to $3\times10^{14}$ virus genome copies per kg of the subject's body weight. In certain preferred embodiments, the EV-D68 and/or a modified form thereof or the isolated nucleic acid molecule as described above can be administered 3 times a day, 2 times a day, 1 time a day, once every 2 days or once a week, optionally the above dosage regimen can be repeated weekly or monthly as appropriate.

In certain preferred embodiments, the method further comprises administering an additional therapy. This additional therapy may be any therapy known for tumors, such as surgery, chemotherapy, radiation therapy, immunotherapy, hormone therapy or gene therapy. This additional therapy may be administered before, concurrently with, or after the administration of the method described above.

In certain preferred embodiments, the subject is a mammal, such as a human.

In certain preferred embodiments, the tumor includes, but is not limited to, cervical cancer, ovarian cancer, endometrial cancer, lung cancer, liver cancer, kidney cancer, neuroblastoma, glioma, breast cancer, melanoma, prostate cancer, bladder cancer, pancreatic cancer, gastric cancer, colorectal cancer, esophageal cancer, thyroid cancer, laryngeal cancer, osteosarcoma, hematopoietic malignancy (e.g., lymphoma or leukemia).

Pharmaceutical composition

In a third aspect, the present invention provides a pharmaceutical composition comprising an EV-D68 and/or a modified form thereof as defined in the first or second aspect, or an isolated nucleic acid molecule as defined in the first or second aspect.

In certain preferred embodiments, the pharmaceutical composition comprises the EV-D68 and/or a modified form thereof as defined in the first or second aspect. In certain preferred embodiments, the EV-D68 and/or modified forms thereof may be used in combination. Therefore, the pharmaceutical composition of the present invention may comprise one or several of the EV-D68 and/or modified forms thereof. In certain preferred embodiments, the pharmaceutical composition comprises a unit dose of the EV-D68 and/or a modified form thereof, for example at least $1\times10^{2}$ pfu, at least $1\times10^{3}$ pfu, at least $1\times10^{4}$ pfu, $1\times10^{5}$ pfu, $1\times10^{6}$ pfu, at least $1\times10^{7}$ pfu, at least $1\times10^{8}$ pfu, at least $1\times10^{9}$ pfu, at least $1\times10^{10}$ pfu, at least $1\times10^{11}$ pfu, at least $1\times10^{12}$ pfu, at least $1\times10^{13}$ pfu, at least $1\times10^{14}$ pfu, or at least $1\times10^{16}$ pfu of the EV-D68 and/or a modified form thereof. In certain preferred embodiments, the pharmaceutical composition comprises $1\times10^{2}$ pfu to $1\times10^{17}$ pfu of the EV-D68 and/or a modified form thereof.

In certain preferred embodiments, the pharmaceutical composition comprises an isolated nucleic acid molecule as defined in the first aspect or the second aspect. In certain preferred embodiments, the isolated nucleic acid molecules can be used in combination. Therefore, the pharmaceutical composition of the present invention may include one or several of the isolated nucleic acid molecules. In certain preferred embodiments, the pharmaceutical composition comprises a unit dose of the isolated nucleic acid molecule, for example $3\times10^{10}$ to $3\times10^{14}$ virus genome copies of the isolated nucleic acid molecule.

In certain preferred embodiments, the pharmaceutical composition may be in any form known in the medical arts. For example, the pharmaceutical composition may be in the form of a tablet, a pill, a suspension, an emulsion, a solution, a gel, a capsule, a powder, a granule, an elixir, a lozenge, a suppository, or an injection (including injection solution, lyophilized powder) and so on. In some embodiments, the medicament is an injection solution or a lyophilized powder.

In certain preferred embodiments, the pharmaceutical composition further comprises a pharmaceutically acceptable carrier or excipient. In certain preferred embodiments, the pharmaceutical composition comprises a stabilizer.

In certain preferred embodiments, the pharmaceutical composition optionally further comprises an additional pharmaceutically active agent. In a preferred embodiment, the additional pharmaceutically active agent is a medicament having antitumor activity, such as an additional oncolytic virus, chemotherapeutic agent or immunotherapeutic agent.

In certain preferred embodiments, the pharmaceutical composition is used to treat a tumor in a subject.

In certain preferred embodiments, the subject is a mammal, such as a human.

In certain preferred embodiments, the tumor includes, but is not limited to, cervical cancer, ovarian cancer, endometrial cancer, lung cancer, liver cancer, kidney cancer, neuroblastoma, glioma, breast cancer, melanoma, prostate cancer, bladder cancer, pancreatic cancer, gastric cancer, colorectal cancer, esophageal cancer, thyroid cancer, laryngeal cancer, osteosarcoma, hematopoietic malignancy (e.g., lymphoma or leukemia).

## Modified EV-D68

In a fourth aspect, the present invention provides a modified EV-D68 having a substitution, insertion, or deletion of one or more nucleotides in the genome compared to wild-type EV-D68.

In certain preferred embodiments, the genomic sequence of the wild-type EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from the nucleotide sequence as shown in SEQ ID NO: 12. In certain preferred embodiments, the genomic sequence of the wild-type EV-D68 is a nucleotide sequence as shown in SEQ ID NO: 12.

In certain preferred embodiments, the cDNA sequence of the wild-type EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 1. In certain preferred embodiments, the cDNA sequence of the wild-type EV-D68 is a nucleotide sequence as shown in SEQ ID NO: 1.

In certain preferred embodiments, as compared to the wild-type EV-D68, the modified EV-D68 has one or more modifications selected from the following:

(1) one or more mutations in an untranslated region (e.g., 5'UTR or 3'UTR);

(2) an insertion of one or more exogenous nucleic acids;

(3) a deletion or mutation of one or more endogenous genes; and

(4) any combination of the above three items.

In certain preferred embodiments, the modified EV-D68 includes one or more mutations in the 5' untranslated region (5'UTR).

In certain preferred embodiments, the modified EV-D68 has a substitution of all or part of the 5'UTR sequence. In certain preferred embodiments, the internal ribosome entry site (IRES) sequence in the 5'UTR of the modified EV-D68 is replaced with an exogenous IRES sequence, such as the interior ribosome entry site sequence of human rhinovirus 2 (HRV2). In certain preferred embodiments, the internal ribosome entry site sequence of the human rhinovirus 2 (HRV2) is shown in SEQ ID NO: 2.

In certain preferred embodiments, the modified EV-D68 comprises an exogenous nucleic acid.

In certain preferred embodiments, the exogenous nucleic acid encodes a cytokine (e.g., a GM-CSF, preferably a human GM-CSF), or an antitumor protein or polypeptide (e.g., a scFv against PD-1 or PD-L1, preferably a scFv against human PD-1 or PD-L1). In certain preferred embodiments, the exogenous nucleic acid is inserted between the 5'UTR gene and the VP4 gene, or between the VP1 gene and the 2A gene of the genome of the modified EV-D68.

In certain preferred embodiments, the exogenous nucleic acid comprises a target sequence of microRNA (miRNA) (e.g., miR-133 or miR-206). In certain preferred embodiments, the target sequence of microRNA is inserted in the 3' untranslated region (3'UTR) of the genome of the modified EV-D68.

In certain preferred embodiments, the exogenous nucleic acid comprises a target sequence of one or more (e.g., 2, 3, or 4) microRNA as described above. In certain preferred embodiments, the exogenous nucleic acid comprises a target sequence of miR-133 and/or miR-206. In certain preferred embodiments, the target sequence of miR-133 is shown in SEQ ID NO: 3. In certain preferred embodiments, the target sequence of miR-206 is shown in SEQ ID NO: 4.

In certain preferred embodiments, the modified EV-D68 comprises at least one insertion of the exogenous nucleic acid as described above and/or at least one mutation in the untranslated region as described above.

In certain preferred embodiments, the genomic sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 13-16. In certain preferred embodiments, the genomic sequence of the modified EV-D68 is selected from the nucleotide sequences as shown in any one of SEQ ID NOs: 13-16.

In certain preferred embodiments, the cDNA sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 8-11. In certain preferred embodiments, the cDNA sequence of the modified EV-D68 is selected from the nucleotide sequences as shown in any one of SEQ ID NOs: 8-11.

In the present invention, the modified EV-D68 can be obtained by reverse genetics technology, and the reverse genetics technology is known in the art, for example, see Yang LS, Li SX, Liu YJ, et al Virus Res, 2015, 210: 165-168; Hou WH, Yang LS, Li SX, et al. Virus Res, 2015, 205: 41-44; which are incorporated herein by reference in their entirety. In such embodiments, the modified EV-D68 is typically obtained by modifying the cDNA of wild-type EV-D68 (e.g., insertion of an exogenous nucleic acid, deletion or mutation of an endogenous gene, or mutation in a non-translated region).

In the present invention, the modified EV-D68 may be pretreated to reduce or eliminate the immune response against the virus in a subject, wherein the pretreatment may comprise: packaging the EV-D68 in a lipidosome or micelle, and/or using a protease (e.g., chymotrypsin or trypsin) to remove the capsid protein of the virus to reduce the humoral and/or cellular immunity against the virus in host.

In the present invention, the modified EV-D68 can be serially passaged for adaptation in tumor cells. In certain preferred embodiments, the tumor cells may be tumor cell lines or tumor cell strains known in the art, or may be tumor cells obtained by surgical resection or clinical isolation from an individual (e.g., a subject) having a tumor. In certain preferred embodiments, the modified EV-D68 is serially passaged for adaptation in tumor cells obtained from an individual (e.g., a subject) having a tumor. In certain preferred embodiments, the tumor cells are obtained by surgical resection or clinical isolation from an individual (e.g., a subject) having a tumor. In certain preferred embodiments, the method for serial passaging for adaptation comprises a plurality of (e.g., at least 5, at least 10, at least 15, at least 20) cycles consisting of the following processes: 1) infecting a target tumor cell with a virus; 2) harvesting the virus in a supernatant; and 3) reinfecting a fresh target tumor cell with the obtained virus.

In certain preferred embodiments, the modified EV-D68 is used to treat a tumor in a subject, or to prepare a medicament for treating a tumor in a subject.

In certain preferred embodiments, the tumor includes, but is not limited to, cervical cancer, ovarian cancer, endometrial cancer, lung cancer, liver cancer, kidney cancer, neuroblastoma, glioma, breast cancer, melanoma, Prostate cancer, bladder cancer, pancreatic cancer, gastric cancer, colorectal cancer, esophageal cancer, thyroid cancer, laryngeal cancer, osteosarcoma, hematopoietic malignancy (such as lymphoma or leukemia).

In certain preferred embodiments, the subject is a mammal, such as a human.

In certain preferred embodiments, the modified EV-D68 of the present invention has the internal ribosome entry site (IRES) sequence in the 5'UTR replaced with the internal ribosome entry site sequence of human rhinovirus 2 (HRV2) compared to wild type EV-D68.

In certain preferred embodiments, the modified EV-D68 further comprises an exogenous nucleic acid.

In certain preferred embodiments, the exogenous nucleic acid encodes a cytokine (eg, GM-CSF, preferably human GM-CSF), or an antitumor protein or polypeptide (e.g., a scFv against PD-1 or PD-L1, preferably a scFv against human PD-1 or PD-L1). In certain preferred embodiments, the exogenous nucleic acid is inserted between the 5'UTR and the VP4 gene, or between the VP1 gene and the 2A gene of the genome of the modified EV-D68.

In certain preferred embodiments, the exogenous nucleic acid comprises a target sequence of microRNA (microRNA, miRNA) (eg, miR-133 or miR-206). In certain preferred embodiments, the target sequence of the microRNA is inserted in the 3' untranslated region (3'UTR) of the genome of the modified EV-D68.

In certain preferred embodiments, the exogenous nucleic acid includes a target sequence of one or more (e.g., two, three, or four) microRNAs as described above. In certain preferred embodiments, the exogenous nucleic acid comprises a target sequence of miR-133 and/or miR-206. In certain preferred embodiments, the target sequence of the miR-133 is shown in SEQ ID NO: 3. In certain preferred embodiments, the target sequence of the miR-206 is shown in SEQ ID NO: 4.

In certain preferred embodiments, the modified EV-D68 comprises an insertion of at least one exogenous nucleic acid as described above.

In certain preferred embodiments, the genomic sequence of the modified EV-D68 has at least 70%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide sequence shown in SEQ ID NO: 13. In certain preferred embodiments, the genomic sequence of the modified EV-D68 is a nucleotide sequence as shown in SEQ ID NO: 13.

In certain preferred embodiments, the cDNA sequence of the modified EV-D68 has at least 70%, at least 80%, at least 85%, at least 90%, at least 91%, at least 92%, at least 93%, at least 94%, at least 95%, at least 96%, at least 97%, at least 98%, at least 99%, or 100% sequence identity to the nucleotide sequence shown in SEQ ID NO: 8. In certain preferred embodiments, the cDNA sequence of the modified EV-D68 is a nucleotide sequence as shown in SEQ ID NO: 8.

In certain preferred embodiments, the modified EV-D68 is used to treat a tumor in a subject.

In certain preferred embodiments, the tumor includes, but is not limited to, cervical cancer, ovarian cancer, endometrial cancer, lung cancer, liver cancer, kidney cancer, neuroblastoma, glioma, breast cancer, melanoma, prostate cancer, bladder cancer, pancreatic cancer, gastric cancer, colorectal cancer, esophageal cancer, thyroid cancer, laryngeal cancer, osteosarcoma, hematopoietic malignancy (such as lymphoma or leukemia).

In certain preferred embodiments, the tumor is selected from the group consisting of gastric cancer, endometrial cancer, cervical cancer, and thyroid cancer.

In a fifth aspect, the invention provides an isolated nucleic acid molecule comprising a sequence selected from:

(1) the genomic sequence or cDNA sequence of the modified EV-D68 according to the fourth aspect; and

(2) a complementary sequence of the genomic sequence or cDNA sequence.

In certain preferred embodiments, the isolated nucleic acid molecule consists of the genomic sequence or cDNA sequence of the modified EV-D68 as described above, or the complementary sequence of the genomic sequence or cDNA sequence.

In certain preferred embodiments, the isolated nucleic acid molecule has the genomic sequence of the modified EV-D68 as described above. In certain preferred embodiments, the isolated nucleic acid molecule is RNA. In certain preferred embodiments, the isolated nucleic acid molecule has the nucleotide sequence as shown in any one of SEQ ID NOs: 12-16.

In certain preferred embodiments, the isolated nucleic acid molecule is a vector (e.g. a cloning vector or an expression vector) comprising a genomic sequence or cDNA sequence of EV-D68 or a modified form thereof as described above, or a complementary sequence of the genomic sequence or cDNA sequence. In certain preferred embodiments, the isolated nucleic acid molecule is a vector (e.g., a cloning vector or an expression vector) comprising a cDNA sequence of EV-D68 or a modified form thereof as described above, or a complementary sequence of the cDNA sequence. In certain preferred embodiments, the isolated nucleic acid molecule is a vector (e.g., a cloning vector or an expression vector) comprising a nucleotide sequence as shown in any one of SEQ ID NOs: 1, 8-11 or a complementary sequence thereof.

In certain preferred embodiments, the isolated nucleic acid molecule comprises a complementary sequence of the genomic sequence of the modified EV-D68 as described above. In certain preferred embodiments, the complementary sequence is complementary to a nucleotide sequence selected from:

(1) a nucleotide sequence as shown in any one of SEQ ID NOs: 13-16; and

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in any one of SEQ ID NOs: 13-16.

In certain preferred embodiments, the isolated nucleic acid molecule comprises the complementary sequence of the cDNA sequence of the modified EV-D68 as described above. In certain preferred embodiments, the complementary sequence is complementary to a nucleotide sequence selected from:

(1) a nucleotide sequence as shown in any one of SEQ ID NOs: 8-11; and

(2) a nucleotide sequence having a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in any one of SEQ ID NOs: 8-11.

In certain preferred embodiments, the isolated nucleic acid molecule has a nucleotide sequence as shown in SEQ ID NO: 13, or the isolated nucleic acid molecule is a vector (e.g., a cloning vector or an expression vector) comprising a nucleotide sequence as shown in SEQ ID NO: 8 or a complementary sequence thereof.

In the present invention, the isolated nucleic acid molecule can be delivered by any means known in the art, for example, a naked nucleic acid molecule (e.g., naked RNA) can be directly injected, or a non-viral delivery system can be used. The non-viral delivery system can be obtained from a variety of materials well known in the art, including, but not limited to, the materials described in detail in "Yin H, et al. Nat Rev Genet. 2014 Aug; 15 (8): 541- 55." and "Riley MK, Vermerris W. Nanomaterials (Basel). 2017 Apr 28; 7(5). Pii: E94.", which are incorporated herein by reference in their entirety, such as liposomes, inorganic nanoparticles (such as gold nanoparticles), polymers (such as PEG), and so on.

In certain preferred embodiments, the isolated nucleic acid molecule is used to treat a tumor in a subject, or to prepare a medicament for treating a tumor in a subject.

In certain preferred embodiments, the tumor includes, but is not limited to, cervical cancer, ovarian cancer, endometrial cancer, lung cancer, liver cancer, kidney cancer, neuroblastoma, glioma, breast cancer, melanoma, prostate cancer, bladder cancer, pancreatic cancer, gastric cancer, colorectal cancer, esophageal cancer, thyroid cancer, laryngeal cancer, osteosarcoma, hematopoietic malignancy (such as lymphoma or leukemia).

In certain preferred embodiments, the subject is a mammal, such as a human.

In another aspect, the present invention also relates to a pharmaceutical composition comprising the modified EV-D68 according to the fourth aspect, or the isolated nucleic acid molecule according to the fifth aspect.

In another aspect, the present invention also relates to use of the modified EV-D68 according to the fourth aspect, or the isolated nucleic acid molecule according to the fifth aspect, in treating a tumor in a subject, or in the manufacture of a medicament for treating a tumor in a subject.

In another aspect, the invention also relates to a method for treating a tumor, comprising a step of administering to a subject in need thereof an effective amount of the modified EV-D68 as described in the fourth aspect, or the isolated nucleic acid molecule according to the fifth aspect.

Definition of terms

In the present invention, unless otherwise stated, scientific and technical terms used herein have meanings commonly understood by those skilled in the art. In addition, the laboratory procedures of cell culture, biochemistry, cell biology, nucleic acid chemistry and the like used herein are all routine steps widely used in the corresponding fields. Meanwhile, in order to better understand the present invention, definitions and explanations of related terms are provided below.

As used herein, the term "enterovirus D68 (EV-D68)" refers to one kind of Enterovirus D of the genus *Enteroviruses* of *Picornaviridae* family, the genome of which is a single-stranded positive-sense RNA, consisting of a 5' non-coding region (5'UTR), an open reading frame (ORF), a 3' non-coding region (3'UTR), and a poly(A) tail; wherein its ORF encodes a precursor polyprotein, which can be hydrolyzed and cleaved by its protease to produce structural proteins VP1 to VP4 and non-structural proteins 2A, 2B, 2C, 3A, 3B, 3C and 3D. In order to more clearly describe the present invention, the nucleic acid sequences in the EV-D68 genome corresponding to the above proteins are called VP1 gene, VP2 gene, VP3 gene, VP4 gene, 2A gene, 2B gene, 2C gene, 3A gene, 3B gene, 3C gene, and 3D gene, respectively. In the present invention, the expression "enterovirus D68 (EV-D68)" refers to a wild-type EV-D68, which can be isolated from sources in nature and has not been intentionally modified artificially, examples of which include, but are not limited to, prototype strains AY426531 (CA62-1) and AY426488 (CA62-3), and various clinical isolates (for example, the clinical isolate described in Example 1 of the present invention). The genomic sequence or cDNA sequence of the wild-type EV-D68 is well known in the art and can be found in various public databases (for example, GenBank database, accession number KM881710).

As used herein, the term "modified form" of a virus refers to a modified virus obtained by modifying a wild-type virus, which retains the desired activity (e.g., oncolytic activity) of the wild-type virus. In the present invention, a "modified form" of EV-D68 includes, but is not limited to, a modified EV-D68 virus, the genome sequence of which has a substitution, insertion, or deletion of one or more nucleotides as compared to that of the wild-type EV-D68, and at least retains the oncolytic activity of EV-D68.

As used herein, the term "oncolytic virus" refers to a virus capable of infecting a tumor cell, replicating in the tumor cell, causing the tumor cell death, lysis, or blocking tumor cell growth. Preferably, the virus has minimal toxic effects on a non-tumor cell.

As used herein, the term "tumor-specific" refers to selectively exhibiting a biological function or activity within a tumor cell. For example, in the present invention, when the term "tumor specificity" is used to describe the killing selectivity of a virus, it means that the virus can selectively kill a tumor cell without killing or substantially killing a non-tumor cell, or the virus is more effective in killing a tumor cell than killing a non-tumor cell.

As used herein, the term "oncolytic activity" primarily includes tumor killing activity. When describing the oncolytic activity of a virus, the oncolytic activity of the virus can typically be measured by indicators such as the virus' ability to infect a tumor cell, ability to replicate in a tumor cell, and/or ability to kill a tumor cell. The oncolytic activity of a virus can be measured using any method known in the art. For example, the ability of a virus to infect a tumor cell can be evaluated by measuring the viral dose required to infect a given percentage of tumor cells (for example, 50% of the cells); the ability to replicate in a tumor cell can be evaluated by measuring the growth of the virus in the tumor cell; the ability to kill a tumor cell can be evaluated by monitoring cytopathic effect (CPE) or measuring tumor cell activity.

As used herein, the expression "cDNA sequence of EV-D68" means the DNA form of the viral genomic RNA sequence, which differs from the RNA sequence only in that the ribonucleotides in the RNA sequence are replaced by corresponding deoxyribonucleotides, for example, uracil ribonucleotides (UMP) are replaced by thymine deoxyribonucleotides (dTMP).

As used herein, the term "exogenous nucleic acid" refers to an artificially introduced nucleotide sequence that is foreign to the original sequence. Exogenous nucleic acid includes, but is not limited to, any gene or nucleotide sequence not found in the viral genome. However, in the present invention, it is particularly preferred that the exogenous nucleic acid is composed of at most 1500, such as at most 1200, and at most 1000 nucleotides. In some cases, preferably, the exogenous nucleic acid encodes a protein or polypeptide having antitumor killing activity, such as a cytokine, or an antitumor protein or polypeptide; or, the exogenous nucleic acid comprises a target sequence of microRNA (miRNA). In the present invention, the microRNA is preferably a microRNA having an expression level in a tumor cell significantly lower than that in a normal cell and/or having obvious tissue specificity. Examples of the microRNA include, but are not limited to, miR-122, miR-192, miR-483, etc., which are specifically expressed in liver tissue; miR-1, miR-133a/b, miR-208, etc., which are specifically expressed in heart; miR-192, miR-196a/b, miR-204, miR-215, etc., which are specifically expressed in kidney tissue; miR-133a/b, miR-206, etc., which are specifically expressed in muscle tissue; miR-124a, miR-125a/b, miR-128a/b, miR-138, etc., which are specifically expressed in brain tissue; and miR-34, miR-122a, miR-26a, which are under-expressed in liver tumor tissue; miR-34, which is 1 under-expressed in kidney tumor tissue; miR-143, miR-133a/b, which are under-expressed in bladder tumor tissue; miR-Let-7, miR-29, which are under-expressed in lung tumor tissue; and so on (see, for example, Ruiz AJ and Russell S J. MicroRNAs and oncolytic viruses. [J]. Curr Opin Virol, 2015, 13: 40-48; which is incorporated herein by reference in its entirety).

In the present invention, when the modified EV-D68 comprises the target sequence of microRNA described above, it is regulated by the microRNA in a cell/tissue in which the microRNA is highly expressed or specifically expressed, so that replication of the oncolytic virus is attenuated and even its killing activity is lost, while in a tumor cell/tissue in which the microRNA is under-expressed or even not expressed, the oncolytic virus can normally replicate and thus kill the tumor cell.

As used herein, the term "cytokine" has a meaning well known to those skilled in the art. However, in the present invention, when the oncolytic virus of the present invention is used to treat a tumor, it is particularly preferred that the cytokine is a cytokine that can be used for tumor treatment. Examples of "cytokines" include, but are not limited to, interleukins (e.g., IL-2, IL-12, and IL-15), interferons (e.g., IFN$\alpha$, IFN$\beta$, IFN$\gamma$), tumor necrosis factors (e.g., TNF$\alpha$), and colony-stimulating factors (e.g., GM-CSF), and any combination thereof (see, for example, Ardolino M, Hsu J, Raulet D H. Cytokine treatment in cancer immunotherapy [J]. Oncotarget, 2015, 6 (23): 19346-19347).

As used herein, the term "antitumor protein or polypeptide" refers to a protein or polypeptide having antineoplastic activity, including but not limited to: (1) proteins or polypeptides having toxicity to cells, capable of inhibiting cell proliferation, or inducing apoptosis, examples thereof include, but are not limited to, thymidine kinase TK (TK/GCV), TRAIL, and FasL (see, for example, Candolfi M, King GD, Muhammad AG, et al. Evaluation of proapototic transgenes to use in combination with Flt3L in an immune-stimulatory gene therapy approach for Glioblastoma multiforme (GBM) [J]. FASEB J, 2008, 22: 1077.13); (2) proteins or polypeptides having immunotherapeutic effects, examples thereof include, but are not limited to, single chain antibody (scFv) against cytotoxic T lymphocyte-associated antigen 4 (anti-CTLA-4), against programmed death receptor 1 (anti-PD-1), and against programmed death ligand 1 (anti-PDL-1) (see, for example, Nolan E, Savas P, Policheni AN, et al. Combined immune checkpoint blockade as a therapeutic strategy for BRCA1-mutated breast cancer [J]. Science Trans Med, 2017, 9: eaal 4922; which is incorporated herein by reference in its entirety); (3) proteins or polypeptides that inhibit tumor angiogenesis, examples thereof include, but are not limited to, single-chain antibody (scFv) against vascular endothelial growth factor (anti-VEGF), VEGF-derived polypeptides (e.g., $_D$(LPR), KSVRGKGKGQKRKRKKSRYK, etc.) and ATN-161 (see, for example, Rosca EV, Koskimaki JE, Rivera CG, et al. Anti-angiogenic peptides for cancer therapeutics [J]. Curr Pharm Biotechnol, 2011, 12 (8): 1101-1116; which is incorporated herein by reference in its entirety).

As used herein, the term "scFv" refers to a single polypeptide chain comprising a heavy chain variable region (VH) and a light chain variable region (VL), wherein the VL and VH are linked by a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, No. Volume 113, edited by Roseburg and Moore, Springer-Verlag, New York, pp. 269-315 (1994)). Such scFv molecule may have a general structure: $NH_2$-VL-linker-VH-COOH or $NH_2$-VH-linker-VL-COOH.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two proteins/polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by for example using a computer program such as Align program (DNAstar, Inc.) which is based on the method of Needleman, et al. (J. Mol. Biol. 48:443-453, 1970). The percentage of identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, and with a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and with a gap weight of 16, 14, 12, 10, 8, 6, or 4 and a length weight of 1, 2, 3, 4, 5, or 6.

As used herein, the term "vector" refers to a nucleic acid vehicle into which a polynucleotide can be inserted. When a vector enables expression of a protein encoded by an inserted polynucleotide, the vector is referred to as an expression vector. A vector can be introduced into a host cell by transformation, transduction, or transfection, so that the genetic material elements carried by the vector can be expressed in the host cell. The vector is well known to those skilled in the art and includes, but is not limited to: plasmids; phagemids; cosmids; artificial chromosomes, such as yeast artificial chromosomes (YAC), bacterial artificial chromosomes (BAC) or P1-derived artificial chromosomes (PAC); bacteriophages such as λ-phage or M13 phage and animal viruses. Animal viruses that can be used as vectors include, but are not limited to, retroviruses (including lentiviruses), adenoviruses, adeno-associated viruses, herpesviruses (such as herpes simplex virus), poxviruses, baculoviruses, papillomaviruses, and papovaviruses (such as SV40). A vector may contain a variety of elements that control expression, including, but not limited to, promoter sequences, transcription initiation sequences, enhancer sequences, elements for selection, and reporter genes. In addition, the vector may contain a replication initiation site.

As used herein, the term "internal ribosome entry site (IRES)" refers to a nucleotide sequence located in a messenger RNA (mRNA) sequence that is capable of initiating translation without the need for the 5' cap structure. IRES is usually located in the 5' untranslated region (5'UTR), but may also be located elsewhere in the mRNA.

As used herein, the term "human rhinovirus 2 (HRV2)" refers to a virus of picornaviridae family, the genomic or cDNA sequence of which is well known in the art and can be found in various public databases (e.g., GenBank database, accession number X02316.1).

As used herein, the expression "a nucleic acid molecule comprising a genomic sequence of EV-D68 or a modified form thereof" or "a nucleic acid molecule comprises a genomic sequence of EV-D68 or a modified form thereof" has the meaning commonly understood by those skilled in the art, that is, when the nucleic acid molecule is DNA, the nucleic acid molecule comprises a genomic sequence of EV-D68 or a modified form thereof in form of DNA; when the nucleic acid molecule is RNA, the nucleic acid molecule comprises a genomic sequence of EV-D68 or a modified form thereof.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995), and includes, but is not limited to: pH adjusting agents, surfactants, ionic strength enhancers, agents to maintain osmotic pressure, agents to delay absorption, diluents, adjuvants, preservatives, stabilizers, etc. For example, pH adjusting agents include, but are not limited to, phosphate buffered saline. Surfactants include, but are not limited to, cationic, anionic or non-ionic surfactants, such as Tween-80. Ionic strength enhancers include, but are not limited to, sodium chloride. Agents that maintain osmotic pressure include, but are not limited to, sugar, NaCl, and the like. Agents that delay absorption include, but are not limited to, monostearate and gelatin. Diluents include, but are not limited to, water, aqueous buffers (such as buffered saline), alcohols and polyols (such as glycerol), and the like. Adjuvants include, but are not limited to, aluminum adjuvants (such as aluminum hydroxide), Freund's adjuvants (such as complete Freund's adjuvant), and the like. Preservatives include, but are not limited to, various antibacterial and antifungal agents, such as thimerosal, 2-phenoxyethanol, parabens, trichloro-t-butanol, phenol, sorbic acid, and the like. Stabilizers have the meaning commonly understood by those skilled in the art, which can stabilize the desired activity (such as oncolytic activity) of the active ingredients in the drug, including but not limited to sodium glutamate, gelatin, SPGA, sugars (e.g., sorbitol, mannitol, starch, sucrose, lactose, dextran, or glucose), amino acids (e.g., glutamic acid, glycine), proteins (e.g., dried whey, albumin, or casein) or their degradation products (e.g., lactalbumin hydrolysates).

As used herein, the term "treating" refers to treating or curing a disease (e.g., a tumor), delaying the onset of symptoms of a disease (e.g., a tumor), and/or delaying the development of a disease (e.g., a tumor).

As used herein, the term "effective amount" refers to an amount that can effectively achieve the intended purpose. For example, a therapeutically effective amount can be an amount effective or sufficient to treat or cure a disease (e.g., a tumor), delay the onset of symptoms of a disease (e.g., a tumor), and/or delay the development of a disease (e.g., a tumor). Such an effective amount can be easily determined by a person skilled in the art or a doctor, and can be related to the intended purpose (such as treatment), the general health condition, age, gender, weight of the subject, severity, complications, administration route of the disease to be treated. The determination of such an effective amount is well within the capabilities of those skilled in the art.

As used herein, the term "subject" refers to a mammal, such as a primate mammal, such as a human. In certain embodiments, the subject (e.g., a human) has a tumor, or is at risk for having a tumor.

The beneficial effects of the present invention

Compared with the prior art, the technical solution of the present invention has at least the following beneficial effects:
The inventors of the present application have found for the first time that enterovirus D68 (EV-D68) has broad-spectrum tumor-killing activity. Based on this finding, the present invention further provides an EV-D68-based oncolytic virus, which has a broader-spectrum tumor-killing activity and higher tumor specificity, especially also has a very high killing effect to hematopoietic malignancy (such as lymphoma or leukemia), thus can be used alone for the treatment of tumors, and can also be used as a supplementary method of traditional tumor treatment, or as a treatment in the absence of other treatment methods.

The EV-D68 or a modified form thereof of the present invention has little or no effect on normal cells, and does not induce an immunogenic response against the virus in a subject (for example, a human), and thus can be safely administered to a subject (for example, a human). Therefore, the EV-D68 or a modified form thereof of the present invention has great clinical value.

The embodiments of the present invention will be described in detail below with reference to the drawings and examples, but those skilled in the art will understand that the following drawings and examples are only used to illustrate the present invention, rather than limiting the scope of the present invention. Various objects and advantageous aspects of the present invention will become apparent to those skilled in the art from the following detailed description of drawings and the preferred embodiments.

**Description of the Drawings**

FIG. 1 shows photomicrographs of the in vitro killing tests of the wild-type EV-D68 on human umbilical vein endothelial cell line HUVEC, human esophageal cancer cell line TE-1, human thyroid cancer cell lines SW-579 and TT in Example 2, wherein MOCK represents cells that are not infected with the virus. The results showed that the EV-D68 had a significant oncolytic effect on human tumor cell lines TE-1, SW-579, and TT after 72 hours of infection at a multiplicity of infection (MOI) of 10, but had no effect on HUVEC of human normal cells.

FIG. 2 shows the photos of crystal violet staining of the in vitro killing tests of the wild-type EV-D68 on human liver cancer cell lines HepG2, SMMC7721, BEL7404, BEL7402, and Huh7, human cervical cancer cell lines Hela and Caski, human lung cancer cell lines NCI-H1299 and A549, human foreskin fibroblast cell line HFF-1, human embryonic kidney cell line HEK-293, and differentiated human liver progenitor cell line HepaRG in Example 2, wherein MOCK represents cells that are not infected with the virus. The results showed that the EV-D68 had significant oncolytic effects on human tumor cell lines HepG2, SMMC7721, BEL7404, BEL7402, Huh7, Hela, Caski, NCI-H1299 and A549 after 72 hours of infection at MOIs of 10, 1, and 0.1, but had limited effect on HFF-1, HEK-293 and differentiated HepaRG of human normal cells.

FIG. 3 shows an electrophoresis image of four samples of wild-type EV-D68 virus genomic RNA of the same batch obtained by the in vitro transcription method in Example 2.

FIG. 4 shows the killing effect of the wild-type EV-D68 virus genomic RNA on human cervical cancer tumor cell line Hela in Example 2. The results showed that Hela cells showed obvious CPE after 24 hours of transfection with EV-D68 genomic RNA, and were almost all lysed to death by 48 hours.

FIGs. 5A to 5C show the results of in vivo antitumor experiment of the wild-type EV-D68 in Example 3 on human cervical cancer cell line Hela (A), human glioma cell line U118-MG (B), and human lymphoma cell line Raji (C). The results showed that, in the challenge experimental group, $10^6$ TCID50 per tumor mass of EV-D68 were injected intratumorally every third day. After 5 treatments in total, the growth of tumors formed by subcutaneous inoculation of Hela, U118-MG, or Raji cells in SCID mice significantly slowed down and arrested, and the tumors were even lysed and disappeared. In contrast, the tumors of the negative group (CTRL) without treatment of oncolytic virus maintained the normal growth, and their tumor volumes are significantly larger than those in the challenge group.

FIG. 6 shows the results of toxicity detection of EV-D68-WT in BALB/c mice in Example 4. FIG. 6A shows the survival rates and health scores of 1-day-old BALB/c mice after challenge with EV-D68 at different doses ($10^3$, $10^4$, $10^5$, $10^6$, and $10^7$ TCID50/mouse) by intraperitoneal injection; FIG. 6B shows the survival rates and health scores of BALB/c mice of different ages (1-day-old, 2-day-old, 3-day-old, 7-day-old and 14-day-old) challenged with a very high dose ($10^7$ TCID50/mouse) by intraperitoneal injection. The overall toxicity of EV-D68 to BALB/c mice was relatively weak, and only high doses caused the death of 1-day-old to 3-day-old BALB/c mice, but had no effect on 4- or more-day-old BALB/c mice, indicating that EV-D68 had good safety in vivo.

**Sequence information**

Information of a part of sequences involved in the present invention is provided in Table 1 as below.

Table 1: Sequence description

| SEQ ID NO: | Description |
|---|---|
| 1 | cDNA sequence of wild type EV-D68 (EV-D68-WT) |
| 2 | RNA sequence of the internal ribosome entry site of human rhinovirus 2 (HRV2) |
| 3 | RNA sequence of the target sequence of miR-133 |
| 4 | RNA sequence of the target sequence of miR-206 |
| 5 | RNA sequence of tandem sequence of miR-133 target sequence and miR-206 target sequence |
| 6 | DNA sequence of human granulocyte-macrophage colony-stimulating factor (GM-CSF) gene |
| 7 | DNA sequence of anti-human programmed death receptor 1 single chain antibody (Anti-PD-1 scFv) |
| 8 | cDNA sequence of the modified form of EV-D68 (EV-D68-HRV2) |
| 9 | cDNA sequence of the modified form of EV-D68 (EV-D68-miR133&206T) |
| 10 | cDNA sequence of the modified form of EV-D68 (EV-D68-GM-CSF) |
| 11 | cDNA sequence of the modified form of EV-D68 (EV-D68-Anti-PD1) |
| 12 | Genomic sequence of wild-type EV-D68 (EV-D68-WT) |
| 13 | Genomic sequence of the modified form of EV-D68 (EV-D68-HRV2) |
| 14 | Genomic sequence of the modified form of EV-D68 (EV-D68-miR133 & 206T) |
| 15 | Genomic sequence of the modified form of EV-D68 (EV-D68-GM-CSF) |
| 16 | Genomic sequence of the modified form of EV-D68 (EV-D68-Anti-PD1) |
| 17 | DNA sequence of miR-133 target sequence |
| 18 | DNA sequence of miR-206 target sequence |
| 19 | DNA sequence of tandem sequence of miR-133 target sequence and miR-206 target sequence |
| 20 | DNA sequence of the internal ribosome entry site sequence of human rhinovirus 2 (HRV2) |

**Specific Models for Carrying Out the Invention**

The present invention is now be described with reference to the following examples which are intended to illustrate the present invention (rather than to limit the present invention).

Unless otherwise specified, the molecular biology experimental methods and immunoassays used in the present invention were carried out substantially by referring to the methods described in J. Sambrook et al., Molecular Cloning: A Laboratory Manual, 2nd Edition, Cold Spring Harbor Laboratory Press, 1989, and F. M. Ausubel et al., Short Protocols in Molecular Biology, 3rd Edition, John Wiley & Sons, Inc., 1995; restriction enzymes were used under conditions recommended by the product manufacturer. If the specific conditions were not indicated in the examples, the conventional conditions or the conditions recommended by the manufacturer were used. If the reagents or instruments used were not specified by the manufacturer, they were all conventional products that were commercially available. Those skilled in the art will understand that the examples describe the present invention by way of examples, and are not intended to limit the scope of protection claimed by the present invention. All publications and other references mentioned herein are incorporated by reference in their entirety.

**Example 1: Obtainment and preparation of EV-D68 and its modified form**

1.1 Isolation of enterovirus EV-D68 from patient clinical sample

(1) A throat swab of patient was gained from the Center for Disease Control and Prevention of Xiamen City, China; African green monkey kidney cells (Vero cells; ATCC® Number: CCL-81™) were was kept by the National Institute of Diagnostics and Vaccine Development in Infectious Diseases, Xiamen University, China, and cultured in MEM medium containing 10% fetal bovine serum, as well as glutamine, penicillin and streptomycin.

(2) Sample processing: the throat swab of patient was sufficiently agitated in a sample preservation solution to wash off the virus and virus-containing cells adhering to the swab, and then the sample preservation solution was subjected to a high speed centrifugation at 4000 rpm and 4 °C for 30min;

(3) Inoculation and observation:

A) The Vero cells were plated in a 24-well plate with $1 \times 10^5$ cells/well. The growth medium (MEM medium, containing 10% fetal bovine serum, as well as glutamine, penicillin and streptomycin) was aspirated, and 1 mL of maintenance medium (MEM medium, containing 2% fetal calf serum, as well as glutamine, penicillin and streptomycin) was added in each well. Then except the negative control wells, each well was inoculated with 50 $\mu$L of the sample supernatant, and cultured in an incubator at 37 °C, 5% $CO_2$.

B) The cells were observed under a microscope every day for one week, and the occurrence of specific cytopathic effect (CPE) in the inoculated wells was recorded.

C) If the enterovirus-specific cytopathic effect appeared in the cells in the inoculated wells within 7 days, the cells and supernatant were collected and frozen at -80 °C; if no CPE appeared after 7 days, the cells were subjected to blind passage.

D) If CPE appeared within 6 blind passages, the cells and supernatant were collected and frozen at -80 °C; If CPE did not appear after 6 blind passages, the cells were determined as negative.

(4) Isolation and cloning of viruses:
RT-PCR (Piralla et al., J Clin Microbiol 2015, 53 (5): 1725-1726) and enzyme-linked immunospot method based on specific antibody (Yang et al., Clin Vaccine Immunol 2014, 21 (3): 312 -320; Hou et al., J Virol Methods 2015, 215-216: 56-60) were used to identify the viruses isolated from the clinical sample, and EV-D68 positive cultures were selected and subjected to at least 3 cloning experiments. The virus clones obtained by the limiting dilution method in each experiment were also identified by RT-PCR and ELISPOT, and the EV-D68 positive clones were selected for the next round of cloning. A single EV-D68 strain with strong growth viability was selected as a candidate oncolytic virus strain.

1.2 Rescued strain of enterovirus EV-D68 and its modified form obtained by infectious cloning and reverse genetics technology

This example used wild-type EV-D68 (SEQ ID NO: 1) as an example to show how to obtain EV-D68 and its modified form for the present invention through reverse genetics technology. The specific method was as follows.

(1) Construction of viral infectious clone: the cDNA sequence of wild-type enterovirus EV-D68 (named EV-D68-WT) was shown in SEQ ID NO: 1, and its genomic RNA sequence was SEQ ID NO: 12; or gene insertion or replacement based on the cDNA (SEQ ID NO: 1) of enterovirus EV-D68 was performed, comprising:

Modified form 1: the internal ribosome entry site sequence of wild-type EV-D68 was replaced with the internal ribosome entry site sequence of human rhinovirus 2 (which has a DNA sequence shown in SEQ ID NO: 20) to obtain the cDNA (SEQ ID NO: 8) of the recombinant virus (named as EV-D68-HRV2), which has a genomic RNA sequence shown as SEQ ID NO: 13;

Modified form 2: the tandem sequence (which has a DNA sequence shown in SEQ ID NO: 19) of miR-133 target sequence (which has a DNA sequence shown in SEQ ID NO: 17) and miR-206 target sequence (which has a DNA sequence shown in SEQ ID NO: 18) was inserted between 7293-7294 bp of the 3' untranslated region of the cDNA (SEQ ID NO: 1) of the wild-type EV-D68, to obtain the cDNA (SEQ ID NO : 9) of the recombinant virus (named EV-D68-miR133&206T), which has a genomic RNA sequence shown as SEQ ID NO: 14;

Modified form 3: the human granulocyte-macrophage colony-stimulating factor (GM-CSF) gene (SEQ ID NO: 6) was inserted between the VP1 gene and 2A gene of the cDNA (SEQ ID NO: 1) of wild-type EV-D68 to obtain the cDNA (SEQ ID NO: 10) of the recombinant virus (named EV-D68-GM-CSF), which has a genomic RNA sequence shown as SEQ ID NO: 15;

Modified form 4: the sequence (SEQ ID NO: 7) encoding the single chain antibody against human programmed death receptor 1 (Anti-PD-1 scFv) was inserted between the VP1 gene and 2A gene of the cDNA (SEQ ID NO: 1) of wild-type EV-D68 to obtain the cDNA (SEQ ID NO: 11) of the recombinant virus (named EV-D68-Anti-PD-1), which has a genomic RNA sequence shown as SEQ ID NO: 16.

Then, the cDNA sequences (SEQ ID NO: 1, 8-11) of the above five oncolytic viruses were sent to the gene synthesis company (Shanghai Biotech Engineering Co., Ltd.) for full gene synthesis, and ligated into the pSVA plasmid (Hou et al. Virus Res 2015, 205: 41-44; Yang et al., Virus Res 2015, 210: 165-168) to obtain the infectious cloning plasmids of enterovirus EV-D68 or modified forms thereof (i.e., EV-D68-WT, EV-D68-HRV2, EV-D68-miR133&206T, EV-D68-GM-CSF and EV-D68-Anti-PD-1).

(2) Plasmid mini-kit and *E coli.* DH5α competent cells were purchased from Beijing Tiangen Biochemical Technology Co., Ltd.; Hela cells (ATCC® Number: CCL-2™) and human rhabdomyosarcoma cells (RD cells; ATCC® Number: CCL-136™) were kept by National Institute of Diagnostics and Vaccine Development in Infectious Diseases, Xiamen University, China, and were cultured with DMEM and MEM media respectively, in which 10% fetal bovine serum as well as glutamine, penicillin and streptomycin were added; transfection reagents Lipofactamine2000 and Opti-MEM were purchased from Thermo Fisher Scientific Company.

(3) The infectious cloning plasmids containing the cDNA sequences of the above five oncolytic viruses were transformed into *E coli* DH5α competent cells, the monoclonal strains were picked out and shaken after the outgrowth of clones, and the plasmids were extracted using the plasmid mini-kit, and then sent to the company (Shanghai Biotech Engineering Co., Ltd.) for sequencing analysis.

(4) The infectious cloning plasmids with correct sequence and the helper plasmid pAR3126 were co-transfected into the cells to rescue virus (Hou et al. Virus Res 2015, 205: 41-44; Yang et al. Virus Res 2015, 210: 165-168). Hela cells were first transfected according to the instructions of the transfection reagent; then observed under a microscope. When CPE appeared in Hela cells, the cells and culture supernatant were harvested, and inoculated with RD cells followed by passaging and culturing, thereby obtaining the candidate strain of oncolytic virus.

**Example 2: In vitro antitumor experiment of EV-D68 and modified form thereof**

2.1 Viruses and cell lines as used

(1) Viruses: this example used EV-D68-WT (SEQ ID NO: 12), EV-D68-HRV2 (SEQ ID NO: 13), EV-D68-miR133&206T (SEQ ID NO: 14), EV-D68-GM-CSF (SEQ ID NO: 15) and EV-D68-Anti-PD-1 (SEQ ID NO: 16) as provided in Example 1.

(2) Cell lines: human rhabdomyosarcoma cell RD (ATCC® Number: CCL-136™); human cervical cancer cell lines Hela (ATCC® Number: CCL-2™), SiHa (ATCC® Number: HTB-35™), Caski (ATCC® Number: CRL-1550™) and C-33A (ATCC® Number: HTB-31™); human ovarian cancer cell lines SKOV-3/TR (drug-resistant strain of SKOV-3), SKOV-3 (ATCC® Number: HTB-77™) and Caov3 (ATCC® Number: HTB-75™); human endometrial cancer cell lines Hec-1-A (ATCC® Number: HTB-112™), Hec-1-B (ATCC® Number: HTB-113™) and Ishikawa (ECACC No. 99040201); human lung cancer cell lines SPC-A-1 (CCTCC Deposit Number: GDC050), NCI-H1299 (ATCC® Number: CRL-5803™), NCI-H1417 (ATCC® Number: CRL-5869™), NCI-H1703 (ATCC® Number: CRL-5889™), NCI-H1975 (ATCC® Number: CRL-5908™), A549 (ATCC® Number: CCL-185™), NCI-H661 (ATCC® Number:

HTB-183™), EBC-1 (Thermo Fisher Scientific, Catalog #: 11875101), and DMS114 (ATCC® Number: CRL-2066™); human liver cancer cell lines MHCC97H (purchased from the Institute of Liver Cancer, Fudan University), C3A (ATCC® Number: CRL-10741™), Hep3B (ATCC® Number: HB-8064™), HepG2 (ATCC® Number: HB-8065™), SMMC7721 (purchased from the Basic Medical Cell Center of the Institute of Basic Medical Sciences, Chinese Academy of Medical Sciences, number: 3111C0001CCC000087), BEL7402 (CCTCC Deposit Number: GDC035), BEL7404 (purchased from the Cell Resource Center, Shanghai Institutes of Biological Sciences, Chinese Academy of Sciences, number: 3131C0001000700064), Huh7 (CCTCC Deposit Number: GDC134), PLC/PRF/5 (ATCC® Number: CRL-8024™) and SK-Hep-1 (ATCC® Number: HTB-52™); human kidney cancer cell lines A-498 (ATCC® Number: HTB-44™), 786-O (ATCC® Number: CRL-1932™) and Caki-1 (ATCC® Number: HTB-46™); human neuroblastoma cell lines SH-SY5Y (ATCC® Number: CRL-2266™) and SK-N-BE (2) (ATCC® Number: CRL-2271™); human glioma cell lines U87-MG (ATCC® Number: HTB-14™) and U118-MG (ATCC® Number: HTB-15™); human breast cancer cell lines MCF-7 (ATCC® Number: HTB-22™), BcaP37 (CCTCC Deposit Number: GDC206), BT-474 (ATCC® Number: HTB-20™), MDA-MB-231 (ATCC® Number: CRM-HTB-26™) and MDA-MB-453 (ATCC® Number: HTB-131™); human melanoma cell lines A-375 (ATCC® Number: CRL-1619™), SK-MEL-1 (ATCC® Number: HTB-67™) and MeWo (ATCC® Number: HTB-65™); human prostate cancer cell lines PC-3 (ATCC® Number: CRL-1435™), LNCap (ATCC® Number: CRL-1740™) and DU145 (ATCC® Number: HTB-81™); human bladder cancer cell lines J82 (ATCC® Number: HTB-1™) and 5637 (ATCC® Number: HTB-9™); human pancreatic cancer cell lines Capan-2 (ATCC® Number: HTB-80™), HPAF-2 (ATCC® Number: CRL-1997™), and PANC-1 (ATCC® Number: CRL-1469™); human gastric cancer cell lines AGS (ATCC® Number: CRL-1739™), SGC7901 (CCTCC Deposit Number: GDC150), BGC823 (CCTCC Deposit Number: GDC151), and NCI-N87 (ATCC® Number: CRL-5822™ ); human colorectal cancer cell lines DLD-1 (ATCC® Number: CCL-221™), SW1116 (ATCC® Number: CCL-233™), SW480 (ATCC® Number: CCL-228™), HCT-116 (ATCC® Number: CCL-247™) and HT-29 (ATCC® Number: HTB-38™); human esophageal cancer cell line TE-1 (purchased from the Cell Resource Center, Shanghai Institutes of Biological Sciences, Chinese Academy of Sciences, No. 3131C0001000700089); human thyroid cancer SW-579 (ATCC® Number: HTB-107™) and TT (ATCC® Number: CRL-1803™); human laryngeal cancer Hep-2 (ATCC® Number: CCL-23™); osteosarcoma 143B (ATCC® Number: CRL-8303™) and U2OS (ATCC® Number: HTB-96™); human lymphoma and leukemia cell lines K562 (ATCC® Number: CCL-243™), U937 (ATCC® Number: CRL-1593.2™), THP-1 (ATCC® Number: TIB-202™), Raji (ATCC® Number: CCL-86™), Daudi (ATCC® Number: CCL-213™), Jurkat (ATCC® Number: TIB-152™) and MT-4 (obtained from the National Institutes of Health, USA); human normal cell lines include: human embryo lung fibroblast cell line MRC-5 (ATCC® Number: CCL-171™), human embryonic kidney cell line HEK-293 (ATCC® Number: CRL-1573™), human foreskin fibroblast cell line HFF-1 (ATCC® Number: SCRC-1041™), human skin keratinocyte cell line HaCat (CCTCC Deposit Number: GDC106), human prostate stromal cell line WPMY-1 (ATCC® Number: CRL-2854™), human umbilical vein endothelial cell line HUVEC (Thermo Fisher Scientific, Catalog #: C01510C), and differentiated human liver progenitor cell line HepaRG (with characteristics of primary hepatocytes; Thermo Fisher Scientific, Catalog #: HPRGC10). The above cells were kept by National Institute of Diagnostics and Vaccine Development in Infectious Diseases, Xiamen University, China. HepaRG cells were cultured in WME medium (added with 1.5% DMSO), AGS and TT were cultured with F-12K medium, SW-579 was cultured with L-15 medium, SH-SY5Y and SK-N-BE (2) were cultured with DMEM:F12 (1:1) medium, RD, C-33A, EBC-1, J82, SK-Hep-1, SK-MEL-1 and DU145 were cultured with MEM medium, K562, U937, THP-1, Raji, Daudi, Jurkat, MT-4, 5637, 786-O, TE-1, Caski, NCI-H1417, NCI-H1703, NCI-H1975, NCI-H661, SGC7901, BGC823, DLD-1, SW1116, Hep-2, and LNCap were cultured with RPMI-1640 medium, other cells were cultured with DMEM medium. All the mediums mentioned above were supplemented with 10% fetal bovine serum, glutamine and penicillin-streptomycin. All the above cells were cultured under the standard conditions of 37 °C and 5% $CO_2$.

2.2 Virus culture

RD cells were evenly plated on 10 cm cell culture plates, and the culture conditions included MEM medium containing 10% fetal bovine serum and glutamine, penicillin and streptomycin, 37 °C, 5% $CO_2$, and saturated humidity. When the cell confluence reached 90% or more, the cell culture medium was replaced with serum-free MEM medium, and each plate was inoculated with $10^7$ TCID50 of EV-D68-WT, EV-D68-HRV2, EV-D68-miR133&206T, EV-D68-GM-CSF or EV-D68-Anti-PD-1, the culture environment was changed to 33 °C, 5% $CO_2$, saturated humidity. After 24 hours, the EV-D68 or its modified form proliferated in RD cells and caused CPE in cells. When more than 90% of the cells turned contracted and rounded, showed increased graininess, and became detached and lysed, the cells and culture supernatants thereof were harvested. After freeze-thawing for three cycles, the culture supernatant was collected and centrifuged to remove cell debris, wherein the centrifuge conditions were 4000 rpm, 10min, 4 °C. Finally, the supernatant was filtered with a 0.22 μm disposable filter (Millipore Company) to remove impurities such as cell debris.

2.3 Determination of virus titer

The RD cells were plated in a 96-well plate with a cell density of $10^4$ cells/well. After the cells adhered, the virus solution obtained in Example 2.2 was diluted 10-fold with serum-free MEM medium from the first 10-fold dilution. 50 μl of the dilution of virus was added to the wells with cells. After 7 days, the wells where CPE appeared were monitored and recorded, followed by calculation using Karber method, in which the calculation formula was $lg^{TCID50}$ = L - D (S - 0.5), L: logarithm of the highest dilution, D: difference between the logarithms of dilutions, S: sum of proportions of positive wells. The unit of TCID50 thus calculated was TCID50/50μl, which should be converted to TCID50/ml.

2.4 In vitro antitumor experiment of viruses

Human tumor cells and normal cells were inoculated into 96-well plates at $10^4$ cells/well. After the cells adhered, the medium in each well was replaced with the corresponding cell culture medium without serum, and viruses were inoculated at an MOI of 0.1, 1, 10 or 100. Subsequently, CPE of the cells were monitored daily by a microscope.

FIG. 1 shows micrographs of the human umbilical vein endothelial cell line HUVEC, the human esophageal cancer cell line TE-1, and the human thyroid cancer cell line SW-579 and TT, which were not infected with viruses (negative control group, Mock) or which were treated with EV-D68-WT at MOI = 10 for 72 hours. The results showed that after 72 hours of infection at a multiplicity of infection (MOI) of 10, a significant reduction in the number of the tumor cells, marked shrinking and lysis and the like, were detected in the virus-infected groups; while as compared to the non-tumor cells in the Mock group, the non-tumor cells infected with the viruses showed almost no change in cell morphology. The above results demonstrated that EV-D68 had significant oncolytic effects on human tumor cell lines TE-1, SW-579 and TT, but did not have any effect on non-tumor cells HUVEC.

After 72 hours of virus infection and culture, the cell survival rate was detected using Cell Counting Kit-8 (CCK-8 kit; Shanghai Biyuntian Biotechnology Co., Ltd.) and crystal violet staining method (only for adherent cells), and the specific method was as follows:

(1) Cell survival rate detected by CCK8 method

For adherent cells, the original medium in a 96-well cell culture plate was directly discarded; for suspension cells, the original medium in a 96-well cell culture plate was carefully discarded after centrifugation; and then 100 μl of fresh serum-free medium was added per well. 10 μl of CCK-8 solution was added to each of the wells inoculated with cells, and an equal amount of CCK-8 solution was also added to the blank culture medium as a negative control, followed by incubation at 37 °C in a cell culture incubator for 0.5-3 hours. The absorbance was detected at 450 nm using a microplate reader at 0.5, 1, 2, 3 hours, respectively, and the time point where the absorbance was within a suitable range was selected as a reference for cell survival rate. The CCK-8 test results of EV-D68-WT for each kind of cells were shown in Table 2, where "-" indicated that the cell survival rate after virus treatment was not significantly different from that of the MOCK group; "+" indicated that after virus treatment, the cell number was reduced, the survival rate was still greater than 50% but was significantly different from that of the MOCK group; "++" indicated that the cell survival rate after virus treatment was less than 50%, and was significantly different from that of the MOCK group.

The calculation of cell survival rate was:

$$Cell_survival_rate(\%) = \frac{(reading_of_test_group - reading_of_negative_group)}{(reading_of_positive_group - reading_of_negative_group)} \times 100\% .$$

(2) Cell survival rate detected by crystal violet staining method (only for adherent cells)

After the cells were infected with viruses for 3 days, the culture supernatant in the 96-well cell culture plate was discarded, 100 μl of methanol was added to each well, followed by fixation in the dark for 15 min. Crystal violet powder (Shanghai Biotech Biotechnology Co., Ltd.) was weighed, and formulated as 2% (w/v) crystal violet methanol solution, which was stored at 4 °C. An appropriate amount of 2% crystal violet methanol solution was taken and formulated with PBS solution to prepare 0.2% crystal violet working solution. After fixation for 15 minutes, the methanol fixation solution in the 96-well cell culture plate was discarded, and 100μl of the crystal violet working solution was added to the plate and staining was performed for 30min. After the crystal violet staining solution was discarded, PBS solution was used for washing for 3 to 5 times, until the excess staining solution was washed off, and air-drying was performed. ImmunSpot @ S5 UV Analyzer (Cellular Technology Limited, USA) was used for photographing. FIG. 2 showed the crystal violet staining results of the human liver cancer cell lines HepG2, SMMC7721, BEL7404, BEL7402 and Huh7, the human cervical cancer cell lines Hela and Caski, the human lung cancer cell lines NCI-H1299 and A549, the human foreskin fibroblast cell line HFF-1, the human embryonic kidney cell line HEK-293, and the differentiated human hepatic progenitor cell line HepaRG in the control group (MOCK) and in the experimental groups (infected for 72 hours with EV-D68-WT at MOIs of 0.1, 1, and 10, respectively). As shown in the results, after 72 hours of infection at MOIs of 10, 1, and 0.1, the tumor cells in the experimental groups were significantly reduced as compared to the control group (MOCK) without addition of virus; while the number of non-tumor cells showed no significant change. The above results indicated that the EV-D68-WT had significant oncolytic effects on human tumor cell lines HepG2, SMMC7721, BEL7404, BEL7402, Huh7, Hela, Caski, NCI-H1299 and A549, but had no significant effect on non-tumor cell lines HFF-1, HEK-293 and the differentiated HepaRG.

Table 2: Results of in vitro antitumor experiment of wild-type enterovirus EV-D68

| Multiplicity of infection MOI / Cell Line | 0.1 | 1 | 10 | 100 |
|---|---|---|---|---|
| RD | ++ | ++ | ++ | ++ |
| Hela | ++ | ++ | ++ | ++ |
| SiHa | - | - | ++ | ++ |
| Caski | - | + | ++ | ++ |
| C-33A | - | ++ | ++ | ++ |
| SKOV-3/TR | - | - | - | + |
| SKOV-3 | - | - | ++ | ++ |
| Caov3 | + | ++ | ++ | ++ |
| Hec-1-A | - | - | - | ++ |
| Hec-1-B | - | + | ++ | ++ |
| Ishikawa | - | - | ++ | ++ |
| SPC-A-1 | - | + | ++ | ++ |
| NCI-H1299 | - | ++ | ++ | ++ |
| NCI-H1417 | - | - | - | + |
| NCI-H1703 | - | - | - | + |
| NCI-H1975 | - | ++ | ++ | ++ |
| A549 | + | ++ | ++ | ++ |
| NCI-H661 | - | - | + | ++ |
| EBC-1 | - | - | + | ++ |
| DMS114 | ++ | ++ | ++ | ++ |

| | | | | |
|---|---|---|---|---|
| MHCC97H | + | ++ | ++ | ++ |
| C3A | ++ | ++ | ++ | ++ |
| Hep3B | - | + | + | ++ |
| HepG2 | - | ++ | ++ | ++ |
| SMMC7721 | + | ++ | ++ | ++ |
| BEL7402 | ++ | ++ | ++ | ++ |
| BEL7404 | + | ++ | ++ | ++ |
| Huh7 | ++ | ++ | ++ | ++ |
| PLC/PRF/5 | - | + | ++ | ++ |
| SK-Hep-1 | - | - | + | ++ |
| A-498 | + | ++ | ++ | ++ |
| 786-O | - | - | + | ++ |
| Caki-1 | ++ | ++ | ++ | ++ |
| SH-SY5Y | - | + | ++ | ++ |
| SK-N-BE(2) | - | - | - | + |
| U87-MG | + | ++ | ++ | ++ |
| U118-MG | ++ | ++ | ++ | ++ |
| MCF-7 | - | - | - | + |
| BcaP37 | - | ++ | ++ | ++ |
| BT-474 | - | - | - | + |
| MDA-MB-231 | ++ | ++ | ++ | ++ |
| MDA-MB-453 | - | - | + | ++ |
| A-375 | - | + | ++ | ++ |
| SK-MEL-1 | + | ++ | ++ | ++ |
| MeWo | - | + | ++ | ++ |
| PC-3 | ++ | ++ | ++ | ++ |
| LNCap | - | + | ++ | ++ |
| DU145 | ++ | ++ | ++ | ++ |
| J82 | - | + | ++ | ++ |
| 5637 | - | - | - | + |
| Capan-2 | - | - | + | ++ |
| HPAF-2 | - | + | + | ++ |
| PANC-1 | - | ++ | ++ | ++ |
| AGS | - | - | + | ++ |
| SGC7901 | - | - | - | + |
| BGC823 | - | + | + | ++ |
| NCI-N87 | - | + | ++ | ++ |
| DLD-1 | - | - | - | + |
| SW1116 | + | + | ++ | ++ |
| SW480 | - | + | ++ | ++ |
| HCT-116 | - | - | + | ++ |
| HT-29 | + | ++ | ++ | ++ |
| TE-1 | - | + | ++ | ++ |
| SW-579 | - | - | ++ | ++ |
| TT | - | - | ++ | ++ |
| Hep-2 | - | + | ++ | ++ |
| 143B | - | - | - | + |
| U2OS | + | + | ++ | ++ |
| K562 | - | + | + | ++ |
| U937 | - | - | + | ++ |
| THP-1 | + | ++ | ++ | ++ |
| Raji | ++ | ++ | ++ | ++ |
| Daudi | ++ | ++ | ++ | ++ |
| Jurkat | ++ | ++ | ++ | ++ |
| MT-4 | ++ | ++ | ++ | ++ |

| | | | | |
|---|---|---|---|---|
| MRC-5 | - | - | + | ++ |
| HEK-293 | - | - | - | - |
| HFF-1 | - | - | + | + |
| HaCat | - | - | - | - |
| WPMY-1 | - | - | - | - |
| HUVEC | - | - | - | - |
| HepaRG | - | - | - | + |

Note: "-" indicated that there was no significant difference in cell survival rate between virus treatment group and MOCK group; "+" indicated that after virus treatment, the number of cells was reduced, the survival rate was greater than 50% but was significantly different from that of MOCK group; "++" indicated that the cell survival rate after virus treatment was less than 50%, and was significantly different from that of the MOCK group.

It could be known from Table 2 that the wild-type enterovirus EV-D68 had a killing effect on the tested tumor cells, and therefore had a broad-spectrum anti-tumor activity. In particular, the virus had significant killing effects on liver cancer cell lines, glioma cell lines, prostate cancer cell lines, leukemia and lymphoma cell lines. On the other hand, the virus had little or no toxicity to the non-tumor cell lines tested, except that it was significantly toxic to human embryonic lung fibroblast MRC-5 at higher MOIs.

In addition, in vitro antitumor experiments of EV-D68-HRV2, EV-D68-miR133&206T, EV-D68-GM-CSF and EV-D68-Anti-PD-1 showed that the four modified EV-D68s retained the broad-spectrum killing effect of the wild-type enterovirus EV-D68 on the tested tumor cells, and substantially retained the significant killing effect on the tested tumor cells of human hepatocellular carcinoma cell line, prostate cancer cell line, leukemia and lymphoma cell lines, wherein the CCK-8 test results of oncolytic effect of the four modified EV-D68s on cervical cancer cell line Hela, glioma cell line U118-MG, liver cancer cell line Huh7, prostate cancer cell line PC-3, and lymphoma cell line Raji were shown in Table 3.

Table 3: Results of in vitro antitumor experiment of EV-D68-HRV2, EV-D68-miR133&206T, EV-D68-GM-CSF and EV-D68-Anti-PD-1

| Cell Lines \ Multiplicity of infection MOI | | 0.1 | 1 | 10 | 100 |
|---|---|---|---|---|---|
| EV-D68-HRV2 | Hela | + | + | ++ | ++ |
| | U118-MG | + | ++ | ++ | ++ |
| | Huh7 | ++ | ++ | ++ | ++ |
| | PC-3 | ++ | ++ | ++ | ++ |
| | Raji | - | + | + | ++ |
| EV-D68-miR133&206T | Hela | ++ | ++ | ++ | ++ |
| | U118-MG | ++ | ++ | ++ | ++ |

| | | | | | |
|---|---|---|---|---|---|
| | Huh7 | ++ | ++ | ++ | ++ |
| | PC-3 | ++ | ++ | ++ | ++ |
| | Raji | ++ | ++ | ++ | ++ |
| EV-D68-GM-CSF | Hela | ++ | ++ | ++ | ++ |
| | U118-MG | ++ | ++ | ++ | ++ |
| | Huh7 | ++ | ++ | ++ | ++ |
| | PC-3 | ++ | ++ | ++ | ++ |
| | Raji | ++ | ++ | ++ | ++ |
| EV-D68-Anti-PD-1 | Hela | ++ | ++ | ++ | ++ |
| | U118-MG | ++ | ++ | ++ | ++ |
| | Huh7 | ++ | ++ | ++ | ++ |
| | PC-3 | ++ | ++ | ++ | ++ |
| | Raji | ++ | ++ | ++ | ++ |

Note: "-" indicated that there was no significant difference in cell survival rate between virus treatment group and MOCK group; "+" indicated that after virus treatment, the number of cells was reduced, the survival rate was greater than 50% but was significantly different from that of MOCK group; "++" indicated that the cell survival rate after virus treatment was less than 50%, and was significantly different from that of the MOCK group.

In addition, the inventors unexpectedly found that EV-D68-HRV2 exhibited significantly improved killing activity on some tumors compared to EV-D68-WT, wherein the CCK-8 test results of the oncolytic activity of the human gastric cancer cell line AGS, the human endometrial cancer cell lines HEC-1-A and Ishikawa, the human cervical cancer cell line C-33A, and the human thyroid cancer cell line SW579 were shown in Table 4.

Table 4: Comparison of the results of in vitro oncolytic experiment of EV-D68-WT and EV-D68-HRV2 on some tumor cells

| Cell Line \ MOI | | 0.01 | 0.1 | 1 | 10 |
|---|---|---|---|---|---|
| EV-D68-WT | AGS | - | - | - | + |
| | HEC-1-A | - | - | - | - |
| | Ishikawa | - | - | - | ++ |
| | C-33A | - | - | ++ | ++ |
| | SW579 | - | - | - | ++ |
| EV-D68-HRV2 | AGS | ++ | ++ | ++ | ++ |
| | HEC-1-A | - | + | ++ | ++ |
| | Ishikawa | ++ | ++ | ++ | ++ |
| | C-33A | ++ | ++ | ++ | ++ |
| | SW579 | ++ | ++ | ++ | ++ |

Note: "-" indicated that there was no significant difference in cell survival rate between virus treatment group and MOCK group; "+" indicated that after virus treatment, the number of cells was reduced, the survival rate was greater than 50% but was significantly different from that of MOCK group; "++" indicated that the cell survival rate after virus treatment was less than 50%, and was significantly different from that of the MOCK group.

2.5 Serial passaging of EV-D68 for adaptation

In this example, EV-D68 was serially passaged for adaptation in a certain type of tumor cell to obtain a virus strain with enhanced killing activity to the tumor cell.

The wild-type enterovirus EV-D68 was serially passaged for adaptation in the human cervical cancer cell line SiHa, human ovarian cancer cell line SKOV-3, human liver cancer cell line SK-hep-1, human pancreatic cancer cell line Capan-2, human gastric cancer cell line AGS or human colorectal cancer cell line HCT-116 on which the oncolytic effect of EV-D68 was not very significant, and the specific method was as follows:
One kind of the above tumor cells was evenly plated on a 10 cm cell culture plate, and the culture conditions included a corresponding cell culture media containing 10% fetal bovine serum and glutamine, penicillin and streptomycin, 37 °C, 5% $CO_2$, and saturated humidity. When the cell confluence reached 90% or more, the cell culture medium was replaced with serum-free cell culture medium, each plate was inoculated with $10^7$ TCID50 of EV-D68, the culture environment was changed to 33 °C, 5% $CO_2$, saturated humidity. When EV-D68 proliferated in tumor cells and caused CPE in the cells (after infection for up to 3 days), the cells and their culture supernatant were harvested. After freeze-thawing for three cycles, centrifugation was performed at 4 °C, 4000 rpm for 10 min. The centrifugation supernatant was taken and added onto new tumor cells with a cell confluence of more than 90% to complete one round of virus passage. The passage was repeated for more than 10 times, and a part of the virus solution was taken for virus titer detection in RD cells in each round of passage, and the specific method referred to Example 2.3. Generally, the virus replication ability would increase with the generation, and when a relatively high infectious titer was reached and the virus replication was stable in the tumor cell, the adapted strain of EV-D68 for the tumor cell was obtained.

Subsequently, by the in vitro antitumor experimental method described in Example 2.4, the human tumor cell SiHa, SKOV-3, SK-hep-1, Capan-2, AGS, or HCT-116 was inoculated to a 96-well plate at $10^4$ cells/well. After the cells adhered, the medium in each well was replaced with the corresponding culture medium free of serum, followed by incubation at 37 °C for 30 min, and then the serially passaged EV-D68 virus strains (viral titers of which were detected on RD cells) adapted for each of the above kinds of cells at MOIs of 0.1, 1, 10, and 100 were inoculated. Subsequently, CPE of the cells were monitored daily by a microscope, and the cell survival rate was detected using CCK-8 method 72 hours after the infection and culture of viruses.

The results were shown in Table 5, in which after serial passaging of the wild-type enterovirus EV-D68 in a certain kind of tumor cells on which EV-D68 had poor oncolytic effect, the killing activity thereof on the tumor cells was significantly enhanced, indicating that the serial passaging method could be used to obtain an EV-D68 adapted strain with enhanced oncolytic effect on the tumor cells.

Table 5: Results of in vitro killing experiment of EV-D68 on a tumor cell after serial passaging for adaptation in the tumor cell

| Cell Line | 0.1 | 1 | 10 | 100 |
|---|---|---|---|---|
| SiHa | + | ++ | ++ | ++ |
| SKOV-3 | - | ++ | ++ | ++ |
| SK-hep-1 | - | ++ | ++ | ++ |
| Capan-2 | - | + | ++ | ++ |
| AGS | + | + | ++ | ++ |
| HCT-116 | - | + | ++ | ++ |

Note: "-" indicated that there was no significant difference in cell survival rate between virus treatment group and MOCK group; "+" indicated that after virus treatment, the number of cells was reduced, the survival rate was greater than 50% but was significantly different from that of MOCK group; "++" indicated that the cell survival rate after virus treatment was less than 50%, and was significantly different from that of the MOCK group.

2.6 Evaluation of oncolytic effect of genomic RNA of EV-D68

In this example, a large amount of infectious live viruses of EV-D68 could be produced by transfecting the purified genomic RNA of EV-D68 into a certain kind of tumor cells, and thus kill the tumor cells.

The viral genomic RNA was first obtained by in vitro transcription, and this method could be found in, for example, Hadac E M, Kelly E J and Russell S J. Mol Ther, 2011, 19(6): 1041-1047. Specifically, the infectious cloning plasmid of wild-type EV-D68 obtained in Example 1 was linearized, and the linearized plasmid was used as a template for in vitro transcription using MEGAscript™ T7 Transcription Kit (Thermo Fisher Scientific, AM1333) so as to produce a large amount of viral RNA. And the obtained viral RNA was purified using MEGAclear™ Transcription Clean-Up Kit (Thermo Fisher Scientific, AM1908) for next use. The RNA electropherograms of 4 parallel samples were shown in FIG. 3.

Subsequently, according to the method of the in vitro antitumor experiment described in Example 2.4, the human cervical cancer tumor cell line Hela was inoculated to a 24-well plate at $10^5$ cells/well. After the cells adhered, the medium in each well was replaced with a corresponding cell culture medium free of serum, followed by incubation at 37 °C for 30 min. Then Hela cells were transfected with purified virus RNA at 1 μg per well using transfection reagent Lipofectamine® 2000 (Thermo Fisher Scientific, 11668019), and the negative control group was transfected with irrelevant RNA nucleic acid molecules. Subsequently, CPE of the cells were monitored daily by a microscope.

The results showed that CPE began to appear in the Hela cells transfected with genomic RNA of EV-D68 about 8 hours after transfection, and then the cytopathy gradually increased. After 48 hours, the survival rate was measured using the CCK8 method, the Hela cells had almost all died and lysed. And the micrographs of Hela cells at 0, 24 and 48 hours after infection were shown in FIG. 4. The culture supernatant was inoculated into new Hela cells and CPE was quickly produced. The results indicated that the direct administration with the nucleic acid of EV-D68 also had good killing activity and could be used to treat tumors.

**Example 3: In vivo antitumor experiments of enterovirus EV-D68 and its modified forms**

3.1 Viruses, cell lines and experimental animals

(1) Viruses: the EV-D68-WT (SEQ ID NO: 12), EV-D68-HRV2 (SEQ ID NO: 13), EV-D68-miR133&206T (SEQ ID NO: 14), EV-D68-GM-CSF (SEQ ID NO: 15) and EV-D68-Anti-PD-1 (SEQ ID NO: 16) provided in Example 1 were used in this example. The methods of virus culture and virus titer measurement could be seen in Examples 2.2 and 2.3, respectively.

(2) Cell lines: human cervical cancer cell line Hela (ATCC® Number: CCL-2™), glioma cell line U118-MG (ATCC® Number: HTB-15™), and lymphoma cell line Raji (ATCC® Number: CCL-86™). Except that Raji was cultured with RPMI-1640 medium, the other Hela and U118-MG were all cultured with DMEM medium. These mediums were all supplemented with 10% fetal bovine serum, glutamine and penicillin-streptomycin. All the above cells were cultured under the standard conditions of 37 °C and 5% $CO_2$.

(3) Experimental animals: female C.B17 SCID mice aged 6-8 weeks were from Shanghai Slark Experimental Animal Co., Ltd.; the mice were raised under SPF conditions, according to the protocol approved by the Experimental Animal Center and Ethics Committee of Xiamen University.

3.2 In vivo antitumor experiments of the virus

The tumor cells used for subcutaneous tumor formation in SCID mice were digested with 0.01% trypsin, and then resuspended into a single-cell suspension using a cell culture medium containing 10% fetal bovine serum. The cell density of the suspension was counted. The cells were precipitated by centrifugation under 1000 g for 3 min, and then the cells were resuspended with an appropriate volume of PBS to reach a concentration of about $10^6$-$10^7$ cells/100 μl PBS. The tumor cells were subcutaneously inoculated in the back of SCID mice at $10^6$-$10^7$ cells/100 μl PBS/site with a syringe. When the tumor cells grew into a tumor mass of about 100 $mm^3$ under the skin of SCID mice after about 14-21 days, the tumor-bearing SCID mice were randomly divided into experimental groups (administrated with EV-D68-WT, EV-D68-HRV2, EV-D68-miR133&206T, EV-D68-GM-CSF or EV-D68-Anti-PD-1) and negative control group, with 4 mice (n = 4) in each group. Oncolytic virus (EV-D68-WT, EV-D68-HRV2, EV-D68-miR133&206T, EV-D68-GM-CSF or EV-D68-Anti-PD-1) at $10^6$ TCID50/100μl serum-free medium/tumor mass or equivalent amount of serum-free medium were intratumorally injected every two days, for a total of 5 treatments. The tumor size was measured with a vernier caliper and recorded every two days, and the method for calculating the tumor size was:

$$\text{Tumor size (mm}^3\text{)} = \text{tumor length value} \times (\text{tumor width value})^2 / 2.$$

The treatment results of EV-D68-WT for the above three tumors were shown in FIGs. 5A-5C. The results showed that after the challenge of EV-D68-WT, the growth of the three tested tumors of Hela (A), U118-MG (B) and Raji (C) gradually slowed down and arrested, and the tumors were even lysed and disappeared; by contrast, the tumors of the negative group (CTRL) maintained the normal growth, and their tumor sizes were significantly larger than those of the experimental groups.

Table 6 showed the results obtained after a treatment of the Raji tumor model with EV-D68-WT, EV-D68-HRV2, EV-D68-miR133&206T, EV-D68-GM-CSF or EV-D68-Anti-PD-1 for 10 days. The results showed that the tumor volumes were significantly reduced after treatment with EV-D68-WT, EV-D68-HRV2, EV-D68-miR133&206T, EV-D68-GM-CSF, and EV-D68-Anti-PD as compared with the negative control group that was not treated with oncolytic virus, wherein similar reductions in tumor volume were detected after treatment with 4 oncolytic viruses EV-D68-WT, EV-D68-miR133&206T, EV-D68-GM-CSF and EV-D68-Anti-PD-1. The above results indicated that all of EV-D68-WT, EV-D68-HRV2, EV-D68-miR133&206T, EV-D68-GM-CSF and EV-D68-Anti-PD-1 showed remarkable and favorable antitumor activity in vivo.

Table 6: Results of in vivo anti-tumor experiments of EV-D68-WT, EV-D68-HRV2, EV-D68-miR133&206T, EV-D68-GM-CSF and EV-D68-Anti-PD-1 on human lymphoma cell line Raji

| Oncolytic virus | In vivo oncolytic effect on Raji after 10 days of treatment |
|---|---|
| EV-D68-WT | ++ |
| EV-D68-HRV2 | + |
| EV-D68-miR133&206T | ++ |
| EV-D68-GM-CSF | ++ |
| EV-D68-Anti-PD-1 | ++ |
| Note: "+" indicated that after treatment, the tumor volume reduced and was greater than 50% of the negative control group, but was significantly different from that of the negative control group; "++" indicated that the tumor volume reduced to less than 50% of the negative control group after treatment, and was significantly different from that of the negative control group. | |

**Example 4: Safety evaluation of oncolytic virus**

4.1 Viruses and laboratory animals used

(1) Virus: the EV-D68-WT (SEQ ID NO: 12) provided in Example 1 was used in this example. The methods for virus culture and virus titer measurement could refer to Examples 2.2 and 2.3, respectively.

(2) Experimental animals: BALB/c pregnant mice were from Shanghai Slark Experimental Animal Co., Ltd.; according to the protocol approved by the Experimental Animal Center and Ethics Committee of Xiamen University, the mice were raised under clean conditions, and then the 1-day-old, 2-day-old, 3-day-old, 7-day-old and 14-day-old mice produced by the BALB/c pregnant mice were used for in vivo virulence evaluation of EV-D68.

4.2 Evaluation of the safety of the virus in mice

(1) BALB/c suckling mice aged 1 day were selected for challenge with EV-D68-WT by intraperitoneal injection, and the titer doses for challenge were $10^3$, $10^4$, $10^5$, $10^6$, or $10^7$ TCID50/mouse. Then the survival rates and health scores for the BALB/c mice challenged with different doses were recorded daily, wherein the evaluation criteria of the health score were: score of 5, represents dying or died; score of 4 represents severe limb paralysis; score of 3 represents weakness or mild deformity of limb; score of 2 represents wasting; score of 1 represents lethargy, piloerection, and trembling; and score of 0 represents healthy.

The results were shown in FIG. 6A. Within 20 days after challenge, all mice in the group with extremely high-dose of $10^7$ TCID50 became ill and died within 1 week; 80% of the mice in the group with high-dose of $10^6$ TCID50 eventually survived and only few mice became ill and died; in addition, no morbidity and death occurred in the mice of the challenge groups with other doses.

(2) The 1-day-old, 2-day-old, 3-day-old, 7-day-old and 14-day-old BALB/c mice were injected with EV-D68-WT at an extremely high dose of $10^7$ TCID50/mouse, and then the survival rates and health scores for the BALB/c with different ages in days were recorded daily, wherein the evaluation criteria of the health score were the same as above.

The results were shown in FIG. 6B. Within 20 days after challenge, the 1-day-old mice all died within 1 week; the 2-day-old mice resisted in a certain extent to EV-D68 toxicity, and eventually had a survival rate of 70%, but with a relatively high incidence of disease and relatively severe symptoms; the 3-day-old mice were already not vulnerable to EV-D68, and eventually had a survival rate of 90%, and with a low incidence of disease and mild symptoms; the 4- or more-day-old mice were fully tolerant to the high doses of EV-D68, and no morbidity and death occurred.

The above results showed that the EV-D68-WT was less toxic to mice, and was only lethal to the 1- to 3-day-old BALB/c mice at an extremely high dose of $10^7$ TCID50/mouse, and had no effect on the 4- or more-day-old mice, thereby indicating good safety in vivo.

Although specific embodiments of the present invention have been described in detail, those skilled in the art will understand that according to all the teachings that have been published, various modifications and changes can be made to the detail, and these changes are all within the protection scope of the present invention. The protection scope of the present invention is given by the appended claims and any equivalents thereof.

## Claims

1. Use of an Enterovirus D68 (EV-D68) or a modified form thereof, or an isolated nucleic acid molecule, in treatment of a tumor in a subject, or in the manufacture of a medicament for treating a tumor in a subject; wherein the nucleic acid molecule comprises a sequence selected from the following:

   (1) a genomic sequence or cDNA sequence of the EV-D68 or a modified form thereof; and
   (2) a complementary sequence of the genomic sequence or cDNA sequence.

2. Use according to Claim 1, wherein the EV-D68 is a wild-type EV-D68;
   preferably, the EV-D68 is a clinical isolate isolated from an individual infected with the Enterovirus D68;
   preferably, the EV-D68 or a modified form thereof has a genomic sequence that has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 12; preferably, the genomic sequence of the EV-D68 or a modified form thereof is a nucleotide sequence as shown in SEQ ID NO: 12;
   preferably, the EV-D68 or a modified form thereof has a cDNA sequence that has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 1; preferably, the cDNA sequence of the EV-D68 or a modified form thereof is a nucleotide sequence as shown in SEQ ID NO: 1.

3. Use according to Claim 1 or 2, wherein the modified form is a modified EV-D68, which has a substitution, insertion, or deletion of one or more nucleotides in the genome as compared to a wild-type EV-D68;
   preferably, as compared to the wild-type EV-D68, the modified EV-D68 has one or more modifications selected from the following:

(1) one or more mutations in an untranslated region (e.g., 5'UTR or 3'UTR);
(2) an insertion of one or more exogenous nucleic acids;
(3) a deletion or mutation of one or more endogenous genes; and
(4) any combination of the above three items.

**4.** Use according to Claim 3, wherein the modified EV-D68 comprises one or more mutations in the 5' untranslated region (5'UTR);
preferably, the modified EV-D68 has a substitution of all or part of the 5'UTR sequence; preferably, the internal ribosome entry site (IRES) sequence in the 5'UTR of the modified EV-D68 is replaced with an exogenous IRES sequence, such as the internal ribosome entry site sequence of human rhinovirus 2 (HRV2);
preferably, the internal ribosome entry site sequence of the human rhinovirus 2 (HRV2) is shown in SEQ ID NO: 2.

**5.** Use according to Claim 3 or 4, wherein the modified EV-D68 comprises an exogenous nucleic acid;
preferably, the exogenous nucleic acid encodes a cytokine (e.g., a GM-CSF, preferably a human GM-CSF), or an antitumor protein or polypeptide (e.g., a scFv against PD-1 or PD-L1, preferably a scFv against human PD-1 or PD-L1);
preferably, the exogenous nucleic acid is inserted between the 5'UTR and the VP4 gene, or between the VPl gene and the 2A gene of the genome of the modified EV-D68;
preferably, the exogenous nucleic acid comprises a target sequence of one or more (e.g., 2, 3, or 4) microRNA;
preferably, the target sequence of microRNA is inserted in the 3' untranslated region (3'UTR) of the genome of the modified EV-D68;
preferably, the exogenous nucleic acid comprises a target sequence of miR-133 and/or miR-206;
preferably, the target sequence of miR-133 is shown in SEQ ID NO: 3;
preferably, the target sequence of miR-206 is shown in SEQ ID NO: 4.

**6.** Use according to any one of Claims 3 to 5, wherein the modified EV-D68 comprises at least one insertion of the exogenous nucleic acid as defined in Claim 5 and/or at least one mutation in the untranslated region as defined in Claim 4.

**7.** Use according to any one of Claims 3 to 6, wherein the modified EV-D68 has one of the following characteristics:

(1) the genomic sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 13-16; preferably, the genomic sequence of the modified EV-D68 is selected from the nucleotide sequences as shown in any one of SEQ ID NOs: 13-16; and
2) the cDNA sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 8-11; preferably, the cDNA sequence of the modified EV-D68 is selected from the nucleotide sequences as shown in any one of SEQ ID NOs: 8-11.

**8.** Use according to any one of Claims 1 to 7, wherein the isolated nucleic acid molecule consists of a genomic sequence or cDNA sequence of the EV-D68 or a modified form thereof, or a complementary sequence of the genomic sequence or cDNA sequence;
preferably, the isolated nucleic acid molecule has a genomic sequence of the EV-D68 or a modified form thereof;
preferably, the isolated nucleic acid molecule has a nucleotide sequence as shown in any one of SEQ ID NOs: 12-16.

**9.** Use according to any one of Claims 1 to 7, wherein the isolated nucleic acid molecule is a vector (e.g. a cloning vector or an expression vector) comprising a genomic sequence or cDNA sequence of the EV-D68 or a modified form thereof, or a complementary sequence of the genomic sequence or cDNA sequence;
preferably, the isolated nucleic acid molecule is a vector (e.g., a cloning vector or an expression vector) comprising a cDNA sequence of the EV-D68 or a modified form thereof, or a complementary sequence of the cDNA sequence;
preferably, the isolated nucleic acid molecule is a vector comprising a nucleotide sequence as shown in any one of SEQ ID NOs: 1, 8-11 or a complementary sequence thereof.

**10.** Use according to any one of Claims 1 to 9, wherein the EV-D68 or a modified form thereof, or the isolated nucleic acid molecule, is used in combination with an additional pharmaceutically active agent;
preferably, the additional pharmaceutically active agent has antitumor activity;
preferably, the additional pharmaceutically active agent is selected from an additional oncolytic virus, chemotherapeutic agent or immunotherapeutic agent;
preferably, the additional oncolytic virus is selected from herpesvirus, adenovirus, parvovirus, reovirus, Newcastle disease virus, vesicular stomatitis virus, measles virus or any combination thereof;
preferably, the chemotherapeutic agent is selected from 5-fluorouracil, mitomycin, methotrexate, hydroxyurea, cyclophosphamide, dacarbazine, mitoxantrone, anthracyclines (e.g., epirubicin or doxorubicin), etoposide, platinum compounds (e.g., carboplatin or cisplatin), taxanes (e.g., paclitaxel or taxotere), or any combination thereof;
preferably, the immunotherapeutic agent is selected from immune checkpoint inhibitors (e.g., PD-L1/PD-1 inhibitors or CTLA-4 inhibitors), tumor-specific targeting antibodies (e.g., rituximab or Herceptin) or any combination thereof.

**11.** Use according to any one of Claims 1 to 10, which has at least one of the following characteristics:

(1) the tumor is selected from cervical cancer, ovarian cancer, endometrial cancer, lung cancer, liver cancer, kidney cancer, neuroblastoma, glioma, breast cancer, melanoma, prostate cancer, bladder cancer, pancreatic cancer, gastric cancer, colorectal cancer, esophageal cancer, thyroid cancer, laryngeal cancer, osteosarcoma, hematopoietic malignancy (e.g., lymphoma or leukemia);
(2) the subject is a mammal, such as a human.

**12.** An EV-D68 or a modified form thereof, or an isolated nucleic acid molecule, for use as a medicament; wherein the nucleic acid molecule comprises a sequence selected from the following:

(1) a genomic sequence or cDNA sequence of the EV-D68 or a modified form thereof; and
(2) a complementary sequence of the genomic sequence or cDNA sequence;

preferably, the EV-D68 or a modified form thereof, or the isolated nucleic acid molecule is as defined in any one of Claims 1 to 11.

**13.** A method for treating a tumor, comprising the step of administering to a subject in need thereof an effective amount of an EV-D68 or a modified form thereof, or an effective amount of an isolated nucleic acid molecule; wherein the isolated nucleic acid molecule comprises a sequence selected from:

(1) a genomic sequence or cDNA sequence of the EV-D68 or a modified form thereof; and
(2) a complementary sequence of the genomic sequence or cDNA sequence;

preferably, the tumor is selected from cervical cancer, ovarian cancer, endometrial cancer, lung cancer, liver cancer, kidney cancer, neuroblastoma, glioma, breast cancer, melanoma, prostate cancer, bladder cancer, pancreatic cancer, gastric cancer, colorectal cancer, esophageal cancer, thyroid cancer, laryngeal cancer, osteosarcoma, hematopoietic malignancy (e.g., lymphoma or leukemia);
preferably, the subject is a mammal, such as a human;
preferably, the EV-D68 or a modified form thereof, or the isolated nucleic acid molecule, is as defined in any one of Claims 1 to 11.

**14.** A pharmaceutical composition, which comprises an EV-D68 or a modified form thereof, or an isolated nucleic acid molecule; wherein the nucleic acid molecule comprises a sequence selected from the group consisting of:

(1) a genomic sequence or cDNA sequence of the EV-D68 or a modified form thereof; and
(2) a complementary sequence of the genomic sequence or cDNA sequence;

preferably, the pharmaceutical composition further comprises a pharmaceutically acceptable carrier or excipient;
preferably, the pharmaceutical composition optionally further comprises an additional pharmaceutically active agent;
preferably, the additional pharmaceutically active agent is a medicament having antitumor activity, such as an additional oncolytic virus, chemotherapeutic agent, or immunotherapeutic agent;
preferably, the pharmaceutical composition is used for treating a tumor in a subject;
preferably, the subject is a mammal, such as a human;
preferably, the tumor is selected from cervical cancer, ovarian cancer, endometrial cancer, lung cancer, liver cancer, kidney cancer, neuroblastoma, glioma, breast cancer, melanoma, prostate cancer, bladder cancer, pancreatic cancer, gastric cancer, colorectal cancer, esophageal cancer, thyroid cancer, laryngeal cancer, osteosarcoma, hematopoietic malignancy (e.g., lymphoma or leukemia);
preferably, the EV-D68 or a modified form thereof, or the isolated nucleic acid molecule, is as defined in any one of Claims 1 to 11.

**15.** A modified EV-D68, which has a substitution, insertion, or deletion of one or more nucleotides in the genome as compared to a wild-type EV-D68;
preferably, the genomic sequence of the wild-type EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 12; preferably, the genomic sequence of the wild-type EV-D68 is a nucleotide sequence as shown in SEQ ID NO: 12;
preferably, the cDNA sequence of the wild-type EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence as shown in SEQ ID NO: 1; preferably, the cDNA sequence of the wild-type EV-D68 is a nucleotide sequence as shown in SEQ ID NO: 1;
preferably, the modified EV-D68 has one or more modifications selected from the following as compared to the wild-type EV-D68:

(1) one or more mutations in an untranslated region (e.g., 5'UTR or 3'UTR);
(2) an insertion of one or more exogenous nucleic acids;
(3) a deletion or mutation of one or more endogenous genes; and
(4) any combination of the above three items.

**16.** The modified EV-D68 according to Claim 15, which comprises one or more mutations in the 5' untranslated region (5'UTR);
preferably, the modified EV-D68 has a substitution of all or part of the 5'UTR sequence;
preferably, the internal ribosome entry site (IRES) sequence in the 5'UTR of the modified EV-D68 is replaced with an exogenous IRES sequence, such as the internal ribosome entry site sequence of a human rhinovirus 2 (HRV2);
preferably, the internal ribosome entry site sequence of the human rhinovirus 2 (HRV2) is shown in SEQ ID NO: 2.

**17.** The modified EV-D68 according to Claim 15 or 16, which comprises an exogenous nucleic acid;
preferably, the exogenous nucleic acid encodes a cytokine (e.g., a GM-CSF, preferably a human GM-CSF), or an antitumor protein or polypeptide (e.g., a scFv against PD-1 or PD-L1, preferably a scFv against human PD-1 or PD-L1);
preferably, the exogenous nucleic acid is inserted between the 5'UTR and the VP4 gene, or between the VP1 gene and the 2A gene of the genome of the modified EV-D68;
preferably, the exogenous nucleic acid comprises a target sequence of one or more (e.g., 2, 3, or 4) microRNA;
preferably, the target sequence of the microRNA is inserted in the 3' untranslated region (3'UTR) of the genome of the modified EV-D68;
preferably, the exogenous nucleic acid comprises a target sequence of miR-133 and/or miR-206;
preferably, the target sequence of miR-133 is shown in SEQ ID NO: 3;
preferably, the target sequence of miR-206 is shown in SEQ ID NO: 4.

**18.** The modified EV-D68 according to any one of Claims 15 to 17, wherein the modified EV-D68 comprises at least one insertion of the exogenous nucleic acid as defined in Claim 17 and/or at least one mutation in the untranslated region as defined in Claim 16.

**19.** The modified EV-D68 according to any one of Claims 15 to 18, wherein the modified EV-D68 has at least one of the following characteristics:

(1) the genomic sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 13-16; preferably, the genomic sequence of the modified EV-D68 is selected from the nucleotide sequences as shown in any one of SEQ ID NOs: 13-16; and
2) the cDNA sequence of the modified EV-D68 has a sequence identity of at least 70%, at least 80%, at least 85%, at least 90%, at least 91%, at least 92%, at least 93%, at least 94%, at least 95%, at least 96%, at least 97%, at least 98%, at least 99%, or 100% to a nucleotide sequence selected from: the nucleotide sequences as shown in SEQ ID NOs: 8-11; preferably, the cDNA sequence of the modified EV-D68 is selected from the nucleotide sequences as shown in any one of SEQ ID NOs: 8-11.

**20.** An isolated nucleic acid molecule, which comprises a sequence selected from the group consisting of:

(1) a genomic sequence or cDNA sequence of the modified EV-D68 according to any one of Claims 15 to 19; and
(2) a complementary sequence of the genomic sequence or cDNA sequence.

**21.** The isolated nucleic acid molecule according to Claim 20, wherein the isolated nucleic acid molecule consists of a genomic sequence or cDNA sequence of the EV-D68 or a modified form thereof, or a complementary sequence of the genomic sequence or cDNA sequence;
preferably, the isolated nucleic acid molecule has a genomic sequence of the EV-D68 or a modified form thereof;
preferably, the isolated nucleic acid molecule has a nucleotide sequence as shown in any one of SEQ ID NOs: 12-16.

**22.** The isolated nucleic acid molecule according to Claim 20, wherein the isolated nucleic acid molecule is a vector (e.g. a cloning vector or expression vector) comprising a genomic sequence or cDNA sequence of the EV-D68 or a modified form thereof, or a complementary sequence of the genomic sequence or cDNA sequence;
preferably, the isolated nucleic acid molecule is a vector comprising a cDNA sequence of the EV-D68 or a modified form thereof, or a complementary sequence of the cDNA sequence;
preferably, the isolated nucleic acid molecule is a vector comprising a nucleotide sequence as shown in any one of SEQ ID NOs: 1, 8-11 or a complementary sequence thereof.

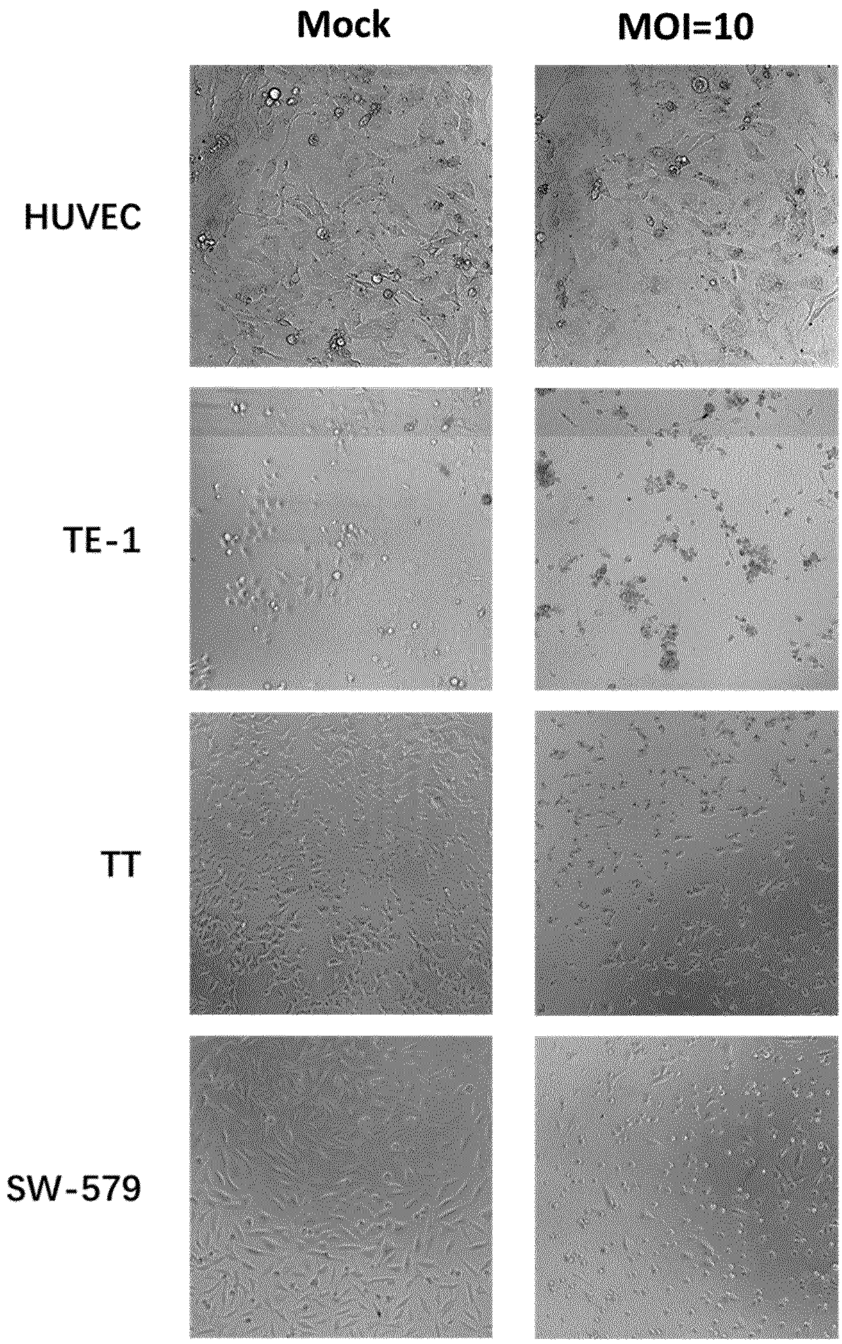


FIG. 1

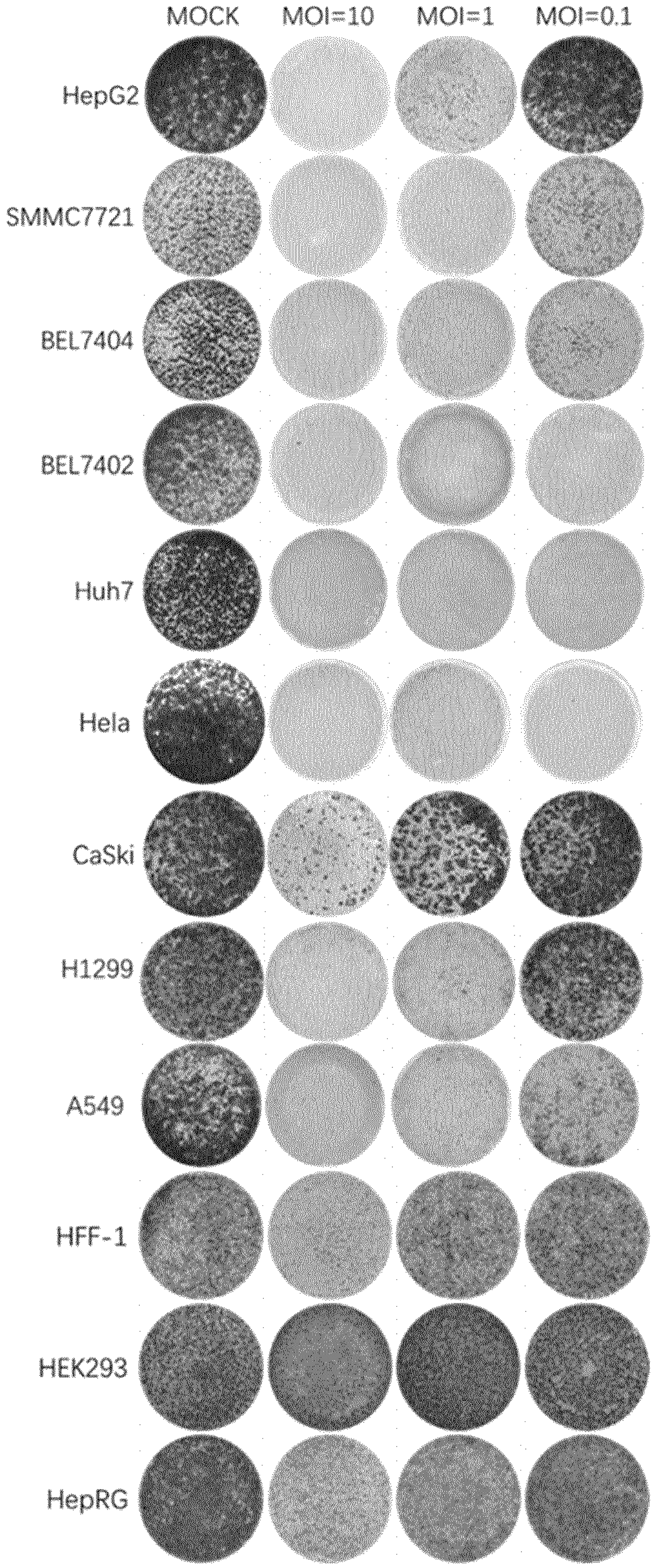
MOCK
MOI=10
MOI=1
MOI=0.1
HepG2
SMMC7721
BEL7404
BEL7402
Huh7
Hela
CaSki
H1299
A549
HFF-1
HEK293
HepRG

**FIG. 2**

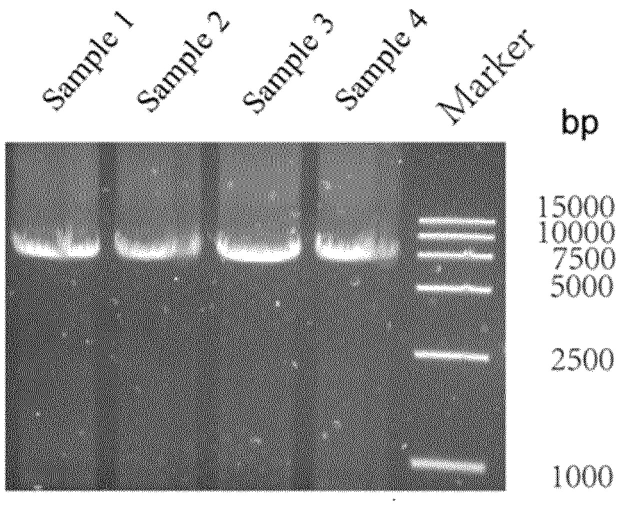
Sample 1
Sample 2
Sample 3
Sample 4
Marker
bp
15000
10000
7500
5000
2500
1000

**FIG. 3**

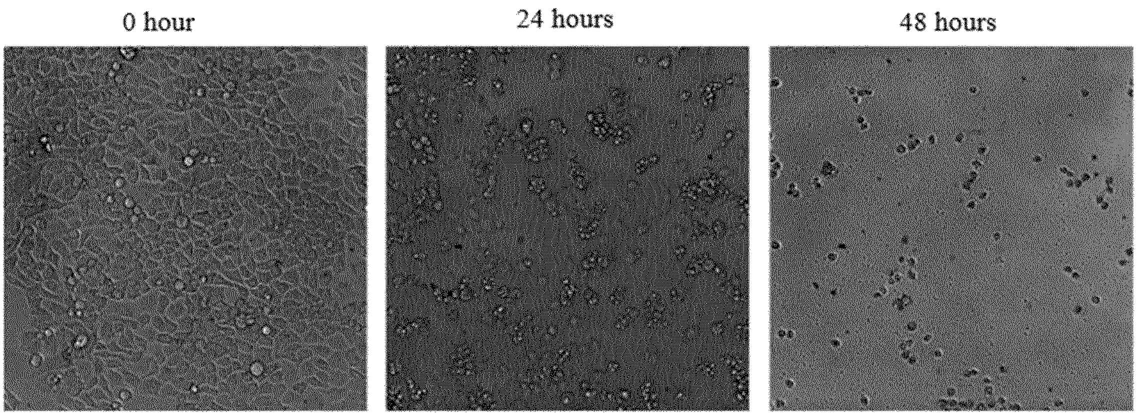
0 hour
24 hours
48 hours

**FIG. 4**

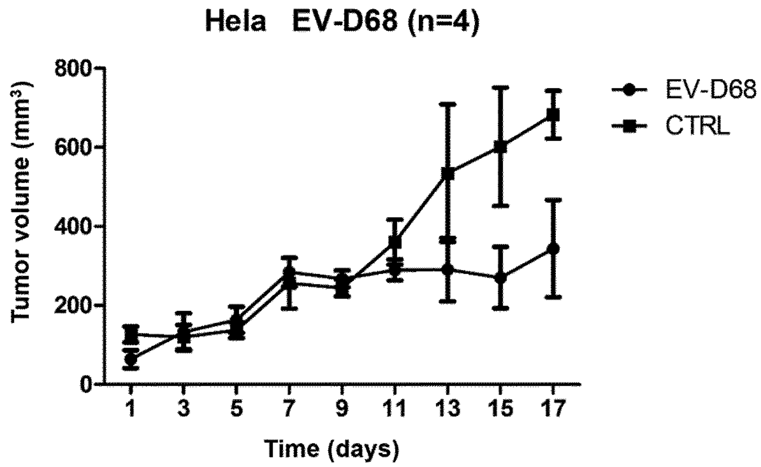


FIG. 5A

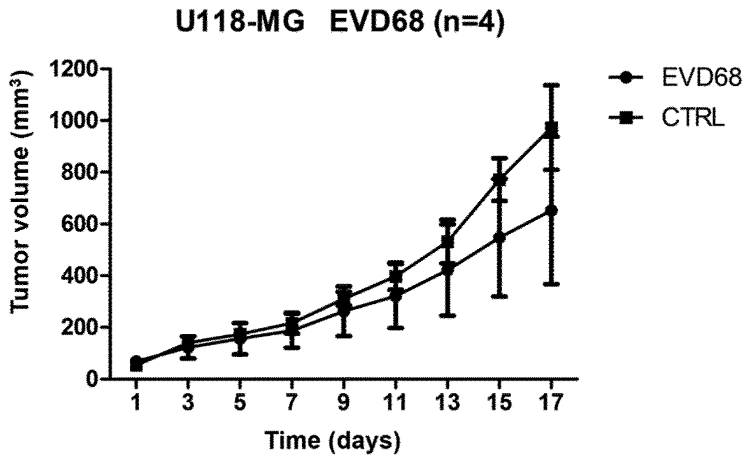


FIG. 5B

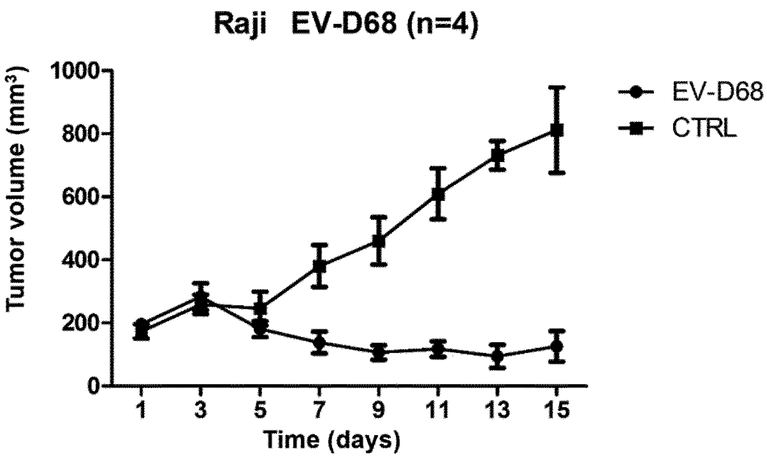


FIG. 5C

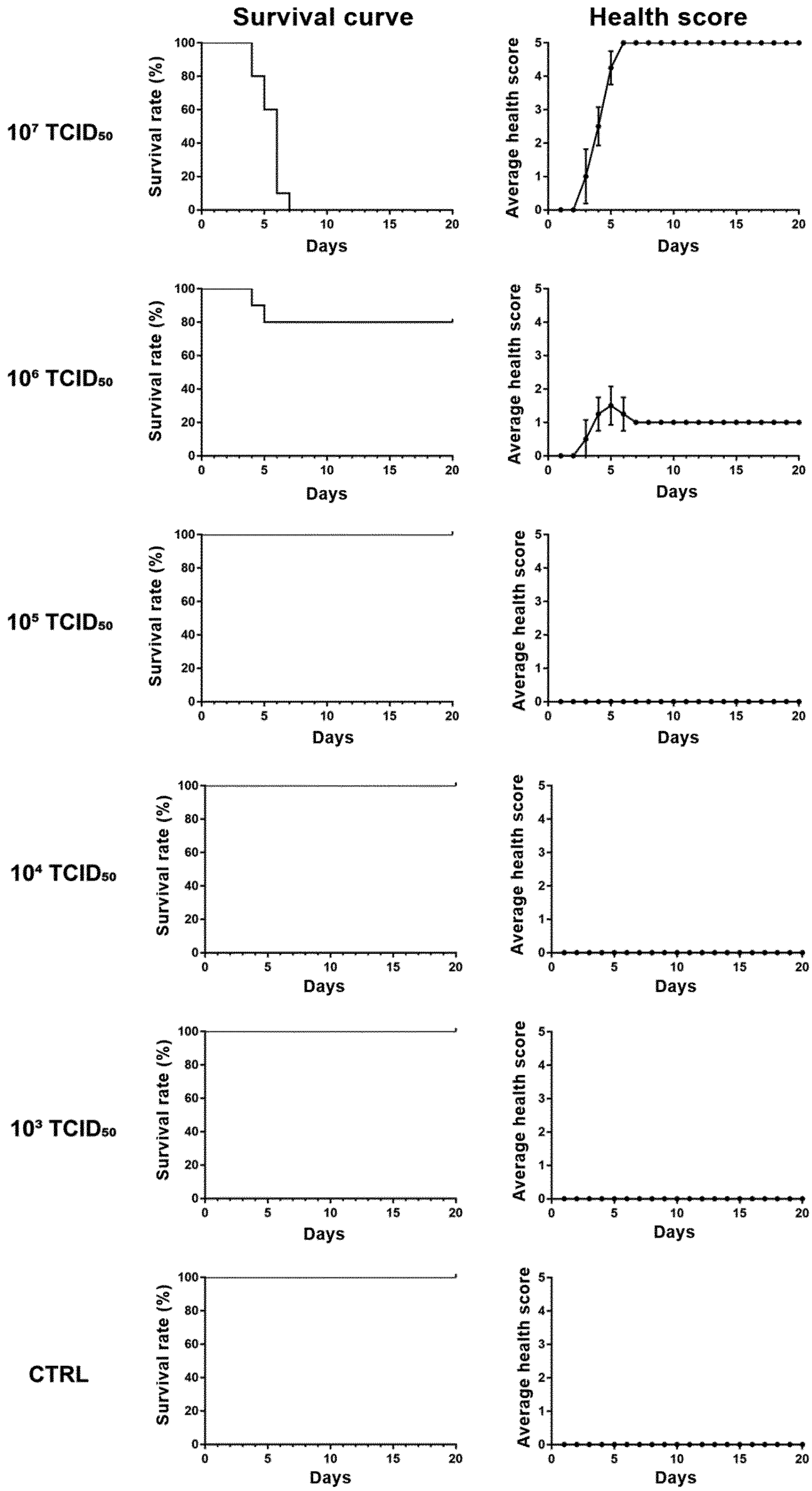
Survival curve
Health score
10⁷ TCID50
10⁶ TCID50
10⁵ TCID50
10⁴ TCID50
10³ TCID50
CTRL
Survival rate (%)
Average health score
Days

FIG. 6A

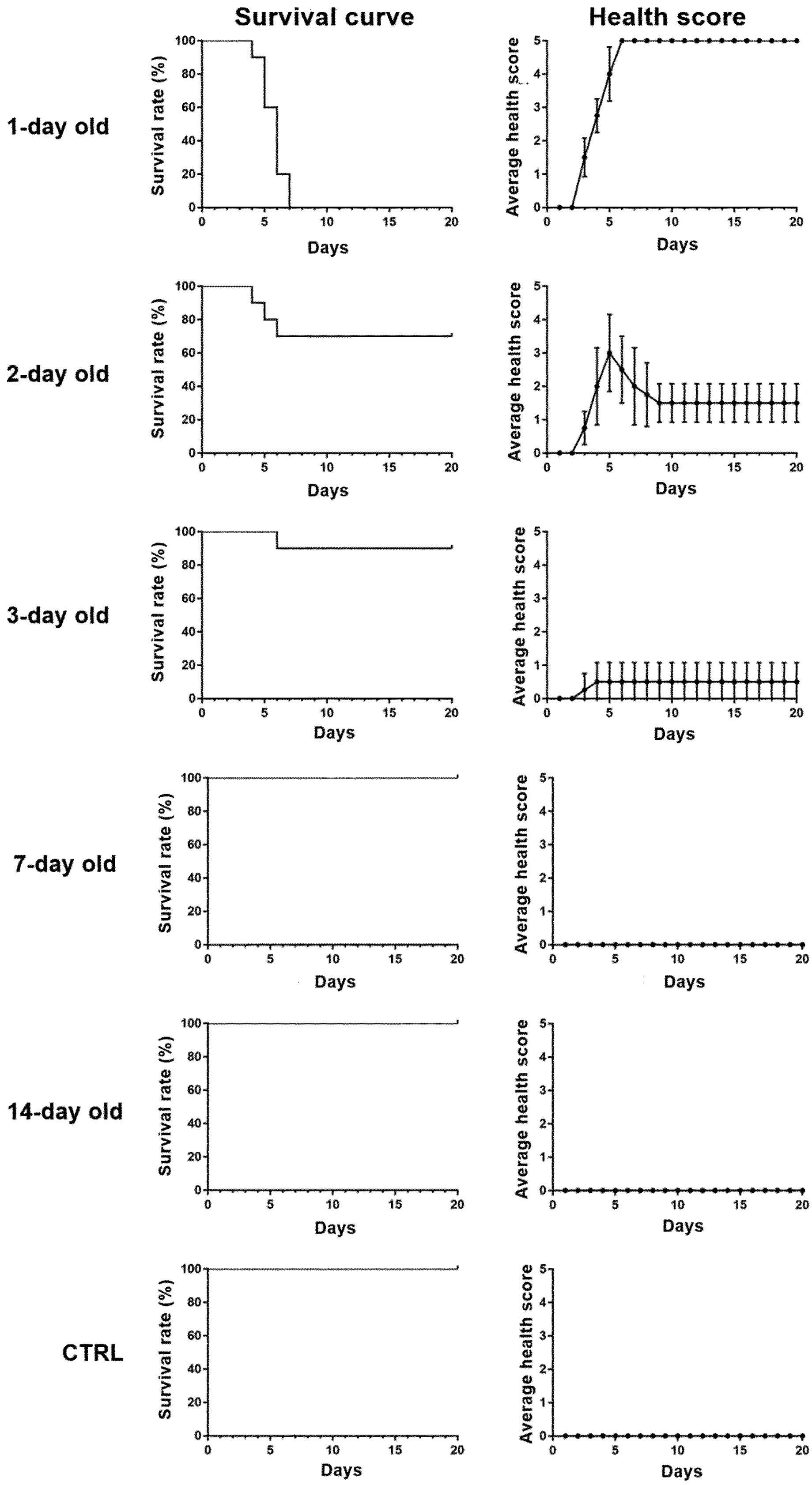
Survival curve
Health score
1-day old
2-day old
3-day old
7-day old
14-day old
CTRL
Survival rate (%)
Average health score
Days

FIG. 6B

**INTERNATIONAL SEARCH REPORT**

International application No.
**PCT/CN2018/096100**

**A. CLASSIFICATION OF SUBJECT MATTER**

C12N 7/00(2006.01)i；C12N 15/11(2006.01)i；A61K 35/76(2015.01)i；A61P 35/00(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS, CPRSABS, CNTXT, CSCD, CJFD, SIPONPL, DWPI, SIPOABS, CATXT, USTXT, EPTXT, WOTXT, KRTXT, JPTXT, CNKI, WEB OF SCIENCE, PUBMED: 肠道病毒, 修饰, 肿瘤, EV-D68, MODIF+, tumor; GENBANK+EMBL+DDBJ+中国专利生物序列检索系统: 对SEQ ID NOs:1和12的序列检索, GENBANK+EMBL+DDBJ+NATIONAL BIO-SEQUENCE DATABASE OF CHINESE PATENT: search for SEQ ID NOs: 1 and 12

**C. DOCUMENTS CONSIDERED TO BE RELEVANT**

| Category* | Citation of document, with indication, where appropriate, of the relevant passages | Relevant to claim No. |
|---|---|---|
| X | "GenBank Accession No: KM851225" *GenBank Database,* 01 December 2014 (2014-12-01), see sequence and related information thereof | 12 (in part) |
| Y | "GenBank Accession No: KM851225" *GenBank Database,* 01 December 2014 (2014-12-01), see sequence and related information thereof | 1-11, 14 (all in part) |
| Y | CN 105518128 A (DITESAN LTD.) 20 April 2016 (2016-04-20) see abstract, and claims 1-14 | 1-11, 14 (all in part) |
| A | US 2016355897 A1 (US HEALTH ET AL.) 08 December 2016 (2016-12-08) see entire document | 1-12, 14 (all in part) |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 October 2018** | **26 October 2018** |

| Name and mailing address of the ISA/CN | Authorized officer |
|---|---|
| **State Intellectual Property Office of the P. R. China**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2018/096100**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

## INTERNATIONAL SEARCH REPORT

International application No.
**PCT/CN2018/096100**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-11**（均为部分）和**13**
because they relate to subject matter not required to be searched by this Authority, namely:

[1] relating to a treatment method for the living human or animal body, which falls within the cases set out in PCT Rule 39.1(iv), for which a search is not required.

[2] The statements and explanations regarding claims 1-11 (all in part) are provided based on "the use in the preparation of medications for treating tumour among subjects".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[1] Group 1: claims 1-12 and 14 (all in part) relate to a wild type EV-D68, the use in medication preparation and a pharmaceutical composition including the wild type EV-D68, the genome and cDNA sequence thereof respectively having at least 70% sequence identity with sequences SEQ ID NO:12 and SEQ ID NO:1;

[2] Group 2: claims 1-12 and 14-22 (all in part) relate to a modificatory EV-D68, the use in medication preparation and a pharmaceutical composition including the wild type EV-D68, the genome and cDNA sequence thereof respectively having at least 70% sequence identity with sequences SEQ ID NO:13 and SEQ ID NO:8;

[3] Group 3: claims 1-12 and 14-22 (all in part) relate to a modificatory EV-D68, the use in medication preparation and a pharmaceutical composition including the wild type EV-D68, the genome and cDNA sequence thereof respectively having at least 70% sequence identity with sequences SEQ ID NO:14 and SEQ ID NO:9;

[4] Group 4: claims 1-12 and 14-22 (all in part) relate to a modificatory EV-D68, the use in medication preparation and a pharmaceutical composition including the wild type EV-D68, the genome and cDNA sequence thereof respectively having at least 70% sequence identity with sequences SEQ ID NO:15 and SEQ ID NO:10; and

[5] Group 5: claims 1-12 and 14-22 (all in part) relate to a modificatory EV-D68, the use in medication preparation and a pharmaceutical composition including the wild type EV-D68, the genome and cDNA sequence thereof respectively having at least 70% sequence identity with sequences SEQ ID NO:16 and SEQ ID NO:11.

[6] The above-mentioned five groups of inventions all have different genomes and cDNA sequences, and the common technical feature is an enterovirus EV-D68. However, it is a known virus, and acquiring the genome and the cDNA thereof is also a conventional technique. Therefore, the common technical feature cannot serve as a specific technical feature (the technical feature which makes contribution to the prior art), namely, the above-mentioned five groups of inventions do not share a same or corresponding specific technical feature, and therefore, the five groups of inventions do not belong to a single general inventive concept, and lack unity, not complying with PCT Rule 13.1.

Form PCT/ISA/210 (continuation of first sheet) (January 2015)

**INTERNATIONAL SEARCH REPORT**

International application No.
**PCT/CN2018/096100**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-12 and 14 (all in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/CN2018/096100**

| Patent document cited in search report | Publication date (day/month/year) | Patent family member(s) | Publication date (day/month/year) |
|---|---|---|---|
| CN 105518128 A | 20 April 2016 | EP 3022297 A1 | 25 May 2016 |
| | | EP 2826856 A1 | 21 January 2015 |
| | | BR 112016000874 A2 | 21 March 2017 |
| | | EA 201690232 A1 | 31 May 2016 |
| | | GE P201706763 B | 25 October 2017 |
| | | JP 6293882 B2 | 14 March 2018 |
| | | JP 2016529886 A | 29 September 2016 |
| | | WO 2015007788 A1 | 22 January 2015 |
| | | MD 4480 C1 | 31 December 2017 |
| | | CA 2917584 C | 27 February 2018 |
| | | EP 2826856 B1 | 23 December 2015 |
| | | MD 20160014 A2 | 31 July 2016 |
| | | AU 2014292114 A1 | 18 February 2016 |
| | | UA 116387 C2 | 12 March 2018 |
| | | US 2016376562 A1 | 29 December 2016 |
| | | ES 2566146 T3 | 11 April 2016 |
| | | EP 2826856 B9 | 04 May 2016 |
| | | MX 2016000462 A | 26 July 2016 |
| | | MD 4480 B1 | 31 May 2017 |
| | | CA 2917584 A1 | 22 January 2015 |
| | | HK 1219500 A1 | 07 April 2017 |
| US 2016355897 A1 | 08 December 2016 | US 9938588 B2 | 10 April 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008103755 A1
- US 20160143969 A1


### Non-patent literature cited in the description


- **DOBRIKOVA et al.** *Mol Ther,* 2008, vol. 16 (11), 1865-1872
- **AU et al.** *Virol J,* 2011, vol. 8, 22
- **SHAFREN et al.** *Int J Cancer,* 2005, vol. 115 (2), 320-328
- **HALEY et al.** *J Mol Med (Berl),* 2009, vol. 87 (4), 385-399
- **SCHIEBLE et al.** *Am J Epidemiol,* 1967, vol. 85 (2), 297-310
- **MERRILL et al.** *J Virol,* 2006, vol. 80 (7), 3147-3156
- **MERRILL ; GROMEIER.** *J Virol,* 2006, vol. 80 (14), 6936-6942
- **NEPLIOUEVA et al.** *PLoS One,* 2010, vol. 5 (7), e11710
- **DOBRIKOV et al.** *Mol Cell Biol,* 2011, vol. 31 (14), 2947-2959
- **DOBRIKOV et al.** *Mol Cell Biol,* 2013, vol. 33 (5), 937-946
- **BAOHONG ZHANG et al.** *Developmental Biology,* 01 February 2007, vol. 302 (1), 1-12
- **YANG LS ; LI SX ; LIU YJ et al.** *Virus Res,* 2015, vol. 210, 165-168
- **HOU WH ; YANG LS ; LI SX et al.** *Virus Res,* 2015, vol. 205, 41-44
- **YIN H et al.** *Nat Rev Genet.,* August 2014, vol. 15 (8), 541-55
- **RILEY MK ; VERMERRIS W.** *Nanomaterials (Basel),* 28 April 2017, vol. 7 (5), E94
- **YIN H et al.** *Nat Rev Genet.,* August 2014, vol. 15 (8), 541-55
- **RUIZ AJ ; RUSSELL S J.** MicroRNAs and oncolytic viruses. [J. *Curr Opin Virol,* 2015, vol. 13, 40-48
- **ARDOLINO M ; HSU J ; RAULET D H.** Cytokine treatment in cancer immunotherapy. *J]. Oncotarget,* 2015, vol. 6 (23), 19346-19347
- **CANDOLFI M ; KING GD ; MUHAMMAD AG et al.** Evaluation of proapoptotic transgenes to use in combination with Flt3L in an immune-stimulatory gene therapy approach for Glioblastoma multiforme (GBM) [J. *FASEB J,* 2008, vol. 22, 1077.13
- **NOLAN E ; SAVAS P ; POLICHENI AN et al.** Combined immune checkpoint blockade as a therapeutic strategy for BRCA1-mutated breast cancer [J. *Science Trans Med,* 2017, vol. 9, 4922
- **ROSCA EV ; KOSKIMAKI JE ; RIVERA CG et al.** Anti-angiogenic peptides for cancer therapeutics [J. *Curr Pharm Biotechnol,* 2011, vol. 12 (8), 1101-1116
- **BIRD et al.** *Science,* 1988, vol. 242, 423-426
- **HUSTON et al.** *Proc. Natl. Acad. Sci. USA,* 1988, vol. 85, 5879-5883
- **PLUCKTHUN.** The Pharmacology of Monoclonal Antibodies. Springer-Verlag, 1994, 269-315
- **NEEDLEMAN et al.** *J. Mol. Biol.,* 1970, vol. 48, 443-453
- **E. MEYERS ; W. MILLER.** *Comput. Appl. Biosci.,* 1988, vol. 4, 11-17
- **NEEDLEMAN ; WUNSCH.** *J. Mol. Biol.,* 1970, vol. 48, 444-453
- Remington's Pharmaceutical Sciences. Mack Publishing Company, 1995
- **J. SAMBROOK et al.** Molecular Cloning: A Laboratory Manual. Cold Spring Harbor Laboratory Press, 1989
- **F. M. AUSUBEL et al.** Short Protocols in Molecular Biology. John Wiley & Sons, Inc, 1995
- **PIRALLA et al.** *J Clin Microbiol,* 2015, vol. 53 (5), 1725-1726
- **YANG et al.** *Clin Vaccine Immunol,* 2014, vol. 21 (3), 312-320
- **HOU et al.** *J Virol Methods,* 2015, vol. 215-216, 56-60
- **HOU et al.** *Virus Res,* 2015, vol. 205, 41-44
- **YANG et al.** *Virus Res,* 2015, vol. 210, 165-168
- **HADAC E M ; KELLY E J ; RUSSELL S J.** *Mol Ther,* 2011, vol. 19 (6), 1041-1047

SEQUENCE LISTING

<110> XIAMEN UNIVERSITY; YANG SHENG TANG COMPANY, LTD.

<120> A VIRUS FOR TREATMENT OF TUMOR

<130> IEC170083PCT

<160> 20

<170> PatentIn version 3.5

<210> 1
<211> 7383
<212> DNA
<213> Artificial Sequence

<220>
<223> cDNA sequence of wild type EV-D68 (EV-D68-WT)

<400> 1
taatacgact cactataggt taaaacagcc ttggggttgt tcccactcca agggcccacg 60

tggcggctag tactctggta cttcggtacc tttgtacgcc tgttttatct cccttcccaa 120

tgtaacttag aagttcttaa atcaatgctc aataggtggg gcgcaaacca gcgctctcat 180

gagcaagcac tcctgtctcc ccggtgaggt tgtataaact gttcccacgg ttgaaaacaa 240

cctatccgtt atccgctata gtacttcgag aaacctagta ccacctttgg attgttgacg 300

cgttgcgctc agcacactaa cccgtgtgta gcttgggtcg atgagtctgg acatacctca 360

ctggcgacag tggtccaggc tgcgttggcg gcctactcat ggtgaaagcc atgagacgct 420

agacatgaac aaggtgtgaa gagtctattg agctactata gagtcctccg gcccctgaat 480

gcggctaatc ctaaccatgg agcaagtgct cacaggccag tgagttgctt gtcgtaatgc 540

gcaagtccgt ggcggaaccg actactttgg gtgtccgtgt ttcacttttt acttttatga 600

ctgcttatgg tgacaatttg atattgttac catttagctt gtcaaatcaa ttgcaaaaga 660

tcctaaatct tatttatcaa cttgcatctt gataacttta atttgaaaat tttaacaatg 720

ggagctcagg ttactagaca acaaactggc actcatgaaa atgccaacat tgccacaaat 780

ggatctcata tcacatacaa tcagataaac ttttacaagg atagctatgc ggcttcagcc 840

agcaagcagg atttttcaca ggacccatca aaattcactg aaccagtagt ggaaggttta 900

aaagcagggg cgccagtttt gaaatctcct agtgctgagg catgtggcta cagtgataga 960

gtattacagc tcaaattagg aaattcagct attgtcaccc aggaagcagc gaactactgc 1020

tgcgcttatg gtgaatggcc caattactta ccagaccatg aagcagtagc cattgataaa 1080

cctacacaac cagaaactgc tacagataga ttctacactt tgaaatcagt caaatgggaa 1140

actggaagca caggatggtg gtggaaacta cccgatgcac tgaataatat aggcatgttt 1200

ggacagaatg tgcagcatca ctacctatat agatctggtt tcttgattca tgtgcagtgt 1260

aatgccacaa aattccatca aggtgcctta ttagtggtag caattccaga acatcagagg 1320
ggagcgcaca acaccaacac tagcccaggg tttgatgata taatgaaagg tgaagaagga 1380
gggaccttca atcatccata tgtccttgat gatggaacat cattggcttg tgcgacgata 1440
tttccacatc agtggataaa tctgagaacc aacaattcag caacaattgt tcttccctgg 1500
atgaatgctg ctccaatgga tttcccactt agacataatc agtggacgct agcaataata 1560
ccagtggtgc cattaggtac gcgtacaaca tcaagtatgg tcccaataac agtttcaatc 1620
gctccaatgt gttgtgagtt taatggactt agacacgcca ttactcaagg tgtcccaaca 1680
tacctttac caggctcggg acaattccta acaactgatg atcatagctc tgcaccagct 1740
ctcccgtgtt tcaacccaac tccagaaatg catatcccag ggcaggtccg taacatgcta 1800
gaagtggtcc aagtggaatc aatgatggag attaataaca cagaaagtgc agttggcatg 1860
gagcgtctta aggttgatat atcagcattg acagatgtcg atcaattgtt attcaacatt 1920
ccactggaca tacagttgga tgggccactt agaaacactt tggtaggaaa catatctaga 1980
tattacactc attggtctgg atccctagaa atgacgttta tgttttgtgg cagcttcatg 2040
gcaacgggaa aattaatcct gtgctatact cctccaggtg gatcatgccc gacaaccaga 2100
gagaccgcca tgttaggtac acatattgtt tgggattttg gattacaatc tagtgtaacc 2160
ctgataatac cttggattag tggatcccac tacaggatgt ttaataatga tgctaagtca 2220
actaatgcca acgttggcta tgtcacttgt tttatgcaga ccaatctgat agtccccagt 2280
gaatcctctg acacgtgttc cttgataggg ttcatagcag caaaagatga tttctccctc 2340
agattaatga gagacagccc tgacattgga caactagacc atttacatgc agcagaggca 2400
gcctaccaga tcgagagcat catcaaaaca gcgaccgaca ctgtgaaaag tgagattaat 2460
gctgaacttg gtgtggtccc tagcttaaat gcagttgaaa caggtgcaac ttctaacact 2520
gaaccagaag aagccataca aactcgcaca gtgataaatc agcacggtgt atccgagact 2580
ctagtggaga attttctcag tagagcagct ttggtatcaa agagaagttt tgaatacaaa 2640
gatcatactt cgtctgcagc acaagcagac aagaactttt tcaaatggac aattaacacc 2700
agatcctttg tacagttaag aagaaaatta gaattattca catacctta g atttgatgct 2760
gagatcacta tactcacaac tgtagcagtg aatggtagtg gtaataatac atacgtgggt 2820
cttcctgact tgacactcca agcaatgttt gtacccactg gtgctcttac cccagaaaaa 2880
caggactcat tccactggca gtcaggcagt aatgctagtg tattctttaa aatctccgac 2940
cccccagcca gaataaccat accttttatg tgcattaact cagcatactc agttttttat 3000
gatggctttg ccggatttga gaaaaacggt ctgtatggaa taaatccagc tgacactatt 3060
ggtaacttat gtgttagaat agtgaatgaa caccaaccag ttggtttcac agtgaccgtt 3120
agggtttaca tgaagcctaa acacataaaa gcatgggcac cacgaccacc acgaactttg 3180

ccatatatga gtattgcaaa tgcaaattac aaaggtaaag aaagagcacc aaatgcgctc 3240
aatgctataa ttggcaatag agacagtgtc aaaaccatgc ctcataatat agtgaacact 3300
ggtccaggct tcggaggagt ttttgtaggg tctttcaaaa taatcaacta tcacttggcc 3360
actacagaag agagacagtc agctatctat gtggattggc aatcagacgt cttggttacc 3420
cccattgctg ctcatggaag gcaccaaata gcaagatgca agtgcaacac aggggtttac 3480
tattgtaggc acaaaaacag aagttacccg atttgctttg aaggcccagg gattcaatgg 3540
attgaacaaa atgaatatta cccagcaagg taccagacca atgtactatt ggcagttggt 3600
cctgcggaag caggagattg cggtggttta ctagtttgtc cacatggggt aatcggtctt 3660
cttacagcag gagggggtgg aattgtagct ttcactgata tcaggaattt gctatggtta 3720
gatactgatg ctatggaaca aggcattact gattatattc aaaatcttgg taatgccttt 3780
ggagcaggat ttacagaaac aatctctaat aaagccaagg aagtgcaaga tatgctaatt 3840
ggagagagtt cactattaga aaaattgtta aaagctctaa tcaaaatcat atcagcatta 3900
gtaattgtaa tcagaaactc agaagattta gtcacagtca cagccacact agcattgttg 3960
ggatgccatg attcaccatg gagctacttg aaacagaagg tatgttcata cttaggtatt 4020
ccttatgtac ctagacaggg tgaatcgtgg cttaagaaat tcacagaggc atgcaatgct 4080
cttagaggtc tggattggct atcgcaaaag atagataaat tcatcaactg gcttaaaacc 4140
aaaatattac cagaagctag ggagaaatat gaatttgtgc aaaggctcaa acagttaccg 4200
gtgatagaaa accaagttag tacaatcgag catagctgcc caacaacaga acaacaacag 4260
gccttattca acaacgtcca atactattca cactactgta gaaagtacgc accactttac 4320
gcagtggaag caaagagggt agtagctctt gaaaagaaaa taaacaacta catccagttc 4380
aagtccaaat ctcgcattga accggtttgt ttaataatac atggctctcc aggaactggc 4440
aagtcagtgg cttcaaattt aattgccagg gctatcacag agaaattggg gggggacatt 4500
tattccttgc ctccagaccc taaatatttt gatggataca aacagcaaac agtggtcctc 4560
atggatgatt taatgcaaaa tccagatggg aatgacatat ctatgttctg ccaaatggtc 4620
tccactgtag atttcatacc cccaatggct agtttggagg aaaaaggaac tctatacacc 4680
agtccatttt taatagctac taccaatgct ggctcaatac atgcaccaac tgtatcagac 4740
tcaaaggctt tgtcacgcag atttaaattt gacgtggaca ttgaagtcac agattcatac 4800
aaggactcaa ataaattgga tatgtcaagg gcagtcgaga tgtgcaaacc agatggctgt 4860
gcccccacca attacaaaag atgctgccca ttgatctgtg gaaaggctat ccaattcaga 4920
gatcgcagaa ctaatgcaag atccactatt gatatgctag taactgatat tataaaggaa 4980
tatagaacca gaaacagtac acaggataag ctggaagctc tgtttcaggg gcctccacag 5040

tttaaagaga tcaaaatttc agtcacccca gatacaccag ctcctgatgc tataaatgac 5100
cttcttaggt cagtggattc tcaagaagtt agggattatt gccaaaagaa aggatggatt 5160
gtagtacacc catcaaatga gctaatagta gaaaaacaca ttagtagagc ttttattact 5220
ctacaagcca ttgccacctt tgtatcaata gctggtgtag tttatgttat atacaaactt 5280
tttgctggca ttcagggtcc atacacagga atccccaatc ctaaacctaa agtaccctct 5340
ctcagaacag ctaaagtgca aggaccaggg ttcgattttg cacaagccat aatgaagaaa 5400
aataccgtca ttgcaaggac tgaaaagggt gagttcacca tgctgggtgt atatgatagg 5460
gtagcggtca tccccacaca cgcatctgtt ggagaaacca tttacattaa tgatgtagag 5520
actaaagttt tagatgcgtg tgcacttaga gacttgactg atacaaactt agagataacc 5580
atagtcaaat tagaccgtaa tcaaaaattt agagatatca gacattttct gcccagatat 5640
gaggatgatt acaatgacgc tgtgcttagc gtacatacat caaaattccc aaatatgtat 5700
atcccagttg gacaagtcac caattatggc ttcttgaacc taggtggtac accgacgcac 5760
cgcattttaa tgtataactt cccaacaaga gctggccagt gtggtggtgt ggtgacaact 5820
acaggtaagg tgataggaat acatgtaggt ggaaatggag ctcaaggatt tgcagcaatg 5880
ctactacact cttacttttc cgatacacaa ggtgagatag ttagtagtga aaagagtggg 5940
gtgtgcatta acgcaccggc aaagactaaa ctccaaccta gtgttttcca tcaagttttt 6000
gaaggttcaa aggaaccagc agttctcaat ccaaaagatc ctaggcttaa aacagatttc 6060
gaggaggcca ttttctcaaa gtacacaggt aacaaaatta tgttaatgga tgagtacatg 6120
gaagaggcag tggatcatta tgtggggtgt ttagaaccat tagacatcag tgtggatccc 6180
ataccccctgg aaagtgccat gtatggaatg gatggccttg aggcattaga cttaactacc 6240
agtgcaggat tcccttactt actacaaggg aagaagaaaa gggatatatt taatagacat 6300
actagagaca ccagtgaaat gacaaaaatg ttagagaaat atggagttga cctacctttt 6360
gtaacctttg taaaagatga gcttagatca agagaaaaag ttgaaaaagg gaaatcacgc 6420
ctgattgagg ccagttcctt gaatgactca gttgctatga gagttgcctt tggaaacctt 6480
tacgccacat ttcacaacaa tccaggtaca gcaactggta gtgcagttgg ttgtgatcca 6540
gatatatttt ggtcaaaaat ccctattttg ttagatggag aaatctttgc ttttgactac 6600
actggttatg atgctagttt gtcaccagtg tggtttgcct gcttaaagaa agttctaatt 6660
aagttaggtt acacacatca aacgtctttt atagattatt tgtgtcattc agtacattta 6720
tataaggaca aaaaatacat agttaatggt ggaatgccct ctggttcttc aggcaccagc 6780
atattcaaca ctatgatcaa caatataatc ataagaactt tattaattag ggtttacaaa 6840
ggcatagacc tggaccagtt caaaatgatt gcctatgggg atgatgttat tgctagctac 6900
ccacataaga ttgatccagg tttgctggca gaagcaggta aacagtatgg attagtaatg 6960

acgccagcag acaaaggaac cagttttatt gacacaaatt gggaaaatgt aactttctta 7020

aaaagatatt tcagagcaga tgatcaatac ccctttctca tacatccagt gatgccaatg 7080

aaagagatac atgaatctat tagatggact aaagatccca gaaacacaca ggatcatgtt 7140

aggtctttgt gctacctcgc atggcataat ggagaggagg cttataatga attttgcaga 7200

aaaatcagaa gtgtgcctgt gggaagagca ttgacactac ctgcatactc tagtcttaga 7260

cggaaatggt tagattcgtt ctagacaact ctaattgaaa cccaagttat agttactttc 7320

atttagaggt aaattttggt cacttggggg ccaaaaaaaa aaaaaaaaaa aaaaaaagtc 7380

gac 7383

<210> 2
<211> 507
<212> RNA
<213> Artificial Sequence

<220>
<223> RNA sequence of the internal ribosome entry site of HRV2

<400> 2
aacuuagaag uuuuucacaa agaccaauag ccgguaauca gccagauuac ugaaggucaa 60

gcacuucugu uuccccgguc aauguugaua ugcuccaaca gggcaaaaac aacugcgauc 120

guuaaccgca aagcgccuac gcaaagcuua guagcaucuu ugaaaucguu uggcuggucg 180

auccgccauu uccccuggua gaccuggcag augaggcuag aaauacccca cuggcgacag 240

uguucuagcc ugcguggcug ccugcacacc cuauggguga gaagccaaac aauggacaag 300

gugugaagag ccccgugugc ucgcuuugag uccuccggcc ccugaaugug gcuaaccuua 360

acccugcagc uagagcacgu aacccaaugu guaucuaguc guaaugagca auugcgggau 420

gggaccaacu acuuugggug uccguguuuc acuuuuuccu uuauauuugc uuauggugac 480

aauauauaca auauauauau uggcacc 507

<210> 3
<211> 22
<212> RNA
<213> Artificial Sequence

<220>
<223> RNA sequence of the target sequence of miR-133

<400> 3
acagcugguu gaaggggacc aa 22

<210> 4
<211> 22
<212> RNA
<213> Artificial Sequence

<220>
<223> RNA sequence of the target sequence of miR-206

<400> 4
ccacacacuu ccuuacauuc ca 22

<210> 5
<211> 102
<212> RNA
<213> Artificial Sequence

<220>
<223> RNA sequence of tandem sequence of miR-133 target sequence and miR-206 target sequence

<400> 5
acagcugguu gaaggggacc aacgauacag cugguugaag ggaccaaac cgguccacac 60

acuuccuuac auuccaucac ccacacacuu ccuuacauuc ca 102

<210> 6
<211> 435
<212> DNA
<213> Artificial Sequence

<220>
<223> DNA sequence of GM-CSF gene

<400> 6
atgtggctgc agagcctgct gctcttgggc actgtggcct gcagcatctc tgcacccgcc 60

cgctcgccca gccccagcac gcagccctgg gagcatgtga atgccatcca ggaggcccgg 120

cgtctcctga acctgagtag agacactgct gctgagatga atgaaacagt agaagtcatc 180

tcagaaatgt ttgacctcca ggagccgacc tgcctacaga cccgcctgga gctgtacaag 240

cagggcctgc ggggcagcct caccaagctc aagggcccct tgaccatgat ggccagccac 300

tacaagcagc actgccctcc aaccccggaa acttcctgtg caacccagat tatcaccttt 360

gaaagtttca aagagaacct gaaggacttt ctgcttgtca tcccctttga ctgctgggag 420

ccagtccagg agtga 435

<210> 7
<211> 795
<212> DNA
<213> Artificial Sequence

<220>
<223> DNA sequence encoding Anti-PD-1 scFv

<400> 7
atgaagcacc tgtggttctt cctgctgctg gtggccgctc ctaggtgggt gctgtcccag 60

gtgcagctgg tgcagagcgg cgtggaggtg aagaagcccg gcgcttccgt gaaggtgtcc 120

tgcaaggcct ccggctacac cttcaccaac tactacatgt actgggtgag gcaggcccct 180

ggacagggac tggagtggat gggcggcatc aacccttcca acggcggcac caacttcaac 240

gagaagttca agaaccgggt gaccctgacc accgactcct ccaccaccac cgcctacatg 300

gagctgaagt ccctgcagtt tgacgacacc gccgtgtact actgcgccag gagggactac 360

cggttcgaca tgggcttcga ctactggggc cagggcacaa ccgtgaccgt gtccagcgga 420

ggtggcggat ctggaggggg tggtagcggt ggaggcggga gtgagatcgt gctgacccag 480

tcccctgcta cactgtccct gtcccccggc gagagggcta cactgagctg cagggcctcc 540

aagggcgtgt ccacctccgg ctactcctac ctgcactggt accagcagaa gcctggacag 600

gctcccaggc tgctgatcta cctggcctcc tacctggagt ccggcgtgcc tgctaggttt 660

tccggcagcg gcagcggcac cgatttcacc ctgaccatct cctccctgga gcccgaggac 720

ttcgccgtgt actactgcca gcactccagg gatctgcctc tgaccttcgg cggcggcacc 780

aaggtggaga tcaag 795

<210> 8
<211> 7306
<212> DNA
<213> Artificial Sequence

<220>
<223> cDNA sequence of EV-D68-HRV2

<400> 8
taatacgact cactataggt taaaacagcc ttggggttgt tcccactcca agggcccacg 60

tggcggctag tactctggta cttcggtacc tttgtacgcc tgttttatct cccttcccaa 120

tgtaacttag aagaacttag aagtttttca caaagaccaa tagccggtaa tcagccagat 180

tactgaaggt caagcacttc tgtttccccg gtcaatgttg atatgctcca acagggcaaa 240

aacaactgcg atcgttaacc gcaaagcgcc tacgcaaagc ttagtagcat ctttgaaatc 300

gtttggctgg tcgatccgcc atttcccctg gtagacctgg cagatgaggc tagaaatacc 360

ccactggcga cagtgttcta gcctgcgtgg ctgcctgcac accctatggg tgtgaagcca 420

aacaatggac aaggtgtgaa gagccccgtg tgctcgcttt gagtcctccg gcccctgaat 480

gtggctaacc ttaaccctgc agctagagca cgtaacccaa tgtgtatcta gtcgtaatga 540

gcaattgcgg gatgggacca actactttgg gtgtccgtgt ttcacttttt cctttatatt 600

tgcttatggt gacaatatat acaatatata tattggcacc atgggagctc aggttactag 660

acaacaaact ggcactcatg aaaatgccaa cattgccaca aatggatctc atatcacata 720

caatcagata aacttttaca aggatagcta tgcggcttca gccagcaagc aggatttttc 780

acaggaccca tcaaaattca ctgaaccagt agtggaaggt ttaaaagcag ggcgccagt 840

tttgaaatct cctagtgctg aggcatgtgg ctacagtgat agagtattac agctcaaatt 900

aggaaattca gctattgtca cccaggaagc agcgaactac tgctgcgctt atggtgaatg 960

gcccaattac ttaccagacc atgaagcagt agccattgat aaacctacac aaccagaaac 1020
tgctacagat agattctaca ctttgaaatc agtcaaatgg gaaactggaa gcacaggatg 1080
gtggtggaaa ctacccgatg cactgaataa tataggcatg tttggacaga atgtgcagca 1140
tcactaccta tatagatctg gtttcttgat tcatgtgcag tgtaatgcca caaaattcca 1200
tcaaggtgcc ttattagtgg tagcaattcc agaacatcag aggggagcgc acaacaccaa 1260
cactagccca gggtttgatg atataatgaa aggtgaagaa ggagggacct tcaatcatcc 1320
atatgtcctt gatgatggaa catcattggc ttgtgcgacg atatttccac atcagtggat 1380
aaatctgaga accaacaatt cagcaacaat tgttcttccc tggatgaatg ctgctccaat 1440
ggatttccca cttagacata atcagtggac gctagcaata ataccagtgg tgccattagg 1500
tacgcgtaca acatcaagta tggtcccaat aacagtttca atcgctccaa tgtgttgtga 1560
gtttaatgga cttagacacg ccattactca aggtgtccca acataccttt taccaggctc 1620
gggacaattc ctaacaactg atgatcatag ctctgcacca gctctcccgt gtttcaaccc 1680
aactccagaa atgcatatcc cagggcaggt ccgtaacatg ctagaagtgg tccaagtgga 1740
atcaatgatg gagattaata acacagaaag tgcagttggc atggagcgtc ttaaggttga 1800
tatatcagca ttgacagatg tcgatcaatt gttattcaac attccactgg acatacagtt 1860
ggatgggcca cttagaaaca ctttggtagg aaacatatct agatattaca ctcattggtc 1920
tggatcccta gaaatgacgt ttatgttttg tggcagcttc atggcaacgg gaaaattaat 1980
cctgtgctat actcctccag gtggatcatg cccgacaacc agagagaccg ccatgttagg 2040
tacacatatt gtttgggatt ttggattaca atctagtgta accctgataa taccttggat 2100
tagtggatcc cactacagga tgtttaataa tgatgctaag tcaactaatg ccaacgttgg 2160
ctatgtcact tgttttatgc agaccaatct gatagtcccc agtgaatcct ctgacacgtg 2220
ttccttgata gggttcatag cagcaaaaga tgatttctcc ctcagattaa tgagagacag 2280
ccctgacatt ggacaactag accatttaca tgcagcagag gcagcctacc agatcgagag 2340
catcatcaaa acagcgaccg acactgtgaa aagtgagatt aatgctgaac ttggtgtggt 2400
ccctagctta aatgcagttg aaacaggtgc aacttctaac actgaaccag aagaagccat 2460
acaaactcgc acagtgataa atcagcacgg tgtatccgag actctagtgg agaattttct 2520
cagtagagca gctttggtat caaagagaag ttttgaatac aaagatcata cttcgtctgc 2580
agcacaagca gacaagaact ttttcaaatg gacaattaac accagatcct ttgtacagtt 2640
aagaagaaaa ttagaattat tcacatacct tagatttgat gctgagatca ctatactcac 2700
aactgtagca gtgaatggta gtggtaataa tacatacgtg ggtcttcctg acttgacact 2760
ccaagcaatg tttgtaccca ctggtgctct taccccagaa aaacaggact cattccactg 2820

gcagtcaggc agtaatgcta gtgtattctt taaaatctcc gaccccccag ccagaataac 2880
catacctttt atgtgcatta actcagcata ctcagttttt tatgatggct ttgccggatt 2940
tgagaaaaac ggtctgtatg gaataaatcc agctgacact attggtaact tatgtgttag 3000
aatagtgaat gaacaccaac cagttggttt cacagtgacc gttagggttt acatgaagcc 3060
taaacacata aaagcatggg caccacgacc accacgaact ttgccatata tgagtattgc 3120
aaatgcaaat tacaaaggta aagaaagagc accaaatgcg ctcaatgcta taattggcaa 3180
tagagacagt gtcaaaacca tgcctcataa tatagtgaac actggtccag gcttcggagg 3240
agtttttgta gggtctttca aaataatcaa ctatcacttg gccactacag aagagagaca 3300
gtcagctatc tatgtggatt ggcaatcaga cgtcttggtt acccccattg ctgctcatgg 3360
aaggcaccaa atagcaagat gcaagtgcaa cacaggggtt tactattgta ggcacaaaaa 3420
cagaagttac ccgatttgct ttgaaggccc agggattcaa tggattgaac aaaatgaata 3480
ttacccagca aggtaccaga ccaatgtact attggcagtt ggtcctgcgg aagcaggaga 3540
ttgcggtggt ttactagttt gtccacatgg ggtaatcggt cttcttacag caggaggggg 3600
tggaattgta gctttcactg atatcaggaa tttgctatgg ttagatactg atgctatgga 3660
acaaggcatt actgattata ttcaaaatct tggtaatgcc tttggagcag gatttacaga 3720
aacaatctct aataaagcca aggaagtgca agatatgcta attggagaga gttcactatt 3780
agaaaaattg ttaaaagctc taatcaaaat catatcagca ttagtaattg taatcagaaa 3840
ctcagaagat ttagtcacag tcacagccac actagcattg ttgggatgcc atgattcacc 3900
atggagctac ttgaaacaga aggtatgttc atacttaggt attccttatg tacctagaca 3960
gggtgaatcg tggcttaaga aattcacaga ggcatgcaat gctcttagag gtctggattg 4020
gctatcgcaa aagatagata aattcatcaa ctggcttaaa accaaaatat taccagaagc 4080
tagggagaaa tatgaatttg tgcaaaggct caaacagtta ccggtgatag aaaaccaagt 4140
tagtacaatc gagcatagct gcccaacaac agaacaacaa caggccttat tcaacaacgt 4200
ccaatactat tcacactact gtagaaagta cgcaccactt tacgcagtgg aagcaaagag 4260
ggtagtagct cttgaaaaga aaataaacaa ctacatccag ttcaagtcca aatctcgcat 4320
tgaaccggtt tgtttaataa tacatggctc tccaggaact ggcaagtcag tggcttcaaa 4380
tttaattgcc agggctatca cagagaaatt ggggggggac atttattcct tgcctccaga 4440
ccctaaatat tttgatggat acaaacagca aacagtggtc ctcatggatg atttaatgca 4500
aaatccagat gggaatgaca tatctatgtt ctgccaaatg gtctccactg tagatttcat 4560
acccccaatg gctagtttgg aggaaaaagg aactctatac accagtccat ttttaatagc 4620
tactaccaat gctggctcaa tacatgcacc aactgtatca gactcaaagg ctttgtcacg 4680
cagatttaaa tttgacgtgg acattgaagt cacagattca tacaaggact caaataaatt 4740

ggatatgtca agggcagtcg agatgtgcaa accagatggc tgtgccccca ccaattacaa 4800

aagatgctgc ccattgatct gtggaaaggc tatccaattc agagatcgca gaactaatgc 4860

aagatccact attgatatgc tagtaactga tattataaag gaatatagaa ccagaaacag 4920

tacacaggat aagctggaag ctctgtttca ggggcctcca cagtttaaag agatcaaaat 4980

ttcagtcacc ccagatacac cagctcctga tgctataaat gaccttctta ggtcagtgga 5040

ttctcaagaa gttagggatt attgccaaaa gaaaggatgg attgtagtac acccatcaaa 5100

tgagctaata gtagaaaaac acattagtag agcttttatt actctacaag ccattgccac 5160

ctttgtatca atagctggtg tagtttatgt tatatacaaa ctttttgctg gcattcaggg 5220

tccatacaca ggaatcccca atcctaaacc taaagtaccc tctctcagaa cagctaaagt 5280

gcaaggacca gggttcgatt ttgcacaagc cataatgaag aaaaataccg tcattgcaag 5340

gactgaaaag ggtgagttca ccatgctggg tgtatatgat agggtagcgg tcatccccac 5400

acacgcatct gttggagaaa ccatttacat taatgatgta gagactaaag ttttagatgc 5460

gtgtgcactt agagacttga ctgatacaaa cttagagata accatagtca aattagaccg 5520

taatcaaaaa tttagagata tcagacattt tctgcccaga tatgaggatg attacaatga 5580

cgctgtgctt agcgtacata catcaaaatt cccaaatatg tatatcccag ttggacaagt 5640

caccaattat ggcttcttga acctaggtgg tacaccgacg caccgcattt taatgtataa 5700

cttcccaaca agagctggcc agtgtggtgg tgtggtgaca actacaggta aggtgatagg 5760

aatacatgta ggtggaaatg gagctcaagg atttgcagca atgctactac actcttactt 5820

ttccgataca caaggtgaga tagttagtag tgaaaagagt ggggtgtgca ttaacgcacc 5880

ggcaaagact aaactccaac ctagtgtttt ccatcaagtt tttgaaggtt caaaggaacc 5940

agcagttctc aatccaaaag atcctaggct taaaacagat ttcgaggagg ccattttctc 6000

aaagtacaca ggtaacaaaa ttatgttaat ggatgagtac atggaagagg cagtggatca 6060

ttatgtgggg tgtttagaac cattagacat cagtgtggat cccatacccc tggaaagtgc 6120

catgtatgga atggatggcc ttgaggcatt agacttaact accagtgcag gattccctta 6180

cttactacaa gggaagaaga aaagggatat atttaataga catactagag acaccagtga 6240

aatgacaaaa atgttagaga aatatggagt tgacctacct tttgtaacct ttgtaaaaga 6300

tgagcttaga tcaagagaaa aagttgaaaa agggaaatca cgcctgattg aggccagttc 6360

cttgaatgac tcagttgcta tgagagttgc ctttggaaac ctttacgcca catttcacaa 6420

caatccaggt acagcaactg gtagtgcagt tggttgtgat ccagatatat tttggtcaaa 6480

aatccctatt ttgttagatg gagaaatctt tgcttttgac tacactggtt atgatgctag 6540

tttgtcacca gtgtggtttg cctgcttaaa gaaagttcta attaagttag gttacacaca 6600

tcaaacgtct tttatagatt atttgtgtca ttcagtacat ttatataagg acaaaaaata 6660

catagttaat ggtggaatgc cctctggttc ttcaggcacc agcatattca acactatgat 6720

caacaatata atcataagaa ctttattaat tagggtttac aaaggcatag acctggacca 6780

gttcaaaatg attgcctatg ggatgatgt tattgctagc tacccacata agattgatcc 6840

aggtttgctg gcagaagcag gtaaacagta tggattagta atgacgccag cagacaaagg 6900

aaccagtttt attgacacaa attgggaaaa tgtaactttc ttaaaaagat atttcagagc 6960

agatgatcaa taccccttc tcatacatcc agtgatgcca atgaaagaga tacatgaatc 7020

tattagatgg actaaagatc ccagaaacac acaggatcat gttaggtctt tgtgctacct 7080

cgcatggcat aatggagagg aggcttataa tgaattttgc agaaaaatca gaagtgtgcc 7140

tgtgggaaga gcattgacac tacctgcata ctctagtctt agacggaaat ggttagattc 7200

gttctagaca actctaattg aaacccaagt tatagttact ttcatttaga ggtaaatttt 7260

ggtcacttgg gggccaaaaa aaaaaaaaaa aaaaaaaaaa gtcgac 7306

<210> 9
<211> 7485
<212> DNA
<213> Artificial Sequence

<220>
<223> cDNA sequence of EV-D68-miR133&206T

<400> 9
taatacgact cactataggt taaaacagcc ttggggttgt tcccactcca agggcccacg 60

tggcggctag tactctggta cttcggtacc tttgtacgcc tgttttatct cccttcccaa 120

tgtaacttag aagttcttaa atcaatgctc aataggtggg gcgcaaacca gcgctctcat 180

gagcaagcac tcctgtctcc ccggtgaggt tgtataaact gttcccacgg ttgaaaacaa 240

cctatccgtt atccgctata gtacttcgag aaacctagta ccacctttgg attgttgacg 300

cgttgcgctc agcacactaa cccgtgtgta gcttgggtcg atgagtctgg acatacctca 360

ctggcgacag tggtccaggc tgcgttggcg gcctactcat ggtgaaagcc atgagacgct 420

agacatgaac aaggtgtgaa gagtctattg agctactata gagtcctccg gcccctgaat 480

gcggctaatc ctaaccatgg agcaagtgct cacaggccag tgagttgctt gtcgtaatgc 540

gcaagtccgt ggcggaaccg actactttgg gtgtccgtgt ttcacttttt acttttatga 600

ctgcttatgg tgacaatttg atattgttac catttagctt gtcaaatcaa ttgcaaaaga 660

tcctaaatct tatttatcaa cttgcatctt gataacttta atttgaaaat tttaacaatg 720

ggagctcagg ttactagaca acaaactggc actcatgaaa atgccaacat tgccacaaat 780

ggatctcata tcacatacaa tcagataaac ttttacaagg atagctatgc ggcttcagcc 840

agcaagcagg atttttcaca ggacccatca aaattcactg aaccagtagt ggaaggttta 900

aaagcagggg cgccagtttt gaaatctcct agtgctgagg catgtggcta cagtgataga 960
gtattacagc tcaaattagg aaattcagct attgtcaccc aggaagcagc gaactactgc 1020
tgcgcttatg gtgaatggcc caattactta ccagaccatg aagcagtagc cattgataaa 1080
cctacacaac cagaaactgc tacagataga ttctacactt tgaaatcagt caaatgggaa 1140
actggaagca caggatggtg gtggaaacta cccgatgcac tgaataatat aggcatgttt 1200
ggacagaatg tgcagcatca ctacctatat agatctggtt tcttgattca tgtgcagtgt 1260
aatgccacaa aattccatca aggtgcctta ttagtggtag caattccaga acatcagagg 1320
ggagcgcaca acaccaacac tagcccaggg tttgatgata taatgaaagg tgaagaagga 1380
gggaccttca atcatccata tgtccttgat gatggaacat cattggcttg tgcgacgata 1440
tttccacatc agtggataaa tctgagaacc aacaattcag caacaattgt tcttccctgg 1500
atgaatgctg ctccaatgga tttcccactt agacataatc agtggacgct agcaataata 1560
ccagtggtgc cattaggtac gcgtacaaca tcaagtatgg tcccaataac agtttcaatc 1620
gctccaatgt gttgtgagtt taatggactt agacacgcca ttactcaagg tgtcccaaca 1680
tacctttttac caggctcggg acaattccta acaactgatg atcatagctc tgcaccagct 1740
ctcccgtgtt tcaacccaac tccagaaatg catatcccag ggcaggtccg taacatgcta 1800
gaagtggtcc aagtggaatc aatgatggag attaataaca cagaaagtgc agttggcatg 1860
gagcgtctta aggttgatat atcagcattg acagatgtcg atcaattgtt attcaacatt 1920
ccactggaca tacagttgga tgggccactt agaaacactt tggtaggaaa catatctaga 1980
tattacactc attggtctgg atccctagaa atgacgttta tgttttgtgg cagcttcatg 2040
gcaacgggaa aattaatcct gtgctatact cctccaggtg gatcatgccc gacaaccaga 2100
gagaccgcca tgttaggtac acatattgtt tgggattttg gattacaatc tagtgtaacc 2160
ctgataatac cttggattag tggatcccac tacaggatgt ttaataatga tgctaagtca 2220
actaatgcca acgttggcta tgtcacttgt tttatgcaga ccaatctgat agtccccagt 2280
gaatcctctg acacgtgttc cttgataggg ttcatagcag caaaagatga tttctccctc 2340
agattaatga gagacagccc tgacattgga caactagacc atttacatgc agcagaggca 2400
gcctaccaga tcgagagcat catcaaaaca gcgaccgaca ctgtgaaaag tgagattaat 2460
gctgaacttg gtgtggtccc tagcttaaat gcagttgaaa caggtgcaac ttctaacact 2520
gaaccagaag aagccataca aactcgcaca gtgataaatc agcacggtgt atccgagact 2580
ctagtggaga attttctcag tagagcagct ttggtatcaa agagaagttt tgaatacaaa 2640
gatcatactt cgtctgcagc acaagcagac aagaactttt tcaaatggac aattaacacc 2700
agatcctttg tacagttaag aagaaaatta gaattattca catacettag atttgatgct 2760

gagatcacta tactcacaac tgtagcagtg aatggtagtg gtaataatac atacgtgggt 2820
cttcctgact tgacactcca agcaatgttt gtacccactg gtgctcttac cccagaaaaa 2880
caggactcat tccactggca gtcaggcagt aatgctagtg tattctttaa aatctccgac 2940
cccccagcca gaataaccat accttttatg tgcattaact cagcatactc agttttttat 3000
gatggctttg ccggatttga gaaaaacggt ctgtatggaa taaatccagc tgacactatt 3060
ggtaacttat gtgttagaat agtgaatgaa caccaaccag ttggtttcac agtgaccgtt 3120
agggtttaca tgaagcctaa acacataaaa gcatgggcac cacgaccacc acgaactttg 3180
ccatatatga gtattgcaaa tgcaaattac aaaggtaaag aaagagcacc aaatgcgctc 3240
aatgctataa ttggcaatag agacagtgtc aaaaccatgc ctcataatat agtgaacact 3300
ggtccaggct tcggaggagt ttttgtaggg tctttcaaaa taatcaacta tcacttggcc 3360
actacagaag agagacagtc agctatctat gtggattggc aatcagacgt cttggttacc 3420
cccattgctg ctcatggaag gcaccaaata gcaagatgca agtgcaacac aggggtttac 3480
tattgtaggc acaaaaacag aagttacccg atttgctttg aaggcccagg gattcaatgg 3540
attgaacaaa atgaatatta cccagcaagg taccagacca atgtactatt ggcagttggt 3600
cctgcggaag caggagattg cggtggttta ctagtttgtc cacatggggt aatcggtctt 3660
cttacagcag gaggggtgg aattgtagct ttcactgata tcaggaattt gctatggtta 3720
gatactgatg ctatggaaca aggcattact gattatattc aaaatcttgg taatgccttt 3780
ggagcaggat ttacagaaac aatctctaat aaagccaagg aagtgcaaga tatgctaatt 3840
ggagagagtt cactattaga aaaattgtta aaagctctaa tcaaaatcat atcagcatta 3900
gtaattgtaa tcagaaactc agaagattta gtcacagtca cagccacact agcattgttg 3960
ggatgccatg attcaccatg gagctacttg aaacagaagg tatgttcata cttaggtatt 4020
ccttatgtac ctagacaggg tgaatcgtgg cttaagaaat tcacagaggc atgcaatgct 4080
cttagaggtc tggattggct atcgcaaaag atagataaat tcatcaactg gcttaaaacc 4140
aaaatattac cagaagctag ggagaaatat gaatttgtgc aaaggctcaa acagttaccg 4200
gtgatagaaa accaagttag tacaatcgag catagctgcc caacaacaga acaacaacag 4260
gccttattca acaacgtcca atactattca cactactgta gaaagtacgc accactttac 4320
gcagtggaag caaagagggt agtagctctt gaaaagaaaa taaacaacta catccagttc 4380
aagtccaaat ctcgcattga accggtttgt ttaataatac atggctctcc aggaactggc 4440
aagtcagtgg cttcaaattt aattgccagg gctatcacag agaaattggg gggggacatt 4500
tattccttgc ctccagaccc taaatatttt gatggataca aacagcaaac agtggtcctc 4560
atggatgatt taatgcaaaa tccagatggg aatgacatat ctatgttctg ccaaatggtc 4620
tccactgtag atttcatacc cccaatggct agtttggagg aaaaaggaac tctatacacc 4680

agtccatttt taatagctac taccaatgct ggctcaatac atgcaccaac tgtatcagac 4740
tcaaaggctt tgtcacgcag atttaaattt gacgtggaca ttgaagtcac agattcatac 4800
aaggactcaa ataaattgga tatgtcaagg gcagtcgaga tgtgcaaacc agatggctgt 4860
gcccccacca attacaaaag atgctgccca ttgatctgtg gaaaggctat ccaattcaga 4920
gatcgcagaa ctaatgcaag atccactatt gatatgctag taactgatat tataaaggaa 4980
tatagaacca gaaacagtac acaggataag ctggaagctc tgtttcaggg gcctccacag 5040
tttaaagaga tcaaaatttc agtcacccca gatacaccag ctcctgatgc tataaatgac 5100
cttcttaggt cagtggattc tcaagaagtt agggattatt gccaaaagaa aggatggatt 5160
gtagtacacc catcaaatga gctaatagta gaaaaacaca ttagtagagc ttttattact 5220
ctacaagcca ttgccacctt tgtatcaata gctggtgtag tttatgttat atacaaactt 5280
tttgctggca ttcagggtcc atacacagga atccccaatc ctaaacctaa agtaccctct 5340
ctcagaacag ctaaagtgca aggaccaggg ttcgattttg cacaagccat aatgaagaaa 5400
aataccgtca ttgcaaggac tgaaaagggt gagttcacca tgctgggtgt atatgatagg 5460
gtagcggtca tccccacaca cgcatctgtt ggagaaacca tttacattaa tgatgtagag 5520
actaaagttt tagatgcgtg tgcacttaga gacttgactg atacaaactt agagataacc 5580
atagtcaaat tagaccgtaa tcaaaaattt agagatatca gacattttct gcccagatat 5640
gaggatgatt acaatgacgc tgtgcttagc gtacatacat caaaattccc aaatatgtat 5700
atcccagttg gacaagtcac caattatggc ttcttgaacc taggtggtac accgacgcac 5760
cgcattttaa tgtataactt cccaacaaga gctggccagt gtggtggtgt ggtgacaact 5820
acaggtaagg tgataggaat acatgtaggt ggaaatggag ctcaaggatt tgcagcaatg 5880
ctactacact cttacttttc cgatacacaa ggtgagatag ttagtagtga aaagagtggg 5940
gtgtgcatta acgcaccggc aaagactaaa ctccaaccta gtgttttcca tcaagttttt 6000
gaaggttcaa aggaaccagc agttctcaat ccaaaagatc ctaggcttaa aacagatttc 6060
gaggaggcca ttttctcaaa gtacacaggt aacaaaatta tgttaatgga tgagtacatg 6120
gaagaggcag tggatcatta tgtggggtgt ttagaaccat tagacatcag tgtggatccc 6180
ataccccctgg aaagtgccat gtatggaatg gatggccttg aggcattaga cttaactacc 6240
agtgcaggat tcccttactt actacaaggg aagaagaaaa gggatatatt taatagacat 6300
actagagaca ccagtgaaat gacaaaaatg ttagagaaat atggagttga cctacctttt 6360
gtaacctttg taaaagatga gcttagatca agagaaaaag ttgaaaaagg gaaatcacgc 6420
ctgattgagg ccagttcctt gaatgactca gttgctatga gagttgcctt tggaaacctt 6480
tacgccacat ttcacaacaa tccaggtaca gcaactggta gtgcagttgg ttgtgatcca 6540

gatatatttt ggtcaaaaat ccctattttg ttagatggag aaatctttgc ttttgactac 6600

actggttatg atgctagttt gtcaccagtg tggtttgcct gcttaaagaa agttctaatt 6660

aagttaggtt acacacatca aacgtctttt atagattatt tgtgtcattc agtacattta 6720

tataaggaca aaaaatacat agttaatggt ggaatgccct ctggttcttc aggcaccagc 6780

atattcaaca ctatgatcaa caatataatc ataagaactt tattaattag ggtttacaaa 6840

ggcatagacc tggaccagtt caaaatgatt gcctatgggg atgatgttat tgctagctac 6900

ccacataaga ttgatccagg tttgctggca gaagcaggta aacagtatgg attagtaatg 6960

acgccagcag acaaaggaac cagttttatt gacacaaatt gggaaaatgt aactttctta 7020

aaaagatatt tcagagcaga tgatcaatac ccctttctca tacatccagt gatgccaatg 7080

aaagagatac atgaatctat tagatggact aaagatccca gaaacacaca ggatcatgtt 7140

aggtctttgt gctacctcgc atggcataat ggagaggagg cttataatga attttgcaga 7200

aaaatcagaa gtgtgcctgt gggaagagca ttgacactac ctgcatactc tagtcttaga 7260

cggaaatggt tagattcgtt ctagacaact ctaacagctg gttgaagggg accaacgata 7320

cagctggttg aaggggacca aaccggtcca cacacttcct tacattccat cacccacaca 7380

cttccttaca ttccaattga aacccaagtt atagttactt tcatttagag gtaaattttg 7440

gtcacttggg ggccaaaaaa aaaaaaaaaa aaaaaaaaag tcgac 7485

<210> 10
<211> 7860
<212> DNA
<213> Artificial Sequence

<220>
<223> cDNA sequence of EV-D68-GM-CSF

<400> 10
taatacgact cactataggt taaaacagcc ttggggttgt tcccactcca agggcccacg 60

tggcggctag tactctggta cttcggtacc tttgtacgcc tgttttatct cccttcccaa 120

tgtaacttag aagttcttaa atcaatgctc aataggtggg gcgcaaacca gcgctctcat 180

gagcaagcac tcctgtctcc ccggtgaggt tgtataaact gttcccacgg ttgaaaacaa 240

cctatccgtt atccgctata gtacttcgag aaacctagta ccacctttgg attgttgacg 300

cgttgcgctc agcacactaa cccgtgtgta gcttgggtcg atgagtctgg acatacctca 360

ctggcgacag tggtccaggc tgcgttggcg gcctactcat ggtgaaagcc atgagacgct 420

agacatgaac aaggtgtgaa gagtctattg agctactata gagtcctccg gcccctgaat 480

gcggctaatc ctaaccatgg agcaagtgct cacaggccag tgagttgctt gtcgtaatgc 540

gcaagtccgt ggcggaaccg actactttgg gtgtccgtgt ttcacttttt acttttatga 600

ctgcttatgg tgacaatttg atattgttac catttagctt gtcaaatcaa ttgcaaaaga 660

```
tcctaaatct tatttatcaa cttgcatctt gataacttta atttgaaaat tttaacaatg      720
ggagctcagg ttactagaca acaaactggc actcatgaaa atgccaacat tgccacaaat      780
ggatctcata tcacatacaa tcagataaac ttttacaagg atagctatgc ggcttcagcc      840
agcaagcagg atttttcaca ggacccatca aaattcactg aaccagtagt ggaaggttta      900
aaagcagggg cgccagtttt gaaatctcct agtgctgagg catgtggcta cagtgataga      960
gtattacagc tcaaattagg aaattcagct attgtcaccc aggaagcagc gaactactgc     1020
tgcgcttatg gtgaatggcc caattactta ccagaccatg aagcagtagc cattgataaa     1080
cctacacaac cagaaactgc tacagataga ttctacactt tgaaatcagt caaatgggaa     1140
actggaagca caggatggtg gtggaaacta cccgatgcac tgaataatat aggcatgttt     1200
ggacagaatg tgcagcatca ctacctatat agatctggtt tcttgattca tgtgcagtgt     1260
aatgccacaa aattccatca aggtgcctta ttagtggtag caattccaga acatcagagg     1320
ggagcgcaca acaccaacac tagcccaggg tttgatgata taatgaaagg tgaagaagga     1380
gggaccttca atcatccata tgtccttgat gatggaacat cattggcttg tgcgacgata     1440
tttccacatc agtggataaa tctgagaacc aacaattcag caacaattgt tcttccctgg     1500
atgaatgctg ctccaatgga tttcccactt agacataatc agtggacgct agcaataata     1560
ccagtggtgc cattaggtac gcgtacaaca tcaagtatgg tcccaataac agtttcaatc     1620
gctccaatgt gttgtgagtt taatggactt agacacgcca ttactcaagg tgtcccaaca     1680
taccttttac caggctcggg acaattccta acaactgatg atcatagctc tgcaccagct     1740
ctcccgtgtt tcaacccaac tccagaaatg catatcccag ggcaggtccg taacatgcta     1800
gaagtggtcc aagtggaatc aatgatggag attaataaca cagaaagtgc agttggcatg     1860
gagcgtctta aggttgatat atcagcattg acagatgtcg atcaattgtt attcaacatt     1920
ccactggaca tacagttgga tgggccactt agaaacactt tggtaggaaa catatctaga     1980
tattacactc attggtctgg atccctagaa atgacgttta tgttttgtgg cagcttcatg     2040
gcaacgggaa aattaatcct gtgctatact cctccaggtg gatcatgccc gacaaccaga     2100
gagaccgcca tgttaggtac acatattgtt tgggattttg gattacaatc tagtgtaacc     2160
ctgataatac cttggattag tggatcccac tacaggatgt ttaataatga tgctaagtca     2220
actaatgcca acgttggcta tgtcacttgt tttatgcaga ccaatctgat agtccccagt     2280
gaatcctctg acacgtgttc cttgataggg ttcatagcag caaaagatga tttctccctc     2340
agattaatga gagacagccc tgacattgga caactagacc atttacatgc agcagaggca     2400
gcctaccaga tcgagagcat catcaaaaca gcgaccgaca ctgtgaaaag tgagattaat     2460
gctgaacttg gtgtggtccc tagcttaaat gcagttgaaa caggtgcaac ttctaacact     2520
```

gaaccagaag aagccataca aactcgcaca gtgataaatc agcacggtgt atccgagact 2580
ctagtggaga attttctcag tagagcagct ttggtatcaa agagaagttt tgaatacaaa 2640
gatcatactt cgtctgcagc acaagcagac aagaactttt tcaaatggac aattaacacc 2700
agatcctttg tacagttaag aagaaaatta gaattattca cataccttag atttgatgct 2760
gagatcacta tactcacaac tgtagcagtg aatggtagtg gtaataatac atacgtgggt 2820
cttcctgact tgacactcca agcaatgttt gtacccactg gtgctcttac cccagaaaaa 2880
caggactcat tccactggca gtcaggcagt aatgctagtg tattctttaa aatctccgac 2940
cccccagcca gaataaccat accttttatg tgcattaact cagcatactc agttttttat 3000
gatggctttg ccggatttga gaaaaacggt ctgtatggaa taaatccagc tgacactatt 3060
ggtaacttat gtgttagaat agtgaatgaa caccaaccag ttggtttcac agtgaccgtt 3120
agggtttaca tgaagcctaa acacataaaa gcatgggcac cacgaccacc acgaactttg 3180
ccatatatga gtattgcaaa tgcaaattac aaaggtaaag aaagagcacc aaatgcgctc 3240
aatgctataa ttggcaatag agacagtgtc aaaaccatgc ctcataatat agtgaacact 3300
ggtccaggct tctggctgca gagcctgctg ctcttgggca ctgtggcctg cagcatctct 3360
gcacccgccc gctcgcccag ccccagcacg cagccctggg agcatgtgaa tgccatccag 3420
gaggcccggc gtctcctgaa cctgagtaga gacactgctg ctgagatgaa tgaaacagta 3480
gaagtcatct cagaaatgtt tgacctccag gagccgacct gcctacagac ccgcctggag 3540
ctgtacaagc agggcctgcg gggcagcctc accaagctca agggcccctt gaccatgatg 3600
gccagccact acaagcagca ctgccctcca accccggaaa cttcctgtgc aacccagatt 3660
atcacctttg aaagtttcaa agagaacctg aaggactttc tgcttgtcat cccctttgac 3720
tgctgggagc cagtccagga gagtgtcaaa accatgcctc ataatatagt gaacactggt 3780
ccaggcttcg gaggagtttt tgtagggtct ttcaaaataa tcaactatca cttggccact 3840
acagaagaga gacagtcagc tatctatgtg gattggcaat cagacgtctt ggttaccccc 3900
attgctgctc atggaaggca ccaaatagca agatgcaagt gcaacacagg ggtttactat 3960
tgtaggcaca aaaacagaag ttacccgatt tgctttgaag gcccagggat tcaatggatt 4020
gaacaaaatg aatattaccc agcaaggtac cagaccaatg tactattggc agttggtcct 4080
gcggaagcag gagattgcgg tggtttacta gtttgtccac atggggtaat cggtcttctt 4140
acagcaggag ggggtggaat tgtagctttc actgatatca ggaatttgct atggttagat 4200
actgatgcta tggaacaagg cattactgat tatattcaaa atcttggtaa tgcctttgga 4260
gcaggattta cagaaacaat ctctaataaa gccaaggaag tgcaagatat gctaattgga 4320
gagagttcac tattagaaaa attgttaaaa gctctaatca aaatcatatc agcattagta 4380
attgtaatca gaaactcaga agatttagtc acagtcacag ccacactagc attgttggga 4440

tgccatgatt caccatggag ctacttgaaa cagaaggtat gttcatactt aggtattcct 4500
tatgtaccta gacagggtga atcgtggctt aagaaattca cagaggcatg caatgctctt 4560
agaggtctgg attggctatc gcaaaagata gataaattca tcaactggct taaaaccaaa 4620
atattaccag aagctaggga gaaatatgaa tttgtgcaaa ggctcaaaca gttaccggtg 4680
atagaaaacc aagttagtac aatcgagcat agctgcccaa caacagaaca acaacaggcc 4740
ttattcaaca acgtccaata ctattcacac tactgtagaa agtacgcacc actttacgca 4800
gtggaagcaa agagggtagt agctcttgaa aagaaaataa acaactacat ccagttcaag 4860
tccaaatctc gcattgaacc ggtttgttta ataatacatg gctctccagg aactggcaag 4920
tcagtggctt caaatttaat tgccagggct atcacagaga aattgggggg ggacatttat 4980
tccttgcctc cagaccctaa atattttgat ggatacaaac agcaaacagt ggtcctcatg 5040
gatgatttaa tgcaaaatcc agatgggaat gacatatcta tgttctgcca aatggtctcc 5100
actgtagatt tcataccccc aatggctagt ttggaggaaa aaggaactct atacaccagt 5160
ccatttttaa tagctactac caatgctggc tcaatacatg caccaactgt atcagactca 5220
aaggctttgt cacgcagatt taaatttgac gtggacattg aagtcacaga ttcatacaag 5280
gactcaaata aattggatat gtcaagggca gtcgagatgt gcaaaccaga tggctgtgcc 5340
cccaccaatt acaaaagatg ctgcccattg atctgtggaa aggctatcca attcagagat 5400
cgcagaacta atgcaagatc cactattgat atgctagtaa ctgatattat aaaggaatat 5460
agaaccagaa acagtacaca ggataagctg gaagctctgt ttcaggggcc tccacagttt 5520
aaagagatca aaatttcagt caccccagat acaccagctc ctgatgctat aaatgacctt 5580
cttaggtcag tggattctca agaagttagg gattattgcc aaaagaaagg atggattgta 5640
gtacacccat caaatgagct aatagtagaa aaacacatta gtagagcttt tattactcta 5700
caagccattg ccacctttgt atcaatagct ggtgtagttt atgttatata caaacttttt 5760
gctggcattc agggtccata cacaggaatc cccaatccta aacctaaagt accctctctc 5820
agaacagcta aagtgcaagg accagggttc gattttgcac aagccataat gaagaaaaat 5880
accgtcattg caaggactga aaagggtgag ttcaccatgc tgggtgtata tgatagggta 5940
gcggtcatcc ccacacacgc atctgttgga gaaaccattt acattaatga tgtagagact 6000
aaagttttag atgcgtgtgc acttagagac ttgactgata caaacttaga gataaccata 6060
gtcaaattag accgtaatca aaaatttaga gatatcagac attttctgcc cagatatgag 6120
gatgattaca atgacgctgt gcttagcgta catacatcaa aattcccaaa tatgtatatc 6180
ccagttggac aagtcaccaa ttatggcttc ttgaacctag gtggtacacc gacgcaccgc 6240
attttaatgt ataacttccc aacaagagct ggccagtgtg gtggtgtggt gacaactaca 6300

ggtaaggtga taggaataca tgtaggtgga aatggagctc aaggatttgc agcaatgcta 6360

ctacactctt acttttccga tacacaaggt gagatagtta gtagtgaaaa gagtggggtg 6420

tgcattaacg caccggcaaa gactaaactc caacctagtg ttttccatca agtttttgaa 6480

ggttcaaagg aaccagcagt tctcaatcca aaagatccta ggcttaaaac agatttcgag 6540

gaggccattt tctcaaagta cacaggtaac aaaattatgt taatggatga gtacatggaa 6600

gaggcagtgg atcattatgt ggggtgttta gaaccattag acatcagtgt ggatcccata 6660

cccctggaaa gtgccatgta tggaatggat ggccttgagg cattagactt aactaccagt 6720

gcaggattcc cttacttact acaagggaag aagaaaaggg atatatttaa tagacatact 6780

agagacacca gtgaaatgac aaaaatgtta gagaaatatg gagttgacct acctttgta 6840

acctttgtaa aagatgagct tagatcaaga gaaaaagttg aaaaagggaa atcacgcctg 6900

attgaggcca gttccttgaa tgactcagtt gctatgagag ttgcctttgg aaacctttac 6960

gccacatttc acaacaatcc aggtacagca actggtagtg cagttggttg tgatccagat 7020

atattttggt caaaaatccc tattttgtta gatggagaaa tctttgcttt tgactacact 7080

ggttatgatg ctagtttgtc accagtgtgg tttgcctgct taaagaaagt tctaattaag 7140

ttaggttaca cacatcaaac gtcttttata gattatttgt gtcattcagt acatttatat 7200

aaggacaaaa aatacatagt taatggtgga atgccctctg gttcttcagg caccagcata 7260

ttcaacacta tgatcaacaa tataatcata agaactttat taattagggt ttacaaaggc 7320

atagacctgg accagttcaa aatgattgcc tatggggatg atgttattgc tagctaccca 7380

cataagattg atccaggttt gctggcagaa gcaggtaaac agtatggatt agtaatgacg 7440

ccagcagaca aaggaaccag ttttattgac acaaattggg aaaatgtaac tttcttaaaa 7500

agatatttca gagcagatga tcaatacccc tttctcatac atccagtgat gccaatgaaa 7560

gagatacatg aatctattag atggactaaa gatcccagaa acacacagga tcatgttagg 7620

tctttgtgct acctcgcatg gcataatgga gaggaggctt ataatgaatt ttgcagaaaa 7680

atcagaagtg tgcctgtggg aagagcattg acactacctg catactctag tcttagacgg 7740

aaatggttag attcgttcta gacaactcta attgaaaccc aagttatagt tactttcatt 7800

tagaggtaaa ttttggtcac ttgggggcca aaaaaaaaaa aaaaaaaaaa aaaagtcgac 7860

<210> 11
<211> 8226
<212> DNA
<213> Artificial Sequence

<220>
<223> cDNA sequence of EV-D68-Anti-PD1

<400> 11
taatacgact cactataggt taaaacagcc ttggggttgt tcccactcca agggcccacg 60

tggcggctag tactctggta cttcggtacc tttgtacgcc tgttttatct cccttcccaa 120
tgtaacttag aagttcttaa atcaatgctc aataggtggg gcgcaaacca gcgctctcat 180
gagcaagcac tcctgtctcc ccggtgaggt tgtataaact gttcccacgg ttgaaaacaa 240
cctatccgtt atccgctata gtacttcgag aaacctagta ccacctttgg attgttgacg 300
cgttgcgctc agcacactaa cccgtgtgta gcttgggtcg atgagtctgg acatacctca 360
ctggcgacag tggtccaggc tgcgttggcg gcctactcat ggtgaaagcc atgagacgct 420
agacatgaac aaggtgtgaa gagtctattg agctactata gagtcctccg gcccctgaat 480
gcggctaatc ctaaccatgg agcaagtgct cacaggccag tgagttgctt gtcgtaatgc 540
gcaagtccgt ggcggaaccg actactttgg gtgtccgtgt ttcacttttt acttttatga 600
ctgcttatgg tgacaatttg atattgttac catttagctt gtcaaatcaa ttgcaaaaga 660
tcctaaatct tatttatcaa cttgcatctt gataacttta atttgaaaat tttaacaatg 720
ggagctcagg ttactagaca acaaactggc actcatgaaa atgccaacat tgccacaaat 780
ggatctcata tcacatacaa tcagataaac ttttacaagg atagctatgc ggcttcagcc 840
agcaagcagg atttttcaca ggacccatca aaattcactg aaccagtagt ggaaggttta 900
aaagcagggg cgccagtttt gaaatctcct agtgctgagg catgtggcta cagtgataga 960
gtattacagc tcaaattagg aaattcagct attgtcaccc aggaagcagc gaactactgc 1020
tgcgcttatg gtgaatggcc caattactta ccagaccatg aagcagtagc cattgataaa 1080
cctacacaac cagaaactgc tacagataga ttctacactt tgaaatcagt caaatgggaa 1140
actggaagca caggatggtg gtggaaacta cccgatgcac tgaataatat aggcatgttt 1200
ggacagaatg tgcagcatca ctacctatat agatctggtt tcttgattca tgtgcagtgt 1260
aatgccacaa aattccatca aggtgcctta ttagtggtag caattccaga acatcagagg 1320
ggagcgcaca acaccaacac tagcccaggg tttgatgata taatgaaagg tgaagaagga 1380
gggaccttca atcatccata tgtccttgat gatggaacat cattggcttg tgcgacgata 1440
tttccacatc agtggataaa tctgagaacc aacaattcag caacaattgt tcttccctgg 1500
atgaatgctg ctccaatgga tttcccactt agacataatc agtggacgct agcaataata 1560
ccagtggtgc cattaggtac gcgtacaaca tcaagtatgg tcccaataac agtttcaatc 1620
gctccaatgt gttgtgagtt taatggactt agacacgcca ttactcaagg tgtcccaaca 1680
tacctttttac caggctcggg acaattccta acaactgatg atcatagctc tgcaccagct 1740
ctcccgtgtt tcaacccaac tccagaaatg catatcccag ggcaggtccg taacatgcta 1800
gaagtggtcc aagtggaatc aatgatggag attaataaca cagaaagtgc agttggcatg 1860
gagcgtctta aggttgatat atcagcattg acagatgtcg atcaattgtt attcaacatt 1920

ccactggaca tacagttgga tgggccactt agaaacactt tggtaggaaa catatctaga 1980
tattacactc attggtctgg atccctagaa atgacgttta tgttttgtgg cagcttcatg 2040
gcaacgggaa aattaatcct gtgctatact cctccaggtg gatcatgccc gacaaccaga 2100
gagaccgcca tgttaggtac acatattgtt tgggattttg gattacaatc tagtgtaacc 2160
ctgataatac cttggattag tggatcccac tacaggatgt ttaataatga tgctaagtca 2220
actaatgcca acgttggcta tgtcacttgt tttatgcaga ccaatctgat agtccccagt 2280
gaatcctctg acacgtgttc cttgataggg ttcatagcag caaaagatga tttctccctc 2340
agattaatga gagacagccc tgacattgga caactagacc atttacatgc agcagaggca 2400
gcctaccaga tcgagagcat catcaaaaca gcgaccgaca ctgtgaaaag tgagattaat 2460
gctgaacttg gtgtggtccc tagcttaaat gcagttgaaa caggtgcaac ttctaacact 2520
gaaccagaag aagccataca aactcgcaca gtgataaatc agcacggtgt atccgagact 2580
ctagtggaga attttctcag tagagcagct ttggtatcaa agagaagttt tgaatacaaa 2640
gatcatactt cgtctgcagc acaagcagac aagaactttt tcaaatggac aattaacacc 2700
agatcctttg tacagttaag aagaaaatta gaattattca catacettag atttgatgct 2760
gagatcacta tactcacaac tgtagcagtg aatggtagtg gtaataatac atacgtgggt 2820
cttcctgact tgacactcca agcaatgttt gtacccactg gtgctcttac cccagaaaaa 2880
caggactcat tccactggca gtcaggcagt aatgctagtg tattctttaa aatctccgac 2940
cccccagcca gaataaccat accttttatg tgcattaact cagcatactc agtttttttat 3000
gatggctttg ccggatttga gaaaaacggt ctgtatggaa taaatccagc tgacactatt 3060
ggtaacttat gtgttagaat agtgaatgaa caccaaccag ttggtttcac agtgaccgtt 3120
agggtttaca tgaagcctaa acacataaaa gcatgggcac cacgaccacc acgaactttg 3180
ccatatatga gtattgcaaa tgcaaattac aaaggtaaag aaagagcacc aaatgcgctc 3240
aatgctataa ttggcaatag agacagtgtc aaaaccatgc ctcataatat agtgaacact 3300
ggtccaggct tcatgaagca cctgtggttc ttcctgctgc tggtggccgc tcctaggtgg 3360
gtgctgtccc aggtgcagct ggtgcagagc ggcgtggagg tgaagaagcc cggcgcttcc 3420
gtgaaggtgt cctgcaaggc ctccggctac accttcacca actactacat gtactgggtg 3480
aggcaggccc ctggacaggg actggagtgg atgggcggca tcaacccttc caacggcggc 3540
accaacttca acgagaagtt caagaaccgg gtgaccctga ccaccgactc ctccaccacc 3600
accgcctaca tggagctgaa gtccctgcag tttgacgaca ccgccgtgta ctactgcgcc 3660
aggagggact accggttcga catgggcttc gactactggg gccagggcac aaccgtgacc 3720
gtgtccagcg gaggtggcgg atctggaggg ggtggtagcg gtggaggcgg gagtgagatc 3780
gtgctgaccc agtcccctgc tacactgtcc ctgtccccg gcgagagggc tacactgagc 3840

tgcagggcct ccaagggcgt gtccacctcc ggctactcct acctgcactg gtaccagcag 3900
aagcctggac aggctcccag gctgctgatc tacctggcct cctacctgga gtccggcgtg 3960
cctgctaggt tttccggcag cggcagcggc accgatttca ccctgaccat ctcctccctg 4020
gagcccgagg acttcgccgt gtactactgc cagcactcca gggatctgcc tctgaccttc 4080
ggcggcggca ccaaggtgga gatcaagagt gtcaaaacca tgcctcataa tatagtgaac 4140
actggtccag gcttcggagg agtttttgta gggtctttca aaataatcaa ctatcacttg 4200
gccactacag aagagagaca gtcagctatc tatgtggatt ggcaatcaga cgtcttggtt 4260
acccccattg ctgctcatgg aaggcaccaa atagcaagat gcaagtgcaa cacaggggtt 4320
tactattgta ggcacaaaaa cagaagttac ccgatttgct ttgaaggccc agggattcaa 4380
tggattgaac aaaatgaata ttacccagca aggtaccaga ccaatgtact attggcagtt 4440
ggtcctgcgg aagcaggaga ttgcggtggt ttactagttt gtccacatgg ggtaatcggt 4500
cttcttacag caggaggggg tggaattgta gctttcactg atatcaggaa tttgctatgg 4560
ttagatactg atgctatgga acaaggcatt actgattata ttcaaaatct tggtaatgcc 4620
tttggagcag gatttacaga aacaatctct aataaagcca aggaagtgca agatatgcta 4680
attggagaga gttcactatt agaaaaattg ttaaaagctc taatcaaaat catatcagca 4740
ttagtaattg taatcagaaa ctcagaagat ttagtcacag tcacagccac actagcattg 4800
ttgggatgcc atgattcacc atggagctac ttgaaacaga aggtatgttc atacttaggt 4860
attccttatg tacctagaca gggtgaatcg tggcttaaga aattcacaga ggcatgcaat 4920
gctcttagag gtctggattg gctatcgcaa aagatagata aattcatcaa ctggcttaaa 4980
accaaaatat taccagaagc tagggagaaa tatgaatttg tgcaaaggct caaacagtta 5040
ccggtgatag aaaaccaagt tagtacaatc gagcatagct gcccaacaac agaacaacaa 5100
caggccttat tcaacaacgt ccaatactat tcacactact gtagaaagta cgcaccactt 5160
tacgcagtgg aagcaaagag ggtagtagct cttgaaaaga aaataaacaa ctacatccag 5220
ttcaagtcca aatctcgcat tgaaccggtt tgtttaataa tacatggctc tccaggaact 5280
ggcaagtcag tggcttcaaa tttaattgcc agggctatca cagagaaatt ggggggggac 5340
atttattcct tgcctccaga ccctaaatat tttgatggat acaaacagca aacagtggtc 5400
ctcatggatg atttaatgca aaatccagat gggaatgaca tatctatgtt ctgccaaatg 5460
gtctccactg tagatttcat acccccaatg gctagtttgg aggaaaaagg aactctatac 5520
accagtccat ttttaatagc tactaccaat gctggctcaa tacatgcacc aactgtatca 5580
gactcaaagg ctttgtcacg cagatttaaa tttgacgtgg acattgaagt cacagattca 5640
tacaaggact caaataaatt ggatatgtca agggcagtcg agatgtgcaa accagatggc 5700

tgtgccccca ccaattacaa aagatgctgc ccattgatct gtggaaaggc tatccaattc 5760
agagatcgca gaactaatgc aagatccact attgatatgc tagtaactga tattataaag 5820
gaatatagaa ccagaaacag tacacaggat aagctggaag ctctgtttca ggggcctcca 5880
cagtttaaag agatcaaaat ttcagtcacc ccagatacac cagctcctga tgctataaat 5940
gaccttctta ggtcagtgga ttctcaagaa gttagggatt attgccaaaa gaaaggatgg 6000
attgtagtac acccatcaaa tgagctaata gtagaaaaac acattagtag agcttttatt 6060
actctacaag ccattgccac ctttgtatca atagctggtg tagtttatgt tatatacaaa 6120
ctttttgctg gcattcaggg tccatacaca ggaatcccca atcctaaacc taaagtaccc 6180
tctctcagaa cagctaaagt gcaaggacca gggttcgatt ttgcacaagc cataatgaag 6240
aaaaataccg tcattgcaag gactgaaaag ggtgagttca ccatgctggg tgtatatgat 6300
agggtagcgg tcatccccac acacgcatct gttggagaaa ccatttacat taatgatgta 6360
gagactaaag ttttagatgc gtgtgcactt agagacttga ctgatacaaa cttagagata 6420
accatagtca aattagaccg taatcaaaaa tttagagata tcagacattt tctgcccaga 6480
tatgaggatg attacaatga cgctgtgctt agcgtacata catcaaaatt cccaaatatg 6540
tatatcccag ttggacaagt caccaattat ggcttcttga acctaggtgg tacaccgacg 6600
caccgcattt taatgtataa cttcccaaca agagctggcc agtgtggtgg tgtggtgaca 6660
actacaggta aggtgatagg aatacatgta ggtggaaatg gagctcaagg atttgcagca 6720
atgctactac actcttactt ttccgataca caaggtgaga tagttagtag tgaaaagagt 6780
ggggtgtgca ttaacgcacc ggcaaagact aaactccaac ctagtgtttt ccatcaagtt 6840
tttgaaggtt caaaggaacc agcagttctc aatccaaaag atcctaggct taaaacagat 6900
ttcgaggagg ccatttctc aaagtacaca ggtaacaaaa ttatgttaat ggatgagtac 6960
atggaagagg cagtggatca ttatgtgggg tgtttagaac cattagacat cagtgtggat 7020
cccatacccc tggaaagtgc catgtatgga atggatggcc ttgaggcatt agacttaact 7080
accagtgcag gattccctta cttactacaa gggaagaaga aaagggatat atttaataga 7140
catactagag acaccagtga aatgacaaaa atgttagaga aatatggagt tgacctacct 7200
tttgtaacct ttgtaaaaga tgagcttaga tcaagagaaa aagttgaaaa agggaaatca 7260
cgcctgattg aggccagttc cttgaatgac tcagttgcta tgagagttgc ctttggaaac 7320
ctttacgcca catttcacaa caatccaggt acagcaactg gtagtgcagt tggttgtgat 7380
ccagatatat tttggtcaaa aatccctatt ttgttagatg gagaaatctt tgcttttgac 7440
tacactggtt atgatgctag tttgtcacca gtgtggtttg cctgcttaaa gaaagttcta 7500
attaagttag gttacacaca tcaaacgtct tttatagatt atttgtgtca ttcagtacat 7560
ttatataagg acaaaaaata catagttaat ggtggaatgc cctctggttc ttcaggcacc 7620

agcatattca acactatgat caacaatata atcataagaa ctttattaat tagggtttac 7680

aaaggcatag acctggacca gttcaaaatg attgcctatg gggatgatgt tattgctagc 7740

tacccacata agattgatcc aggtttgctg gcagaagcag gtaaacagta tggattagta 7800

atgacgccag cagacaaagg aaccagtttt attgacacaa attgggaaaa tgtaactttc 7860

ttaaaaagat atttcagagc agatgatcaa tacccctttc tcatacatcc agtgatgcca 7920

atgaaagaga tacatgaatc tattagatgg actaaagatc ccagaaacac acaggatcat 7980

gttaggtctt tgtgctacct cgcatggcat aatggagagg aggcttataa tgaattttgc 8040

agaaaaatca gaagtgtgcc tgtgggaaga gcattgacac tacctgcata ctctagtctt 8100

agacggaaat ggttagattc gttctagaca actctaattg aaacccaagt tatagttact 8160

ttcatttaga ggtaaatttt ggtcacttgg gggccaaaaa aaaaaaaaaa aaaaaaaaaa 8220

gtcgac 8226

<210> 12
<211> 7383
<212> RNA
<213> Artificial Sequence

<220>
<223> Genomic sequence of EV-D68-WT

<400> 12
uaauacgacu cacuauaggu uaaaacagcc uuggggguugu ucccacucca agggcccacg 60

uggcggcuag uacucuggua cuucgguacc uuuguacgcc uguuuuaucu cccuucccaa 120

uguaacuuag aaguucuuaa aucaaugcuc aauaggugggg gcgcaaacca gcgcucucau 180

gagcaagcac uccugucucc ccggugaggu uguauaaacu guucccacgg uugaaaacaa 240

ccuauccguu auccgcuaua guacuucgag aaaccuagua ccaccuuugg auuguugacg 300

cguugcgcuc agcacacuaa cccgugugua gcuuggggucg augagucugg acauaccuca 360

cuggcgacag ugguccaggc ugcguuggcg gccuacucau ggugaaagcc augagacgcu 420

agacaugaac aaggugugaa gagucuauug agcuacuaua gaguccuccg gccccugaau 480

gcggcuaauc cuaaccaugg agcaagugcu cacaggccag ugaguugcuu gucguaaugc 540

gcaaguccgu ggcggaaccg acuacuuugg guguccgugu uucacuuuuu acuuuuauga 600

cugcuuaugg ugacaauuug auauuguuac cauuuagcuu gucaaaucaa uugcaaaaga 660

uccuaaaucu uauuuaucaa cuugcaucuu gauaacuuua auuugaaaau uuuaacaaug 720

ggagcucagg uuacuagaca acaaacuggc acucaugaaa augccaacau ugccacaaau 780

ggaucucaua ucacauacaa ucagauaaac uuuuacaagg auagcuaugc ggcuucagcc 840

agcaagcagg auuuuucaca ggacccauca aaauucacug aaccaguagu ggaagguuua 900

aaagcagggg cgccaguuuu gaaaucuccu agugcugagg cauguggcua cagugauaga 960
guauuacagc ucaaauuagg aaauucagcu auugucaccc aggaagcagc gaacuacugc 1020
ugcgcuuaug gugaauggcc caauuacuua ccagaccaug aagcaguagc cauugauaaa 1080
ccuacacaac cagaaacugc uacagauaga uucuacacuu ugaaaucagu caaaugggaa 1140
acuggaagca caggauggug guggaaacua cccgaugcac ugaauaauau aggcauguuu 1200
ggacagaaug ugcagcauca cuaccuauau agaucugguu ucuugauuca ugugcagugu 1260
aaugccacaa aauuccauca aggugccuua uuagugguag caauuccaga acaucagagg 1320
ggagcgcaca acaccaacac uagcccaggg uuugaugaua uaaugaaagg ugaagaagga 1380
gggaccuuca aucauccaua uguccuugau gauggaacau cauuggcuug ugcgacgaua 1440
uuuccacauc aguggauaaa ucugagaacc aacaauucag caacaauugu ucuucccugg 1500
augaaugcug cuccaaugga uuucccacuu agacauaauc aguggacgcu agcaauaaua 1560
ccagugguge cauuagguac gcguacaaca ucaaguaugg ucccaauaac aguuucaauc 1620
gcuccaaugu guugugaguu uaauggacuu agacacgcca uuacucaagg ugucccaaca 1680
uaccuuuuac caggcucggg acaauuccua acaacugaug aucauagcuc ugcaccagcu 1740
cucccguguu ucaacccaac uccagaaaug cauaucccag ggcagguccg uaacaugcua 1800
gaaguggucc aaguggaauc aaugauggag auuaauaaca cagaaagugc aguuggcaug 1860
gagcgucuua agguugauau aucagcauug acagaugucg aucaauuguu auucaacauu 1920
ccacuggaca uacaguugga ugggccacuu agaaacacuu ugguaggaaa cauaucuaga 1980
uauuacacuc auuggucugg aucccuagaa augacguuua uguuuugugg cagcuucaug 2040
gcaacgggaa aauuaauccu gugcuauacu ccuccaggug gaucaugccc gacaaccaga 2100
gagaccgcca uguuagguac acauauuguu ugggauuuug gauuacaauc uaguguaacc 2160
cugauaauac cuuggauuag uggaucccac uacaggaugu uuaauaauga ugcuaaguca 2220
acuaaugcca acguuggcua ugucacuugu uuuaugcaga ccaaucugau aguccccagu 2280
gaauccucug acacguguuc cuugauaggg uucauagcag caaaagauga uuucuccuc 2340
agauuaauga gagacagccc ugacauugga caacuagacc auuuacaugc agcagaggca 2400
gccuaccaga ucgagagcau caucaaaaca gcgaccgaca cugugaaaag ugagauuaau 2460
gcugaacuug guguggucc uagcuuaaau gcaguugaaa caggugcaac uucuaacacu 2520
gaaccagaag aagccauaca aacucgcaca gugauaaauc agcacggugu auccgagacu 2580
cuaguggaga auuuucucag uagagcagcu uugguaucaa agagaaguuu ugaauacaaa 2640
gaucauacuu cgucugcagc acaagcagac aagaacuuuu ucaaauggac aauuaacacc 2700
agauccuuug uacaguuaag aagaaaauua gaauuauuca cauaccuuag auuugaugcu 2760
gagaucacua uacucacaac uguagcagug aaugguagug guaauaauac auacgugggu 2820

cuuccugacu ugacacucca agcaauguuu guacccacug gugcucuuac cccagaaaaa 2880
caggacucau uccacuggca gucaggcagu aaugcuagug uauucuuuaa aaucuccgac 2940
cccccagcca gaauaaccau accuuuuaug ugcauuaacu cagcauacuc aguuuuuuau 3000
gauggcuuug ccggauuuga gaaaaacggu cuguauggaa uaaauccagc ugacacuauu 3060
gguaacuuau guguuagaau agugaaugaa caccaaccag uugguuucac agugaccguu 3120
aggguuuaca ugaagccuaa acacauaaaa gcaugggcac cacgaccacc acgaacuuug 3180
ccauauauga guauugcaaa ugcaaauuac aaagguaaag aaagagcacc aaaugcgcuc 3240
aaugcuauaa uuggcaauag agacaguguc aaaaccaugc cucauaauau agugaacacu 3300
gguccaggcu ucggaggagu uuuuguaggg ucuuucaaaa uaaucaacua ucacuuggcc 3360
acuacagaag agagacaguc agcuaucuau guggauuggc aaucagacgu cuugguuacc 3420
cccauugcug cucauggaag gcaccaaaua gcaagaugca agugcaacac aggguuuac 3480
uauuguaggc acaaaaacag aaguuacccg auuugcuuug aaggcccagg gauucaaugg 3540
auugaacaaa augaauauua cccagcaagg uaccagacca auguacuauu ggcaguuggu 3600
ccugcggaag caggagauug cgguggguua cuaguuuguc cacaugggu aaucggucuu 3660
cuuacagcag gagggggugg aauuguagcu uucacugaua ucaggaauuu gcuaugguua 3720
gauacugaug cuauggaaca aggcauuacu gauuauauuc aaaaucuugg uaaugccuuu 3780
ggagcaggau uuacagaaac aaucucuaau aaagccaagg aagugcaaga uaugcuaauu 3840
ggagagaguu cacuauuaga aaaauuguua aaagcucuaa ucaaaaucau aucagcauua 3900
guaauuguaa ucagaaacuc agaagauuua gucacaguca cagccacacu agcauuguug 3960
ggaugccaug auucaccaug gagcuacuug aaacagaagg uauguucaua cuuagguauu 4020
ccuuauguac cuagacaggg ugaaucgugg cuuaagaaau ucacagaggc augcaaugcu 4080
cuuagagguc uggauuggcu aucgcaaaag auagauaaau ucaucaacug gcuuaaaacc 4140
aaaauauuac cagaagcuag ggagaaauau gaauuugugc aaaggcucaa acaguuaccg 4200
gugauagaaa accaaguuag uacaaucgag cauagcugcc caacaacaga acaacaacag 4260
gccuuauuca acaacgucca auacuauuca cacuacugua gaaaguacgc accacuuuac 4320
gcaguggaag caaagagggu aguagcucuu gaaaagaaaa uaaacaacua cauccaguuc 4380
aaguccaaau cucgcauuga accgguuugu uuaauaauac auggcucucc aggaacuggc 4440
aagucagugg cuucaaauuu aauugccagg gcuaucacag agaaauuggg gggggacauu 4500
uauuccuugc cuccagaccc uaaauauuuu gauggauaca aacagcaaac aguggguccuc 4560
auggaugauu uaaugcaaaa uccagauggg aaugacauau cuauguucug ccaaaugguc 4620
uccacuguag auuucauacc cccaauggcu aguuuggagg aaaaaggaac ucuauacacc 4680

agucccauuuu uaauagcuac uaccaaugcu ggcucaauac augcaccaac uguaucagac 4740
ucaaaggcuu ugucacgcag auuuaaauuu gacguggaca uugaagucac agauucauac 4800
aaggacucaa auaaauugga uaugucaagg gcagucgaga ugugcaaacc agauggcugu 4860
gcccccacca auuacaaaag augcugccca uugaucugug gaaaggcuau ccaauucaga 4920
gaucgcagaa cuaaugcaag auccacuauu gauaugcuag uaacugauau uauaaaggaa 4980
uauagaacca gaaacaguac acaggauaag cuggaagcuc uguuucaggg gccuccacag 5040
uuuaaagaga ucaaaauuuc agucaccccca gauacaccag cuccugaugc uauaaaugac 5100
cuucuuaggu caguggauuc ucaagaaguu agggauuauu gccaaaagaa aggauggauu 5160
guaguacacc caucaaauga gcuaauagua gaaaaacaca uuaguagagc uuuuauuacu 5220
cuacaagcca uugccaccuu uguaucaaua gcugguguag uuuauguuau auacaaacuu 5280
uuugcuggca uucaggugcc auacacagga aucccccaauc cuaaaccuaa aguacccucu 5340
cucagaacag cuaaagugca aggaccaggg uucgauuuug cacaagccau aaugaagaaa 5400
aauaccguca uugcaaggac ugaaaagggu gaguucacca ugcugggugu auaugauagg 5460
guagcgguca ucccccacaca cgcaucuguu ggagaaacca uuuacauuaa ugauguagag 5520
acuaaaguuu uagaugcgug ugcacuuaga gacuugacug auacaaacuu agagauaacc 5580
auagucaaau uagaccguaa ucaaaaauuu agagauauca gacauuuucu gcccagauau 5640
gaggaugauu acaaugacgc ugugcuuagc guacauacau caaaauuccc aaauauguau 5700
aucccaguug gacaagucac caauuauggc uucuugaacc uaggugguac accgacgcac 5760
cgcauuuuaa uguauaacuu cccaacaaga gcuggccagu gugguggugu ggugacaacu 5820
acagguaagg ugauaggaau acauguaggu ggaaauggag cucaaggauu ugcagcaaug 5880
cuacuacacu cuuacuuuuc cgauacacaa ggugagauag uuaguaguga aaagaguggg 5940
gugugcauua acgcaccggc aaagacuaaa cuccaaccua guguuuucca ucaaguuuuu 6000
gaagguucaa aggaaccagc aguucucaau ccaaaagauc cuaggcuuaa aacagauuuc 6060
gaggaggcca uuuucucaaa guacacaggu aacaaaauua uguuaaugga ugaguacaug 6120
gaagaggcag uggaucauua ugugggugu uuagaaccau uagacaucag uguggauccc 6180
auaccccugg aaagugccau guauggaaug gauggccuug aggcauuaga cuuaacuacc 6240
agugcaggau ucccuuacuu acuacaaggg aagaagaaaa gggauauauu uaauagacau 6300
acuagagaca ccagugaaau gacaaaaaug uuagagaaau auggaguuga ccuaccuuuu 6360
guaaccuuug uaaaagauga gcuuagauca agagaaaaag uugaaaaagg gaaaucacgc 6420
cugauugagg ccaguuccuu gaaugacuca guugcuauga gaguugccuu uggaaaccuu 6480
uacgccacau uucacaacaa uccagguaca gcaacuggua gugcaguugg uugugaucca 6540
gauauauuuu ggucaaaaau cccuauuuug uuagauggag aaaucuuugc uuuugacuac 6600

acugguuaug augcuaguuu gucaccagug ugguuugccu gcuuaaagaa aguucuaauu 6660

aaguuagguu acacacauca aacgucuuuu auagauuauu ugugucauuc aguacauuua 6720

uauaaggaca aaaaauacau aguuaauggu ggaaugcccu cugguucuuc aggcaccagc 6780

auauucaaca cuaugaucaa caauauaauc auaagaacuu uauuaauuag gguuuacaaa 6840

ggcauagacc uggaccaguu caaaaugauu gccuaugggg augauguuau ugcuagcuac 6900

ccacauaaga uugauccagg uuugcuggca gaagcaggua aacaguaugg auuaguaaug 6960

acgccagcag acaaaggaac caguuuuauu gacacaaauu gggaaaaugu aacuuucuua 7020

aaaagauauu ucagagcaga ugaucaauac cccuuucuca uacauccagu gaugccaaug 7080

aaagagauac augaaucuau uagauggacu aaagauccca gaaacacaca ggaucauguu 7140

aggucuuugu gcuaccucgc auggcauaau ggagaggagg cuuauaauga auuuugcaga 7200

aaaaucagaa gugugccugu gggaagagca uugacacuac cugcauacuc uagucuuaga 7260

cggaaauggu uagauucguu cuagacaacu cuaauugaaa cccaaguuau aguuacuuuc 7320

auuuagaggu aaauuuuggu cacuuggggg ccaaaaaaaa aaaaaaaaaa aaaaaaaguc 7380

gac 7383

<210> 13
<211> 7306
<212> RNA
<213> Artificial Sequence

<220>
<223> Genomic sequence of EV-D68-HRV2

<400> 13
uaauacgacu cacuauaggu uaaaacagcc uuggggaugu ucccacucca agggcccacg 60

uggcggcuag uacucuggua cuucgguacc uuuguacgcc uguuuuaucu cccuucccaa 120

uguaacuuag aagaacuuag aaguuuuuca caaagaccaa uagccgguaa ucagccagau 180

uacugaaggu caagcacuuc uguuuccccg gucaauguug auaugcucca acagggcaaa 240

aacaacugcg aucguuaacc gcaaagcgcc uacgcaaagc uuaguagcau cuuugaaauc 300

guuuggcugg ucgauccgcc auuuccccug guagaccugg cagaugaggc uagaaauacc 360

ccacuggcga caguguucua gccugcgugg cugccugcac acccuauggg ugugaagcca 420

aacaauggac aaggugugaa gagccccgug ugcucgcuuu gaguccuccg gccccugaau 480

guggcuaacc uuaacccugc agcuagagca cguaacccaa uguguaucua gucguaauga 540

gcaauugcgg gaugggacca acuacuuugg guguccgugu uucacuuuuu ccuuuauauu 600

ugcuuauggu gacaauauau acaauauaua uauuggcacc augggagcuc agguuacuag 660

acaacaaacu ggcacucaug aaaaugccaa cauugccaca aauggaucuc auaucacaua 720

caaucagaua aacuuuuaca aggauagcua ugcggcuuca gccagcaagc aggauuuuuc 780
acaggaccca ucaaaauuca cugaaccagu aguggaaggu uuaaaagcag gggcgccagu 840
uuugaaaucu ccuagugcug aggcaugugg cuacagugau agaguauuac agcucaaauu 900
aggaaauuca gcuauuguca cccaggaagc agcgaacuac ugcugcgcuu auggugaaug 960
gcccaauuac uuaccagacc augaagcagu agccauugau aaaccuacac aaccagaaac 1020
ugcuacagau agauucuaca cuuugaaauc agucaaaugg gaaacuggaa gcacaggaug 1080
guggugggaaa cuacccgaug cacugaauaa uauaggcaug uuuggacaga augugcagca 1140
ucacuaccua uauagaucug guuucuugau ucaugugcag uguaaugcca caaaauucca 1200
ucaaggugcc uuauuagugg uagcaauucc agaacaucag aggggagcgc acaacaccaa 1260
cacuagccca ggguuugaug auauaaugaa aggugaagaa ggagggaccu ucaaucaucc 1320
auauguccuu gaugauggaa caucauuggc uugugcgacg auauuuccac aucaguggau 1380
aaaucugaga accaacaauu cagcaacaau uguucuuccc uggaugaaug cugcuccaau 1440
ggauuuccca cuuagacaua aucaguggac gcuagcaaua auaccagugg ugccauuagg 1500
uacgcguaca acaucaagua uggucccaau aacaguuuca aucgcuccaa uguguuguga 1560
guuuaaugga cuuagacacg ccauuacuca aggugcccca acauaccuuu uaccaggcuc 1620
gggacaauuc cuaacaacug augaucauag cucugcacca gcucuccccgu guuucaaccc 1680
aacuccagaa augcauaucc cagggcaggu ccguaacaug cuagaagugg uccaagugga 1740
aucaaugaug gagauuaaua acacagaaag ugcaguuggc auggagcguc uuaagguuga 1800
uauaucagca uugacagaug ucgaucaauu guuauucaac auuccacugg acauacaguu 1860
ggaugggcca cuuagaaaca cuuugguagg aaacauaucu agauauuaca cucauugguc 1920
uggaucccua gaaaugacgu uuauguuuug uggcagcuuc auggcaacgg gaaaauuaau 1980
ccugugcuau acuccuccag guggaucaug cccgacaacc agagagaccg ccauguuagg 2040
uacacauauu guuugggauu uuggauuaca aucuagugua acccugauaa uaccuuggau 2100
uaguggaucc cacuacagga uguuuaauaa ugaugcuaag ucaacuaaug ccaacguugg 2160
cuaugucacu uguuuuaugc agaccaaucu gauagucccc agugaauccu cugacacgug 2220
uuccuugaua ggguucauag cagcaaaaga ugauuucucc cucagauuaa ugagagacag 2280
cccugacauu ggacaacuag accauuuaca ugcagcagag gcagccuacc agaucgagag 2340
caucaucaaa acagcgaccg acacugugaa aagugagauu aaugcugaac uuggugugu 2400
cccuagcuua aaugcaguug aaacaggugc aacuucuaac acugaaccag aagaagccau 2460
acaaacucgc acagugauaa aucagcacgg uguauccgag acucuagugg agaauuuucu 2520
caguagagca gcuuugguau caaagagaag uuuugaauac aaagaucaua cuucgucugc 2580
agcacaagca gacaagaacu uuuucaaaug gacaauuaac accagauccu uuguacaguu 2640

aagaagaaaa uuagaauuau ucacauaccu uagauuugau gcugagauca cuauacucac 2700

aacuguagca gugaauggua gugguaauaa uacauacgug ggucuuccug acuugacacu 2760

ccaagcaaug uuuguaccca cuggugcucu uaccccagaa aaacaggacu cauuccacug 2820

gcagucaggc aguaaugcua guguauucuu uaaaaucucc gacccccag ccagaauaac 2880

cauaccuuuu augugcauua acucagcaua cucaguuuuu uaugauggcu uugccggauu 2940

ugagaaaaac ggucuguaug gaauaaaucc agcugacacu auugguaacu uauguguuag 3000

aauagugaau gaacaccaac caguugguuu cacagugacc guuaggguuu acaugaagcc 3060

uaaacacaua aaagcauggg caccacgacc accacgaacu uugccauaua ugaguauugc 3120

aaaugcaaau uacaaaggua aagaaagagc accaaaugcg cucaaugcua uaauuggcaa 3180

uagagacagu gucaaaacca ugccucauaa uauagugaac acugguccag gcuucggagg 3240

aguuuuugua gggucuuuca aaauaaucaa cuaucacuug gccacuacag aagagagaca 3300

gucagcuauc uauguggauu ggcaaucaga cgucuugguu acccccauug cugcucaugg 3360

aaggcaccaa auagcaagau gcaagugcaa cacagggguu uacuauugua ggcacaaaaa 3420

cagaaguuac ccgauuugcu uugaaggccc agggauucaa uggauugaac aaaaugaaua 3480

uuacccagca agguaccaga ccaauguacu auuggcaguu gguccugcgg aagcaggaga 3540

uugcggugau uuacuaguuu guccacaugg gguaaucggu cuucuuacag caggaggggg 3600

uggaauugua gcuuucacug auaucaggaa uuugcuaugg uuagauacug augcuaugga 3660

acaaggcauu acugauuaua uucaaaaucu ugguaaugcc uuuggagcag gauuuacaga 3720

aacaaucucu aauaaagcca aggaagugca agauaugcua auuggagaga guucacuauu 3780

agaaaaauug uuaaaagcuc uaaucaaaau cauaucagca uuaguaauug uaaucagaaa 3840

cucagaagau uuagucacag ucacagccac acuagcauug uugggaugcc augauucacc 3900

auggagcuac uugaaacaga agguauguuc auacuuaggu auuccuuaug uaccuagaca 3960

gggugaaucg uggcuuaaga aauucacaga ggcaugcaau gcucuuagag gucuggauug 4020

gcuaucgcaa aagauagaua aauucaucaa cuggcuuaaa accaaaauau uaccagaagc 4080

uagggagaaa uaugaauuug ugcaaaggcu caaacaguua ccggugauag aaaaccaagu 4140

uaguacaauc gagcauagcu gcccaacaac agaacaacaa caggccuuau ucaacaacgu 4200

ccaauacuau ucacacuacu guagaaagua cgcaccacuu uacgcagugg aagcaaagag 4260

gguaguagcu cuugaaaaga aaauaaacaa cuacauccag uucaagucca aaucucgcau 4320

ugaaccgguu uguuuaauaa uacauggcuc uccaggaacu ggcaagucag uggcuucaaa 4380

uuuaauugcc agggcuauca cagagaaauu gggggggac auuuauuccu ugccuccaga 4440

cccuaaauau uuugauggau acaaacagca aacaguggu cucauggaug auuuaaugca 4500

aaauccagau gggaaugaca uaucuauguu cugccaaaug gucuccacug uagauuucau 4560
acccccaaug gcuaguuugg aggaaaaagg aacucuauac accaguccau uuuuaauagc 4620
uacuaccaau gcuggcucaa uacaugcacc aacuguauca gacucaaagg cuuugucacg 4680
cagauuuaaa uuugacgugg acauugaagu cacagauuca uacaaggacu caaauaaauu 4740
ggauauguca agggcagucg agaugugcaa accagauggc ugugccccca ccaauuacaa 4800
aagaugcugc ccauugaucu guggaaaggc uauccaauuc agagaucgca gaacuaaugc 4860
aagauccacu auugauaugc uaguaacuga uauuauaaag gaauauagaa ccagaaacag 4920
uacacaggau aagcuggaag cucuguuuca ggggccucca caguuuaaag agaucaaaau 4980
uucagucacc ccagauacac cagcuccuga ugcuauaaau gaccuucuua ggucagugga 5040
uucucaagaa guuagggauu auugccaaaa gaaaggaugg auuguaguac acccaucaaa 5100
ugagcuaaua guagaaaaac acauuaguag agcuuuuauu acucuacaag ccauugccac 5160
cuuuguauca auagcuggug uaguuuaugu uauauacaaa cuuuuugcug gcauucaggg 5220
uccauacaca ggaaucccca auccuaaacc uaaaguaccc ucucucagaa cagcuaaagu 5280
gcaaggacca ggguucgauu uugcacaagc cauaaugaag aaaaauaccg ucauugcaag 5340
gacugaaaag ggugaguuca ccaugcuggg uguauaugau aggguagcgg ucauccccac 5400
acacgcaucu guuggagaaa ccauuuacau uaaugaugua gagacuaaag uuuuagaugc 5460
gugugcacuu agagacuuga cugauacaaa cuuagagaua accauaguca aauuagaccg 5520
uaaucaaaaa uuuagagaua ucagacauuu ucugcccaga uaugaggaug auuacaauga 5580
cgcugugcuu agcguacaua caucaaaauu cccaaauaug uauaucccag uuggacaagu 5640
caccaauuau ggcuucuuga accuaggugg uacaccgacg caccgcauuu uaauguauaa 5700
cuucccaaca agagcuggcc aguguggugg ugguggugaca acuacaggua aggugauagg 5760
aauacaugua gguggaaaug gagcucaagg auuugcagca augcuacuac acucuuacuu 5820
uuccgauaca caaggugaga uaguuaguag ugaaaagagu gggugugca uuaacgcacc 5880
ggcaaagacu aaacuccaac cuaguguuuu ccaucaaguu uuugaagguu caaaggaacc 5940
agcaguucuc aauccaaaag auccuaggcu uaaaacagau uucgaggagg ccauuuucuc 6000
aaaguacaca gguaacaaaa uuauguuaau ggaugaguac auggaagagg caguggauca 6060
uuaugugggg uguuuagaac cauuagacau cagugugggau cccauacccc uggaaagugc 6120
cauguaugga auggauggcc uugaggcauu agacuuaacu accagugcag gauucccuua 6180
cuuacuacaa gggaagaaga aaagggauau auuuaauaga cauacuagag acaccaguga 6240
aaugacaaaa auguuagaga aauauggagu ugaccuaccu uuuguaaccu uuguaaaaga 6300
ugagcuuaga ucaagagaaa aaguugaaaa agggaaauca cgccugauug aggccaguuc 6360
cuugaaugac ucaguugcua ugagaguugc cuuuggaaac cuuuacgcca cauuucacaa 6420

caauccaggu acagcaacug guagugcagu ugguugugau ccagauauau uuuggucaaa 6480
aaucccuauu uuguuagaug gagaaaucuu ugcuuuugac uacacugguu augaugcuag 6540
uuugucacca guguggguuug ccugcuuaaa gaaaguucua auuaaguuag guuacacaca 6600
ucaaacgucu uuuauagauu auuuguguca uucaguacau uuauauaagg acaaaaaaua 6660
cauaguuaau gguggaaugc ccucugguuc uucaggcacc agcauauuca acacuaugau 6720
caacaauaua aucauaagaa cuuuauuaau uaggguuuac aaaggcauag accuggacca 6780
guucaaaaug auugccuaug gggaugaugu uauugcuagc uacccacaua agauugaucc 6840
agguuugcug gcagaagcag guaaacagua uggauuagua augacgccag cagacaaagg 6900
aaccaguuuu auugacacaa auugggaaaa uguaacuuuc uuaaaaagau auuucagagc 6960
agaugaucaa uaccccuuuc ucauacaucc agugaugcca augaaagaga uacaugaauc 7020
uauuagaugg acuaaagauc ccagaaacac acaggaucau guuaggucuu ugugcuaccu 7080
cgcauggcau aauggagagg aggcuuauaa ugaauuuugc agaaaaauca gaagugugcc 7140
ugugggaaga gcauugacac uaccugcaua cucuagucuu agacggaaau gguuagauuc 7200
guucuagaca acucuaauug aaacccaagu uauaguuacu uucauuuaga gguaaauuuu 7260
ggucacuugg gggccaaaaa aaaaaaaaaa aaaaaaaaaa gucgac 7306

<210> 14
<211> 7485
<212> RNA
<213> Artificial Sequence

<220>
<223> Genomic sequence of EV-D68-miR133&206T

<400> 14
uaauacgacu cacuauaggu uaaaacagcc uuggggguugu ucccacucca agggcccacg 60
uggcggcuag uacucuggua cuucgguacc uuuguacgcc uguuuuaucu cccuucccaa 120
uguaacuuag aaguucuuaa aucaaugcuc aauagguggg gcgcaaacca gcgcucucau 180
gagcaagcac uccugucucc ccggugaggu uguauaaacu guucccacgg uugaaaacaa 240
ccuauccguu auccgcuaua guacuucgag aaaccuagua ccaccuuugg auuguugacg 300
cguugcgcuc agcacacuaa cccgugugua gcuugggucg augagucugg acauaccuca 360
cuggcgacag ugguccaggc ugcguuggcg gccuacucau ggugaaagcc augagacgcu 420
agacaugaac aaggugugaa gagucuauug agcuacuaua gaguccuccg gccccugaau 480
gcggcuaauc cuaaccaugg agcaagugcu cacaggccag ugaguugcuu gucguaaugc 540
gcaaguccgu ggcggaaccg acuacuuugg guguccgugu uucacuuuuu acuuuuauga 600
cugcuuaugg ugacaauuug auauuguuac cauuuagcuu gucaaaucaa uugcaaaaga 660

uccuaaaucu uauuuaucaa cuugcaucuu gauaacuuua auuugaaaau uuuaacaaug 720
ggagcucagg uuacuagaca acaaacuggc acucaugaaa augccaacau ugccacaaau 780
ggaucucaua ucacauacaa ucagauaaac uuuuacaagg auagcuaugc ggcuucagcc 840
agcaagcagg auuuuucaca ggacccauca aaauucacug aaccaguagu ggaagguuua 900
aaagcagggg cgccaguuuu gaaaucuccu agugcugagg cauguggcua cagugauaga 960
guauuacagc ucaaauuagg aaauucagcu auugucaccc aggaagcagc gaacuacugc 1020
ugcgcuuaug gugaauggcc caauuacuua ccagaccaug aagcaguagc cauugauaaa 1080
ccuacacaac cagaaacugc uacagauaga uucuacacuu ugaaaucagu caaaugggaa 1140
acuggaagca caggauggug guggaaacua cccgaugcac ugaauaauau aggcauguuu 1200
ggacagaaug ugcagcauca cuaccuauau agaucugguu ucuugauuca ugugcagugu 1260
aaugccacaa aauuccauca aggugccuua uuagugguag caauuccaga acaucagagg 1320
ggagcgcaca acaccaacac uagcccaggg uuugaugaua uaaugaaagg ugaagaagga 1380
gggaccuuca aucauccaua uguccuugau gauggaacau cauuggcuug ugcgacgaua 1440
uuuccacauc aguggauaaa ucugagaacc aacaauucag caacaauugu ucuucccugg 1500
augaaugcug cuccaaugga uuucccacuu agacauaauc aguggacgcu agcaauaaua 1560
ccaguggugc cauuagguac gcguacaaca ucaaguaugg ucccaauaac aguuucaauc 1620
gcuccaaugu guugugaguu uaauggacuu agacacgcca uuacucaagg ugucccaaca 1680
uaccuuuuac caggcucggg acaauuccua acaacugaug aucauagcuc ugcaccagcu 1740
cucccguguu ucaacccaac uccagaaaug cauaucccag ggcagguccg uaacaugcua 1800
gaagugguccc aaguggaauc aaugauggag auuaauaaca cagaaagugc aguuggcaug 1860
gagcgucuua agguugauau aucagcauug acagaugucg aucaauuguu auucaacauu 1920
ccacuggaca uacaguugga ugggccacuu agaaacacuu ugguaggaaa cauaucuaga 1980
uauuacacuc auuggucugg aucccuagaa augacguuua uguuuugugg cagcuucaug 2040
gcaacgggaa aauuaauccu gugcuauacu ccuccaggug gaucaugccc gacaaccaga 2100
gagaccgcca uguuagguac acauauuguu ugggauuuug gauuacaauc uaguguaacc 2160
cugauaauac cuuggauuag uggaucccac uacaggaugu uuaauaauga ugcuaaguca 2220
acuaaugcca acguuggcua ugucacuugu uuuaugcaga ccaaucugau aguccccagu 2280
gaauccucug acacguguuc cuugauaggg uucauagcag caaaagauga uuucucccuc 2340
agauuaauga gagacagccc ugacauugga caacuagacc auuuacaugc agcagaggca 2400
gccuaccaga ucgagagcau caucaaaaca gcgaccgaca cugugaaaag ugagauuaau 2460
gcugaacuug guguggucccc uagcuuaaau gcaguugaaa caggugcaac uucuaacacu 2520
gaaccagaag aagccauaca aacucgcaca gugauaaauc agcacggugu auccgagacu 2580

cuaguggaga auuuucucag uagagcagcu uugguaucaa agagaaguuu ugaauacaaa 2640
gaucauacuu cgucugcagc acaagcagac aagaacuuuu ucaaauggac aauuaacacc 2700
agauccuuug uacaguuaag aagaaaauua gaauuauuca cauaccuuag auuugaugcu 2760
gagaucacua uacucacaac uguagcagug aaugguagug guaauaauac auacgugggu 2820
cuuccugacu ugacacucca agcaauguuu guacccacug gugcucuuac cccagaaaaa 2880
caggacucau uccacuggca gucaggcagu aaugcuagug uauucuuuaa aaucuccgac 2940
cccccagcca gaauaaccau accuuuuaug ugcauuaacu cagcauacuc aguuuuuuau 3000
gauggcuuug ccggauuuga gaaaaacggu cuguauggaa uaaauccagc ugacacuauu 3060
gguaacuuau guguuagaau agugaaugaa caccaaccag uugguuucac agugaccguu 3120
aggguuuaca ugaagccuaa acacauaaaa gcaugggcac cacgaccacc acgaacuuug 3180
ccauauauga guauugcaaa ugcaaauuac aaagguaaag aaagagcacc aaaugcgcuc 3240
aaugcuauaa uuggcaauag agacaguguc aaaaccaugc cucauaauau agugaacacu 3300
gguccaggcu ucggaggagu uuuuguaggg ucuuucaaaa uaaucaacua ucacuuggcc 3360
acuacagaag agagacaguc agcuaucuau guggauuggc aaucagacgu cuugguuacc 3420
cccauugcug cucauggaag gcaccaaaua gcaagaugca agugcaacac aggggguuuac 3480
uauuguaggc acaaaaacag aaguuacccg auuugcuuug aaggcccagg gauucaaugg 3540
auugaacaaa augaauauua cccagcaagg uaccagacca auguacuauu ggcaguuggu 3600
ccugcggaag caggagauug cggugguuua cuaguuuguc cacauggggu aaucggucuu 3660
cuuacagcag gaggggguggg aauuguagcu uucacugaua ucaggaauuu gcuaugguua 3720
gauacugaug cuauggaaca aggcauuacu gauuauauuc aaaaucuugg uaaugccuuu 3780
ggagcaggau uuacagaaac aaucucuaau aaagccaagg aagugcaaga uaugcuaauu 3840
ggagagaguu cacuauuaga aaaauuguua aaagcucuaa ucaaaaucau aucagcauua 3900
guaauuguaa ucagaaacuc agaagauuua gucacaguca cagccacacu agcauuguug 3960
ggaugccaug auucaccaug gagcuacuug aaacagaagg uauguucaua cuuagguauu 4020
ccuuauguac cuagacaggg ugaaucgugg cuuaagaaau ucacagaggc augcaaugcu 4080
cuuagagguc uggauuggcu aucgcaaaag auagauaaau ucaucaacug gcuuaaaacc 4140
aaaauauuac cagaagcuag ggagaaauau gaauuugugc aaaggcucaa acaguuaccg 4200
gugauagaaa accaaguuag uacaaucgag cauagcugcc caacaacaga acaacaacag 4260
gccuuauuca acaacgucca auacuauuca cacuacugua gaaaguacgc accacuuuac 4320
gcaguggaag caaagagggu aguagcucuu gaaaagaaaa uaaacaacua cauccaguuc 4380
aaguccaaau cucgcauuga accgguuugu uuaauaauac auggcucucc aggaacuggc 4440

aagucagugg cuucaaauuu aauugccagg gcuaucacag agaaauuggg gggggacauu 4500
uauuccuugc cuccagaccc uaaauauuuu gauggauaca aacagcaaac agugguccuc 4560
auggaugauu uaaugcaaaa uccagauggg aaugacauau cuauguucug ccaaaugguc 4620
uccacuguag auuucauacc cccaauggcu aguuuggagg aaaaaggaac ucuauacacc 4680
aguccauuuu uaauagcuac uaccaaugcu ggcucaauac augcaccaac uguaucagac 4740
ucaaaggcuu ugucacgcag auuuaaauuu gacguggaca uugaagucac agauucauac 4800
aaggacucaa auaaauugga uaugucaagg gcagucgaga ugugcaaacc agauggcugu 4860
gcccccacca auuacaaaag augcugccca uugaucugug gaaaggcuau ccaauucaga 4920
gaucgcagaa cuaaugcaag auccacuauu gauaugcuag uaacugauau uauaaaggaa 4980
uauagaacca gaaacaguac acaggauaag cuggaagcuc uguuucaggg gccuccacag 5040
uuuaaagaga ucaaaauuuc agucacccca gauacaccag cuccugaugc uauaaaugac 5100
cuucuuaggu caguggauuc ucaagaaguu agggauuauu gccaaaagaa aggauggauu 5160
guaguacacc caucaaauga gcuaauagua gaaaaacaca uuaguagagc uuuuauuacu 5220
cuacaagcca uugccaccuu uguaucaaua gcuggugcag uuuauguuau auacaaacuu 5280
uuugcuggca uucagggucc auacacagga auccccaauc cuaaaccuaa aguacccucu 5340
cucagaacag cuaaagugca aggaccaggg uucgauuuug cacaagccau aaugaagaaa 5400
aauaccguca uugcaaggac ugaaaagggu gaguucacca ugcugggugu auaugauagg 5460
guagcgguca uccccacaca cgcaucuguu ggagaaacca uuuacauuaa ugauguagag 5520
acuaaaguuu uagaugcgug ugcacuuaga gacuugacug auacaaacuu agagauaacc 5580
auagucaaau uagaccguaa ucaaaaauuu agagauauca gacauuuucu gcccagauau 5640
gaggaugauu acaaugacgc ugugcuuagc guacauacau caaaauuccc aaauauguau 5700
aucccaguug gacaagucac caauuauggc uucuugaacc uaggugguac accgacgcac 5760
cgcauuuuaa uguauaacuu cccaacaaga gcuggccagu gugguggugu ggugacaacu 5820
acagguaagg ugauaggaau acauguaggu ggaaauggag cucaaggauu ugcagcaaug 5880
cuacuacacu cuuacuuuuc cgauacacaa ggugagauag uuaguaguga aaagaguggg 5940
gugugcauua acgcaccggc aaagacuaaa cuccaaccua guguuuucca ucaaguuuuu 6000
gaagguucaa aggaaccagc aguucucaau ccaaaagauc cuaggcuuaa aacagauuuc 6060
gaggaggcca uuuucucaaa guacacaggu aacaaaauua uguuaaugga ugaguacaug 6120
gaagaggcag uggaucauua ugugggggugu uuagaaccau uagacaucag uguggauccc 6180
auaccccugg aaagugccau guauggaaug gauggccuug aggcauuaga cuuaacuacc 6240
agugcaggau ucccuuacuu acuacaaggg aagaagaaaa gggauauauu uaauagacau 6300
acuagagaca ccagugaaau gacaaaaaug uuagagaaau auggaguuga ccuaccuuuu 6360

guaaccuuug uaaaagauga gcuuagauca agagaaaaag uugaaaaagg gaaaucacgc 6420

cugauugagg ccaguuccuu gaaugacuca guugcuauga gaguugccuu uggaaaccuu 6480

uacgccacau uucacaacaa uccagguaca gcaacuggua gugcaguugg uugugaucca 6540

gauauauuuu ggucaaaaau cccuauuuug uuagauggag aaaucuuugc uuuugacuac 6600

acugguuaug augcuaguuu gucaccagug ugguuugccu gcuuaaagaa aguucuaauu 6660

aaguuagguu acacacauca aacgucuuuu auagauuauu ugugucauuc aguacauuua 6720

uauaaggaca aaaaauacau aguuaauggu ggaaugcccu cugguucuuc aggcaccagc 6780

auauucaaca cuaugaucaa caauauaauc auaagaacuu uauuaauuag gguuuacaaa 6840

ggcauagacc uggaccaguu caaaaugauu gccuaugggg augauguuau ugcuagcuac 6900

ccacauaaga uugauccagg uuugcuggca gaagcaggua aacaguaugg auuaguaaug 6960

acgccagcag acaaaggaac caguuuuauu gacacaaauu gggaaaaugu aacuuucuua 7020

aaaagauauu ucagagcaga ugaucaauac cccuuucuca uacauccagu gaugccaaug 7080

aaagagauac augaaucuau uagauggacu aaagauccca gaaacacaca ggaucauguu 7140

aggucuuugu gcuaccucgc auggcauaau ggagaggagg cuuauaauga auuuugcaga 7200

aaaaucagaa gugugccugu gggaagagca uugacacuac cugcauacuc uagucuuaga 7260

cggaaauggu uagauucguu cuagacaacu cuaacagcug guugaagggg accaacgaua 7320

cagcugguug aaggggacca aaccggucca cacacuuccu uacauuccau cacccacaca 7380

cuuccuuaca uuccaauuga aacccaaguu auaguuacuu ucauuuagag guaaauuuug 7440

gucacuuggg ggccaaaaaa aaaaaaaaaa aaaaaaaaag ucgac 7485

<210> 15
<211> 7860
<212> RNA
<213> Artificial Sequence

<220>
<223> Genomic sequence of EV-D68-GM-CSF

<400> 15
uaauacgacu cacuauaggu uaaaacagcc uugggguugu ucccacucca agggcccacg 60

uggcggcuag uacucuggua cuucgguacc uuuguacgcc uguuuuaucu cccuucccaa 120

uguaacuuag aaguucuuaa aucaaugcuc aauagguggg gcgcaaacca gcgcucucau 180

gagcaagcac uccugucucc ccggugaggu uguauaaacu guucccacgg uugaaaacaa 240

ccuauccguu auccgcuaua guacuucgag aaaccuagua ccaccuuugg auuguugacg 300

cguugcgcuc agcacacuaa cccgugugua gcuugggucg augagucugg acauaccuca 360

cuggcgacag ugguccaggc ugcguuggcg gccuacucau ggugaaagcc augagacgcu 420

agacaugaac aaggugugaa gagucuauug agcuacuaua gaguccuccg gccccugaau 480

gcggcuaauc cuaaccaugg agcaagugcu cacaggccag ugaguugcuu gucguaaugc 540

gcaaguccgu ggcggaaccg acuacuuugg guguccgugu uucacuuuuu acuuuuauga 600

cugcuuaugg ugacaauuug auauuguuac cauuuagcuu gucaaaucaa uugcaaaaga 660

uccuaaaucu uauuuaucaa cuugcaucuu gauaacuuua auuugaaaau uuuaacaaug 720

ggagcucagg uuacuagaca acaaacuggc acucaugaaa augccaacau ugccacaaau 780

ggaucucaua ucacauacaa ucagauaaac uuuuacaagg auagcuaugc ggcuucagcc 840

agcaagcagg auuuuucaca ggacccauca aaauucacug aaccaguagu ggaagguuua 900

aaagcagggg cgccaguuuu gaaaucuccu agugcugagg cauguggcua cagugauaga 960

guauuacagc ucaaauuagg aaauucagcu auugucaccc aggaagcagc gaacuacugc 1020

ugcgcuuaug gugaauggcc caauuacuua ccagaccaug aagcaguagc cauugauaaa 1080

ccuacacaac cagaaacugc uacagauaga uucuacacuu ugaaaucagu caaaugggaa 1140

acuggaagca caggauggug guggaaacua cccgaugcac ugaauaauau aggcauguuu 1200

ggacagaaug ugcagcauca cuaccuauau agaucugguu ucuugauuca ugugcagugu 1260

aaugccacaa aauuccauca aggugccuua uuagugguag caauuccaga acaucagagg 1320

ggagcgcaca acaccaacac uagcccaggg uuugaugaua uaaugaaagg ugaagaagga 1380

gggaccuuca aucauccaua uguccuugau gauggaacau cauuggcuug ugcgacgaua 1440

uuuccacauc aguggauaaa ucugagaacc aacaauucag caacaauugu ucuucccugg 1500

augaaugcug cuccaaugga uuucccacuu agacauaauc aguggacgcu agcaauaaua 1560

ccagugguge cauuagguac gcguacaaca ucaaguaugg ucccaauaac aguuucaauc 1620

gcuccaaugu guugugaguu uaauggacuu agacacgcca uuacucaagg ugucccaaca 1680

uaccuuuuac caggcucggg acaauuccua acaacugaug aucauagcuc ugcaccagcu 1740

cucccguguu ucaacccaac uccagaaaug cauaucccag ggcagguccg uaacaugcua 1800

gaaguggucc aaguggaauc aaugauggag auuaauaaca cagaaagugc aguuggcaug 1860

gagcgucuua agguugauau aucagcauug acagaugucg aucaauuguu auucaacauu 1920

ccacuggaca uacaguugga ugggccacuu agaaacacuu ugguaggaaa cauaucuaga 1980

uauuacacuc auuggucugg aucccuagaa augacguuua uguuuugugg cagcuucaug 2040

gcaacgggaa aauuaauccu gugcuauacu ccuccaggug gaucaugccc gacaaccaga 2100

gagaccgcca uguuagguac acauauuguu ugggauuuug gauuacaauc uaguguaacc 2160

cugauaauac cuuggauuag uggaucccac uacaggaugu uuaauaauga ugcuaaguca 2220

acuaaugcca acguuggcua ugucacuugu uuuaugcaga ccaaucugau aguccccagu 2280

gaauccucug acacguguuc cuugauaggg uucauagcag caaaagauga uuucucccuc 2340

agauuaauga gagacagccc ugacauugga caacuagacc auuuacaugc agcagaggca 2400
gccuaccaga ucgagagcau caucaaaaca gcgaccgaca cugugaaaag ugagauuaau 2460
gcugaacuug guguggucccc uagcuuaaau gcaguugaaa caggugcaac uucuaacacu 2520
gaaccagaag aagccauaca aacucgcaca gugauaaauc agcacggugu auccgagacu 2580
cuaguggaga auuuucucag uagagcagcu uugguaucaa agagaaguuu ugaauacaaa 2640
gaucauacuu cgucugcagc acaagcagac aagaacuuuu ucaaauggac aauuaacacc 2700
agauccuuug uacaguuaag aagaaaauua gaauuauuca cauaccuuag auuugaugcu 2760
gagaucacua uacucacaac uguagcagug aaugguagug guaauaauac auacgugggu 2820
cuuccugacu ugacacucca agcaauguuu guacccacug gugcucuuac cccagaaaaa 2880
caggacucau uccacuggca gucaggcagu aaugcuagug uauucuuuaa aaucuccgac 2940
cccccagcca gaauaaccau accuuuuaug ugcauuaacu cagcauacuc aguuuuuuau 3000
gauggcuuug ccggauuuga gaaaaacggu cuguauggaa uaaauccagc ugacacuauu 3060
gguaacuuau guguuagaau agugaaugaa caccaaccag uugguuucac agugaccguu 3120
aggguuuaca ugaagccuaa acacauaaaa gcaugggcac cacgaccacc acgaacuuug 3180
ccauauauga guauugcaaa ugcaaauuac aaagguaaag aaagagcacc aaaugcgcuc 3240
aaugcuauaa uuggcaauag agacaguguc aaaaccaugc cucauaauau agugaacacu 3300
gguccaggcu ucuggcugca gagccugcug cucuugggca cuguggccug cagcaucucu 3360
gcacccgccc gcucgcccag ccccagcacg cagcccuggg agcaugugaa ugccauccag 3420
gaggcccggc gucuccugaa ccugaguaga gacacugcug cugagaugaa ugaaacagua 3480
gaagucaucu cagaaauguu ugaccuccag gagccgaccu gccuacagac ccgccuggag 3540
cuguacaagc agggccugcg gggcagccuc accaagcuca agggccccuu gaccaugaug 3600
gccagccacu acaagcagca cugcccucca accccggaaa cuuccugugc aacccagauu 3660
aucaccuuug aaaguuucaa agagaaccug aaggacuuuc ugcuugucau ccccuuugac 3720
ugcugggagc caguccagga gagugucaaa accaugccuc auaauauagu gaacacuggu 3780
ccaggcuucg gaggaguuuu uguagggucu uucaaaauaa ucaacuauca cuuggccacu 3840
acagaagaga gacagucagc uaucuaugug gauuggcaau cagacgucuu gguuaccccc 3900
auugcugcuc auggaaggca ccaaauagca agaugcaagu gcaacacagg gguuuacuau 3960
uguaggcaca aaaacagaag uuacccgauu ugcuuugaag gcccagggau ucaauggauu 4020
gaacaaaaug aauauuaccc agcaagguac cagaccaaug uacuauuggc aguuggucu 4080
gcggaagcag gagauugcgg ugguuuacua guuuguccac augggguaau cggucuucuu 4140
acagcaggag gggguggaau uguagcuuuc acugauauca ggaauuugcu augguuagau 4200

acugaugcua uggaacaagg cauuacugau uauauucaaa aucuugguaa ugccuuugga 4260
gcaggauuua cagaaacaau cucuaauaaa gccaaggaag ugcaagauau gcuaauugga 4320
gagaguucac uauuagaaaa auuguuaaaa gcucuaauca aaaucauauc agcauuagua 4380
auuguaauca gaaacucaga agauuuaguc acagucacag ccacacuagc auuguuggga 4440
ugccaugauu caccauggag cuacuugaaa cagaagguau guucauacuu agguauuccu 4500
uauguaccua gacaggguga aucguggcuu aagaaauuca cagaggcaug caaugcucuu 4560
agaggucugg auuggcuauc gcaaaagaua gauaaauuca ucaacuggcu uaaaaccaaa 4620
auauuaccag aagcuaggga gaaauaugaa uuugugcaaa ggcucaaaca guuaccggug 4680
auagaaaacc aaguuaguac aaucgagcau agcugcccaa caacagaaca acaacaggcc 4740
uuauucaaca acguccaaua cuauucacac uacuguagaa aguacgcacc acuuuacgca 4800
guggaagcaa agaggguagu agcucuugaa aagaaaauaa acaacuacau ccaguucaag 4860
uccaaaucuc gcauugaacc gguuuguuua auaauacaug gcucuccagg aacuggcaag 4920
ucaguggcuu caaauuuaau ugccagggcu aucacagaga aauugggggg ggacauuuau 4980
uccuugccuc cagacccuaa auauuuugau ggauacaaac agcaaacagu gguccucaug 5040
gaugauuuaa ugcaaaaucc agaugggaau gacauaucua uguucugcca aauggucucc 5100
acuguagauu ucauaccccc aauggcuagu uuggaggaaa aaggaacucu auacaccagu 5160
ccauuuuuaa uagcuacuac caaugcuggc ucaauacaug caccaacugu aucagacuca 5220
aaggcuuugu cacgcagauu uaaauuugac guggacauug aagucacaga uucauacaag 5280
gacucaaaua aauuggauau gucaagggca gucgagaugu gcaaaccaga uggcugugcc 5340
cccaccaauu acaaaagaug cugcccauug aucuguggaa aggcuaucca auucagagau 5400
cgcagaacua augcaagauc cacuauugau augcuaguaa cugauauuau aaaggaauau 5460
agaaccagaa acaguacaca ggauaagcug gaagcucugu uucaggggcc uccacaguuu 5520
aaagagauca aaauuucagu caccccagau acaccagcuc cugaugcuau aaaugaccuu 5580
cuuaggucag uggauucuca agaaguuagg gauuauugcc aaaagaaagg auggauugua 5640
guacacccau caaaugagcu aauaguagaa aaacacauua guagagcuuu uauuacucua 5700
caagccauug ccaccuuugu aucaauagcu gguguaguuu auguuauaua caaacuuuuu 5760
gcuggcauuc aggguccaua cacaggaauc cccaauccua aaccuaaagu acccucucuc 5820
agaacagcua aagugcaagg accaggguuc gauuuugcac aagccauaau gaagaaaaau 5880
accgucauug caaggacuga aaagggugag uucaccaugc uggguguaua ugauagggua 5940
gcggucaucc ccacacacgc aucuguugga gaaaccauuu acauuaauga uguagagacu 6000
aaaguuuuag augcgugugc acuuagagac uugacugaua caaacuuaga gauaaccaua 6060
gucaaauuag accguaauca aaaauuuaga gauaucagac auuuucugcc cagauaugag 6120

gaugauuaca augacgcugu gcuuagcgua cauacaucaa aauucccaaa uauguauauc 6180
ccaguuggac aagucaccaa uuauggcuuc uugaaccuag gugguacacc gacgcaccgc 6240
auuuuaaugu auaacuuccc aacaagagcu ggccagugug gugguguggu gacaacuaca 6300
gguaagguga uaggaauaca uguaggugga aauggagcuc aaggauuugc agcaaugcua 6360
cuacacucuu acuuuuccga uacacaaggu gagauaguua guagugaaaa gaguggggug 6420
ugcauuaacg caccggcaaa gacuaaacuc caaccuagug uuuuccauca aguuuuugaa 6480
gguucaaagg aaccagcagu ucucaaucca aaagauccua ggcuuaaaac agauuucgag 6540
gaggccauuu ucucaaagua cacagguaac aaaauuaugu uaauggauga guacauggaa 6600
gaggcagugg aucauuaugu ggggguguua gaaccauuag acaucagugu ggaucccaua 6660
ccccuggaaa gugccaugua uggaauggau ggccuugagg cauuagacuu aacuaccagu 6720
gcaggauucc cuuacuuacu acaagggaag aagaaaaggg auauauuuaa uagacauacu 6780
agagacacca gugaaaugac aaaaauguua gagaaauaug gaguugaccu accuuuugua 6840
accuuuguaa aagaugagcu uagaucaaga gaaaaaguug aaaaagggaa aucacgccug 6900
auugaggcca guuccuugaa ugacucaguu gcuaugagag uugccuuugg aaaccuuuac 6960
gccacauuuc acaacaaucc agguacagca acugguagug caguugguug ugauccagau 7020
auauuuuggu caaaaauccc uauuuuguua gauggagaaa ucuuugcuuu ugacuacacu 7080
gguuaugaug cuaguuuguc accagugugg uuugccugcu uaaagaaagu ucuaauuaag 7140
uuagguuaca cacaucaaac gucuuuuaua gauuauuugu gucauucagu acauuuauau 7200
aaggacaaaa aauacauagu uaauggugga augcccucug guucuucagg caccagcaua 7260
uucaacacua ugaucaacaa uauaaucaua agaacuuuau uaauuagggu uuacaaaggc 7320
auagaccugg accaguucaa aaugauugcc uaugggga ug auguuauugc uagcuaccca 7380
cauaagauug auccagguuu gcuggcagaa gcagguaaac aguauggauu aguaaugacg 7440
ccagcagaca aaggaaccag uuuuauugac acaaauuggg aaaauguaac uuucuuaaaa 7500
agauauuuca gagcagauga ucaauacccc uuucucauac auccagugau gccaaugaaa 7560
gagauacaug aaucuauuag auggacuaaa gaucccagaa acacacagga ucauguuagg 7620
ucuuugugcu accucgcaug gcauaaugga gaggaggcuu auaaugaauu uugcagaaaa 7680
aucagaagug ugccuguggg aagagcauug acacuaccug cauacucuag ucuuagacgg 7740
aaaugguuag auucguucua gacaacucua auugaaaccc aaguuauagu uacuuucauu 7800
uagagguaaa uuuuggucac uugggggcca aaaaaaaaaa aaaaaaaaaa aaaagucgac 7860

<210> 16
<211> 8226
<212> RNA

<213> Artificial Sequence

<220>
<223> Genomic sequence of EV-D68-Anti-PD1

<400> 16
uaauacgacu cacuauaggu uaaaacagcc uuggggguugu ucccacucca agggcccacg 60

uggcggcuag uacucuggua cuucgguacc uuuguacgcc uguuuuaucu cccuucccaa 120

uguaacuuag aaguucuuaa aucaaugcuc aauagguggg gcgcaaacca gcgcucucau 180

gagcaagcac uccugucucc ccggugaggu uguauaaacu guucccacgg uugaaaacaa 240

ccuauccguu auccgcuaua guacuucgag aaaccuagua ccaccuuugg auuguugacg 300

cguugcgcuc agcacacuaa cccgugugua gcuugggucg augagucugg acauaccuca 360

cuggcgacag ugguccaggc ugcguuggcg gccuacucau ggugaaagcc augagacgcu 420

agacaugaac aaggugugaa gagucuauug agcuacuaua gaguccuccg gccccugaau 480

gcggcuaauc cuaaccaugg agcaagugcu cacaggccag ugaguugcuu gucguaaugc 540

gcaaguccgu ggcggaaccg acuacuuugg guguccgugu uucacuuuuu acuuuuauga 600

cugcuuaugg ugacaauuug auauuguuac cauuuagcuu gucaaaucaa uugcaaaaga 660

uccuaaaucu uauuuaucaa cuugcaucuu gauaacuuua auuugaaaau uuuaacaaug 720

ggagcucagg uuacuagaca acaaacuggc acucaugaaa augccaacau ugccacaaau 780

ggaucucaua ucacauacaa ucagauaaac uuuuacaagg auagcuaugc ggcuucagcc 840

agcaagcagg auuuuucaca ggacccauca aaauucacug aaccaguagu ggaagguuua 900

aaagcagggg cgccaguuuu gaaaucuccu agugcugagg cauguggcua cagugauaga 960

guauuacagc ucaaauuagg aaauucagcu auugucaccc aggaagcagc gaacuacugc 1020

ugcgcuuaug gugaauggcc caauuacuua ccagaccaug aagcaguagc cauugauaaa 1080

ccuacacaac cagaaacugc uacagauaga uucuacacuu ugaaaucagu caaaugggaa 1140

acuggaagca caggauggug guggaaacua cccgaugcac ugaauaauau aggcauguuu 1200

ggacagaaug ugcagcauca cuaccuauau agaucugguu ucuugauuca ugugcagugu 1260

aaugccacaa aauuccauca aggugccuua uuaguggu ag caauuccaga acaucagagg 1320

ggagcgcaca acaccaacac uagcccaggg uuugaugaua uaaugaaagg ugaagaagga 1380

gggaccuuca aucauccaua uguccuugau gauggaacau cauuggcuug ugcgacgaua 1440

uuuccacauc aguggauaaa ucugagaacc aacaauucag caacaauugu ucuucccugg 1500

augaaugcug cuccaaugga uuucccacuu agacauaauc aguggacgcu agcaauaaua 1560

ccagugugc cauuagguac gcguacaaca ucaaguaugg ucccaauaac aguuucaauc 1620

gcuccaaugu guugugaguu uaauggacuu agacacgcca uuacucaagg ugucccaaca 1680

uaccuuuuac caggcucggg acaauuccua acaacugaug aucauagcuc ugcaccagcu 1740

cuccccguguu ucaacccaac uccagaaaug cauaucccag ggcagguccg uaacaugcua 1800
gaaguggucc aaguggaauc aaugauggag auuaauaaca cagaaagugc aguuggcaug 1860
gagcgucuua agguugauau aucagcauug acagaugucg aucaauuguu auucaacauu 1920
ccacuggaca uacaguugga ugggccacuu agaaacacuu ugguaggaaa cauaucuaga 1980
uauuacacuc auuggucugg aucccuagaa augacguuua uguuuugugg cagcuucaug 2040
gcaacgggaa aauuaauccu gugcuauacu ccuccaggug gaucaugccc gacaaccaga 2100
gagaccgcca uguuagguac acauauuguu ugggauuuug gauuacaauc uaguguaacc 2160
cugauaauac cuuggauuag uggaucccac uacaggaugu uuaauaauga ugcuaaguca 2220
acuaaugcca acguuggcua ugucacuugu uuuaugcaga ccaaucugau aguccccagu 2280
gaauccucug acacguguuc cuugauaggg uucauagcag caaaagauga uuucuccccuc 2340
agauuaauga gagacagccc ugacauugga caacuagacc auuuacaugc agcagaggca 2400
gccuaccaga ucgagagcau caucaaaaca gcgaccgaca cugugaaaag ugagauuaau 2460
gcugaacuug guguggucccc uagcuuaaau gcaguugaaa caggugcaac uucuaacacu 2520
gaaccagaag aagccauaca aacucgcaca gugauaaauc agcacggugu auccgagacu 2580
cuaguggaga auuuucucag uagagcagcu uugguaucaa agagaaguuu ugaauacaaa 2640
gaucauacuu cgucugcagc acaagcagac aagaacuuuu ucaaauggac aauuaacacc 2700
agauccuuug uacaguuaag aagaaaauua gaauuauuca cauaccuuag auuugaugcu 2760
gagaucacua uacucacaac uguagcagug aaugguagug guaauaauac auacgugggu 2820
cuuccugacu ugacacucca agcaauguuu guacccacug gugcucuuac cccagaaaaa 2880
caggacucau uccacuggca gucaggcagu aaugcuagug uauucuuuaa aaucuccgac 2940
cccccagcca gaauaaccau accuuuuaug ugcauuaacu cagcauacuc aguuuuuuau 3000
gauggcuuug ccggauuuga gaaaaacggu cuguauggaa uaaauccagc ugacacuauu 3060
gguaacuuau guguuagaau agugaaugaa caccaaccag uugguuucac agugaccguu 3120
aggguuuaca ugaagccuaa acacauaaaa gcaugggcac cacgaccacc acgaacuuug 3180
ccauauauga guauugcaaa ugcaaauuac aaagguaaag aaagagcacc aaaugcgcuc 3240
aaugcuauaa uuggcaauag agacaguguc aaaaccaugc cucauaauau agugaacacu 3300
gguccaggcu ucaugaagca ccugugguuc uuccugcugc ugguggccgc uccuaggugg 3360
gugcuguccc aggugcagcu ggugcagagc ggcguggagg ugaagaagcc cggcgcuucc 3420
gugaaggugu ccugcaaggc cuccggcuac accuucacca acuacuacau guacugggug 3480
aggcaggccc cuggacaggg acuggagugg augggcggca ucaacccuuc caacggcggc 3540
accaacuuca acgagaaguu caagaaccgg gugacccuga ccaccgacuc cuccaccacc 3600

accgccuaca uggagcugaa gucccugcag uuugacgaca ccgccgugua cuacugcgcc 3660
aggagggacu accgguucga caugggcuuc gacuacuggg gccagggcac aaccgugacc 3720
guguccagcg gagguggcgg aucuggaggg ggugguagcg guggaggcgg gagugagauc 3780
gugcugaccc aguccccugc uacacugucc cuguccccg gcgagagggc uacacugagc 3840
ugcagggccu ccaagggcgu guccaccucc ggcuacuccu accugcacug guaccagcag 3900
aagccuggac aggcucccag gcugcugauc uaccuggccu ccuaccugga guccggcgug 3960
ccugcuaggu uuuccggcag cggcagcggc accgauuuca cccugaccau cuccucccug 4020
gagcccgagg acuucgccgu guacuacugc cagcacucca gggaucugcc ucugaccuuc 4080
ggcggcggca ccaaggugga gaucaagagu gucaaaacca ugccucauaa uauagugaac 4140
acugguccag gcuucggagg aguuuuugua gggucuuuca aaauaaucaa cuaucacuug 4200
gccacuacag aagagagaca gucagcuauc uauguggauu ggcaaucaga cgucuugguu 4260
accccccauug cugcucaugg aaggcaccaa auagcaagau gcaagugcaa cacaggggguu 4320
uacuauugua ggcacaaaaa cagaaguuac ccgauuugcu uugaaggccc agggauucaa 4380
uggauugaac aaaaugaaua uuacccagca agguaccaga ccaauguacu auuggcaguu 4440
gguccugcgg aagcaggaga uugcggguggu uuacuaguuu guccacaugg gguaaucggu 4500
cuucuuacag caggaggggg uggaauugua gcuuucacug auaucaggaa uuugcuaugg 4560
uuagauacug augcuaugga acaaggcauu acugauuaua uucaaaaucu ugguaaugcc 4620
uuuggagcag gauuuacaga aacaaucucu aauaaagcca aggaagugca agauaugcua 4680
auuggagaga guucacuauu agaaaaauug uuaaaagcuc uaaucaaaau cauaucagca 4740
uuaguaauug uaaucagaaa cucagaagau uuagucacag ucacagccac acuagcauug 4800
uugggaugcc augauucacc auggagcuac uugaaacaga agguauguuc auacuuaggu 4860
auuccuuaug uaccuagaca gggugaaucg uggcuuaaga aauucacaga ggcaugcaau 4920
gcucuuagag gucuggauug gcuaucgcaa aagauagaua aauucaucaa cuggcuuaaa 4980
accaaaauau uaccagaagc uagggagaaa uaugaauuug ugcaaaggcu caaacaguua 5040
ccggugauag aaaaccaagu uaguacaauc gagcauagcu gcccaacaac agaacaacaa 5100
caggccuuau ucaacaacgu ccaauacuau ucacacuacu guagaaagua cgcaccacuu 5160
uacgcagugg aagcaaagag gguaguagcu cuugaaaaga aaauaaacaa cuacauccag 5220
uucaagucca aaucucgcau ugaaccgguu uguuuaauaa uacauggcuc uccaggaacu 5280
ggcaagucag uggcuucaaa uuuaauugcc agggcuauca cagagaaauu gggggggggac 5340
auuuauuccu ugccuccaga cccuaaauau uuugauggau acaaacagca aacaguggguc 5400
cucauggaug auuuaaugca aaauccagau gggaaugaca uaucuauguu cugccaaaug 5460
gucuccacug uagauuucau acccccaaug gcuaguuugg aggaaaaagg aacucuauac 5520

accaguccau uuuuaauagc uacuaccaau gcuggcucaa uacaugcacc aacuguauca 5580

gacucaaagg cuuugucacg cagauuuaaa uuugacgugg acauugaagu cacagauuca 5640

uacaaggacu caaauaaauu ggauauguca agggcagucg agaugugcaa accagauggc 5700

ugugcccccca ccaauuacaa aagaugcugc ccauugaucu guggaaaggc uauccaauuc 5760

agagaucgca gaacuaaugc aagauccacu auugauaugc uaguaacuga uauuauaaag 5820

gaauauagaa ccagaaacag uacacaggau aagcuggaag cucuguuuca ggggccucca 5880

caguuuaaag agaucaaaau uucagucacc ccagauacac cagcuccuga ugcuauaaau 5940

gaccuucuua ggucagugga uucucaagaa guuagggauu auugccaaaa gaaaggaugg 6000

auuguaguac acccaucaaa ugagcuaaua guagaaaaac acauuaguag agcuuuuauu 6060

acucuacaag ccauugccac cuuuguauca auagcuggug uaguuuaugu uauauacaaa 6120

cuuuuugcug gcauucaggg uccauacaca ggaaucccca auccuaaacc uaaaguaccc 6180

ucucucagaa cagcuaaagu gcaaggacca ggguucgauu uugcacaagc cauaaugaag 6240

aaaaauaccg ucauugcaag gacugaaaag ggugaguuca ccaugcuggg uguauaugau 6300

aggguagcgg ucauccccac acacgcaucu guuggagaaa ccauuuacau uaaugaugua 6360

gagacuaaag uuuuagaugc gugugcacuu agagacuuga cugauacaaa cuuagagaua 6420

accauaguca aauuagaccg uaaucaaaaa uuuagagaua ucagacauuu ucugcccaga 6480

uaugaggaug auuacaauga cgcugugcuu agcguacaua caucaaaauu cccaaauaug 6540

uauaucccag uuggacaagu caccaauuau ggcuucuuga accuaggugg uacaccgacg 6600

caccgcauuu uaauguauaa cuucccaaca agagcuggcc aguguggugg uguggugaca 6660

acuacaggua aggugauagg aauacaugua gguggaaaug gagcucaagg auuugcagca 6720

augcuacuac acucuuacuu uuccgauaca caaggugaga uaguuaguag ugaaaagagu 6780

ggggugugca uuaacgcacc ggcaaagacu aaacuccaac cuaguguuuu ccaucaaguu 6840

uuugaagguu caaaggaacc agcaguucuc aauccaaaag auccuaggcu uaaaacagau 6900

uucgaggagg ccauuuucuc aaaguacaca gguaacaaaa uuauguuaau ggaugaguac 6960

auggaagagg caguggauca uuauguggggg uguuuagaac cauuagacau caguguggau 7020

cccauacccc uggaaagugc cauguaugga auggauggcc uugaggcauu agacuuaacu 7080

accagugcag gauucccuua cuuacuacaa gggaagaaga aaagggauau auuuaauaga 7140

cauacuagag acaccaguga aaugacaaaa auguuagaga aauauggagu ugaccuaccu 7200

uuuguaaccu uuguaaaaga ugagcuuaga ucaagagaaa aaguugaaaa agggaaauca 7260

cgccugauug aggccaguuc cuugaaugac ucaguugcua ugagaguugc cuuuggaaac 7320

cuuuacgcca cauuucacaa caauccaggu acagcaacug guagugcagu ugguugugau 7380

ccagauauau uuuggucaaa aaucccuauu uuguuagaug gagaaaucuu ugcuuuugac 7440

uacacugguu augaugcuag uuugucacca guguggauug ccugcuuaaa gaaaguucua 7500

auuaaguuag guuacacaca ucaaacgucu uuuauagauu auuuguguca uucaguacau 7560

uuauauaagg acaaaaaaua cauaguuaau gguggaaugc ccucugguuc uucaggcacc 7620

agcauauuca acacuaugau caacaauaua aucauaagaa cuuuauuaau uaggguuuac 7680

aaaggcauag accuggacca guucaaaaug auugccuaug gggaugaugu uauugcuagc 7740

uacccacaua agauugaucc agguuugcug gcagaagcag guaaacagua uggauuagua 7800

augacgccag cagacaaagg aaccaguuuu auugacacaa auugggaaaa uguaacuuuc 7860

uuaaaaagau auuucagagc agaugaucaa uaccccuuuc ucauacaucc agugaugcca 7920

augaaagaga uacaugaauc uauuagaugg acuaaagauc ccagaaacac acaggaucau 7980

guuaggucuu ugugcuaccu cgcauggcau aauggagagg aggcuuauaa ugaauuuugc 8040

agaaaaauca gaagugugcc ugugggaaga gcauugacac uaccugcaua cucuagucuu 8100

agacggaaau gguuagauuc guucuagaca acucuaauug aaacccaagu uauaguuacu 8160

uucauuuaga gguaaauuuu ggucacuugg gggccaaaaa aaaaaaaaaa aaaaaaaaaa 8220

gucgac 8226

<210> 17
<211> 22
<212> DNA
<213> Artificial Sequence

<220>
<223> DNA sequence of miR-133 target sequence

<400> 17
acagctggtt gaaggggacc aa 22

<210> 18
<211> 22
<212> DNA
<213> Artificial Sequence

<220>
<223> DNA sequence of miR-206 target sequence

<400> 18
ccacacactt ccttacattc ca 22

<210> 19
<211> 102
<212> DNA
<213> Artificial Sequence

<220>
<223> DNA sequence of tandem sequence of miR-133 target sequence and miR-206 target sequence

```
<400>  19
acagctggtt gaaggggacc aacgatacag ctggttgaag gggaccaaac cggtccacac        60

acttccttac attccatcac ccacacactt ccttacattc ca                          102


<210>  20
<211>  507
<212>  DNA
<213>  Artificial Sequence

<220>
<223>  DNA sequence of the internal ribosome entry site sequence of HRV2

<400>  20
aacttagaag tttttcacaa agaccaatag ccggtaatca gccagattac tgaaggtcaa        60

gcacttctgt ttccccggtc aatgttgata tgctccaaca gggcaaaaac aactgcgatc       120

gttaaccgca aagcgcctac gcaaagctta gtagcatctt tgaaatcgtt tggctggtcg       180

atccgccatt tcccctggta gacctggcag atgaggctag aaatacccca ctggcgacag       240

tgttctagcc tgcgtggctg cctgcacacc ctatgggtgt gaagccaaac aatggacaag       300

gtgtgaagag ccccgtgtgc tcgctttgag tcctccggcc cctgaatgtg gctaacctta       360

accctgcagc tagagcacgt aacccaatgt gtatctagtc gtaatgagca attgcgggat       420

gggaccaact actttgggtg tccgtgtttc actttttcct ttatatttgc ttatggtgac       480

aatatataca atatatatat tggcacc                                           507
```